(19) Europäisches Patentamt
European Patent Office
Office européen des brevets

(11) Veröffentlichungsnummer: **0 305 333 A2**

(12) **EUROPÄISCHE PATENTANMELDUNG**

(21) Anmeldenummer: **88810564.0**

(22) Anmeldetag: **18.08.88**

(51) Int. Cl.⁴: **C 07 D 209/46**
C 07 D 207/44, A 01 N 43/36,
A 01 N 43/38, C 07 D 307/58,
C 07 D 307/60,
C 07 D 307/88,
C 07 D 307/89, C 07 D 209/48

(30) Priorität: **27.08.87 CH 3286/87**

(43) Veröffentlichungstag der Anmeldung:
**01.03.89 Patentblatt 89/09**

(84) Benannte Vertragsstaaten:
**AT BE CH DE ES FR GB GR IT LI LU NL SE**

(71) Anmelder: **CIBA-GEIGY AG**
**Klybeckstrasse 141**
**CH-4002 Basel (CH)**

(72) Erfinder: **Pissiotas, Georg, Dr.**
**Am Sonnenrain 71**
**D-7850 Lörrach (DE)**

**Moser, Hans, Dr.**
**Hauptstrasse 67B**
**CH-4312 Magden (CH)**

**Brunner, Hans-Georg, Dr.**
**Wannenstrasse 14**
**CH-4415 Lausen (CH)**

Patentansprüche für folgenden Vertragsstaat: ES.

(54) **Neue herbizide Wirkstoffe.**

(57) Die Verbindungen der Formel I

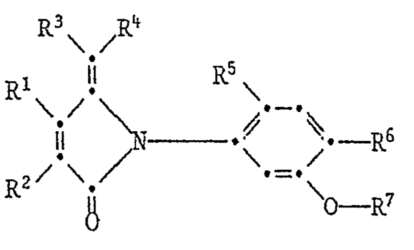

(I),

worin
$R^1$ Wasserstoff; $(C_1\text{-}C_8)$-Alkyl;
$R^2$ Wasserstoff; $(C_1\text{-}C_8)$-Alkyl; oder
$R^1$ und $R^2$ gemeinsam eine gegebenenfalls bis zu zweifach durch $(C^1\text{-}C^4)$-Alkyl substituierte $-(CH_2)_4$-Brücke;
$R^3$ und $R^4$ unabhängig voneinander Wasserstoff; oder $(C_1\text{-}C_4)$-Alkyl; oder
einer der Reste $R^3$ oder $R^4$ Wasserstoff und der andere ein gegebenenfalls bis zu dreifach gleich oder verschieden durch Halogen, $(C_1\text{-}C_4)$-Alky oder $(C_1\text{-}C_4)$-Alkoxy substituierter Phenylrest; oder
$R^3$ und $R^4$ zusammen eine $-(CH_2)_n$-Brücke, mit n = 2, 3, 4, 5 oder 6, die gewünschtenfalls bis zu dreifach durch $(C_1\text{-}C_4)$-Alky substituiert sein kann;
$R^5$ Wasserstoff; oder Fluor;
$R^6$ Halogen;
$R^7$ Wasserstoff; $(C_1\text{-}C_8)$-Alkyl; $(C_3\text{-}C_8)$-Alkenyl; $(C_3\text{-}C_8)$-Alkinyl; $(C_3\text{-}C_7)$-Cycloalkyl; $(C_1\text{-}C_4)$-Alkyl, einfach substituiert durch: $(C_1\text{-}C_4)$-Alkoxy, $(C_1\text{-}C_4)$-Alkylthio, mono-$(C_1\text{-}C_4)$-Alkylamino, di-$(C_1\text{-}C_4)$-Alkylamino, Cyano, $(C_3\text{-}C_7)$-Cycloalkyl, Phenyl, Halogenphenyl, $(C_1\text{-}C_4)$-Alkylphenyl, $(C_1\text{-}C_4)$-Alkoxyphenyl, $(C_1\text{-}C_4)$-Halogenalkyl phenyl, $(C_1\text{-}C_4)$-Halogenalkoxyphenyl, Nitrophenyl, Cyanophenyl, Hydroxy, 1,3-Dioxolan-2-yl, 1,3-Dioxan-2-yl oder CO-$R^8$; $(C_1\text{-}C_8)$-Alkyl, $(C_3\text{-}C_8)$-Alkenyl oder $(C_3\text{-}C_7)$-Cycloalkyl, welche ein oder mehrfach durch Halogen substituiert sind; oder CO-$R^8$.
$R^8$ $(C_1\text{-}C_8)$-Alkyl; $(C_3\text{-}C_8)$-Alkenyl; Benzyl, gegebenenfalls am Phenylring einfach substituiert durch Halogen, $(C_1\text{-}C_4)$-Alkyl, $(C_1\text{-}C_4)$-Alkoxy, $(C_1\text{-}C_4)$-Halogenalkyl, Nitro oder Cyano; $(C_3\text{-}C_7)$-Cycloalkyl; Phenyl, gegebenenfalls bis zu dreifach substituiert durch Halogen, $(C_1\text{-}C_8)$-Alkyl, $(C_1\text{-}C_4)$-Alkoxy, $(C_1\text{-}C_4)$-Halogenalkyl oder einfach substituiert durch Cyano

EP 0 305 333 A2

oder Nitro; Furan; Thiophen; ($C_1$-$C_8$)-Alkoxy; ($C_1$-$C_4$)-Alkoxy-($C_1$-$C_4$)-alkoxy; ($C_3$-$C_8$)-Alkenyloxy; ($C_3$-$C_8$)-Alkinyloxy; ($C_1$-$C_8$)-Alkylthio; ($C_3$-$C_8$)-Alkenylthio; ($C_3$-$C_8$)-Alkinylthio; Amino; mono($C_1$-$C_4$)-Alkylamino; di-($C_1$-$C_4$)-Alkylamino; Phenylamino, gegebenenfalls bis zu dreifach substituiert durch Halogen, ($C_1$-$C_4$)-Alkyl, ($C_1$-$C_4$)-Alkoxy oder ($C_1$-$C_4$)-Halogenalkyl oder einfach substituiert durch Cyano oder Nitro; bedeutet, sind herbizide Wirkstoffe.

Gegenstand der Erfinding sind Verbindungen der Formel I, Verfahren zu deren Herstellung, neue Zwischenprodukte und neue herbizide und wuchsregulatorische Mittel sowie deren Verwendung zur Bekämpfung unerwünschten Pflanzenwuchses oder zur Beeinflussung des Wachstums von Kulturpflanzen.

**Beschreibung**

## Neue herbizide Wirkstoffe

Die Erfindung betrifft neue herbizid wirksame 1-phenylsubstituierte 5-exo-Methylen-pyrrolidin-2-one und 2-phenylsubstituierte 3-exo-Methylen-4,5,6,7-tetrahydroisoindol-(2H)1-one der Formel I, Verfahren zu deren Herstellung, herbizide Mittel und deren Verwendung sowie neue Zwischenverbindungen.

Aus der US-Patentschrift US 3,992,189 sind herbizid wirksame 3-exo-Methylen-4,5,6,7-tetrahydroisoindol-(2H)1-one bekannt geworden, welche in Position 2 des Isoindolsystems einen bis zu dreifach in ortho- und para-Stellung substituierten Phenylrest tragen. Diese aus dem Stand der Technik bekannten Wirkstoffe befriedigen nicht immer im Hinblick auf Wirkung und Selektivität.

Demgegenüber betrifft die Erfindung Verbindungen der Formel I

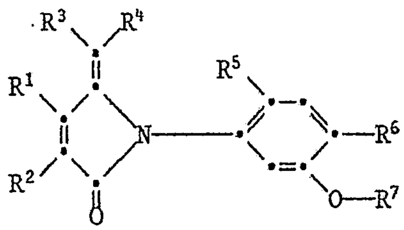
(I),

worin

$R^1$ Wasserstoff; ($C_1$-$C_8$)-Alkyl;

$R^2$ Wasserstoff; ($C_1$-$C_8$)-Alkyl; oder

$R^1$ und $R^2$ gemeinsam eine gegebenenfalls bis zu zweifach durch ($C_1$-$C_4$)-Alkyl substituierte -$(CH_2)_4$-Brücke;

$R^3$ und $R^4$ unabhängig voneinander Wasserstoff; oder ($C_1$-$C_4$)-Alkyl; oder

einer der Reste $R^3$ oder $R^4$ Wasserstoff und der andere ein gegebenenfalls bis zu dreifach gleich oder verschieden durch Halogen, ($C_1$-$C_4$)-Alkyl oder ($C_1$-$C_4$)-Alkoxy substituierter Phenylrest; oder

$R^3$ und $R^4$ zusammen eine -$(CH_2)_n$-Brücke, mit $n = 2, 3, 4, 5$ oder 6, die gewünschtenfalls bis zu dreifach durch ($C_1$-$C_4$)-Alkyl substituiert sein kann;

$R^5$ Wasserstoff; oder Fluor;

$R^6$ Halogen;

$R^7$ Wasserstoff; ($C_1$-$C_8$)-Alkyl; ($C_3$-$C_8$)-Alkenyl; ($C_1$-$C_8$)-Alkinyl; ($C_3$-$C_7$)-Cycloalkyl; ($C_1$-$C_4$)-Alkyl, einfach substituiert durch: ($C_1$-$C_4$)-Alkoxy, ($C_1$-$C_4$)-Alkylthio, mono-($C_1$-$C_4$)-Alkylamino, di-($C_1$-$C_4$)-Alkylamino, Cyano, ($C_3$-$C_7$)-Cycloalkyl, Phenyl, Halogenphenyl, ($C_1$-$C_4$)-Alkylphenyl, ($C_1$-$C_4$)-Alkoxyphenyl, ($C_1$-$C_4$)-Halogenalkylphenyl, ($C_1$-$C_4$)-Halogenalkoxyphenyl, Nitrophenyl, Cyanophenyl, Hydroxy, 1,3-Dioxolan-2-yl, 1,3-Dioxan-2-yl oder CO-$R^8$; ($C_1$-$C_8$)-Alkyl, ($C_3$-$C_8$)-Alkenyl oder ($C_3$-$C_7$)-Cycloalkyl, welche ein oder mehrfach durch Halogen substituiert sind; oder CO-$R^8$.

$R^8$ ($C_1$-$C_8$)-Alkyl; ($C_3$-$C_8$)-Alkenyl; Benzyl, gegebenenfalls am Phenylring einfach substituiert durch Halogen, ($C_1$-$C_4$)-Alkyl, ($C_1$-$C_4$)-Alkoxy, ($C_1$-$C_4$)-Halogenalkyl, Nitro oder Cyano; ($C_3$-$C_7$)-Cycloalkyl; Phenyl, gegebenenfalls bis zu dreifach substituiert durch Halogen, ($C_1$-$C_8$)-Alkyl, ($C_1$-$C_4$)-Alkoxy, ($C_1$-$C_4$)-Halogenalkyl oder einfach substituiert durch Cyano oder Nitro; Furan; Thiophen; ($C_1$-$C_8$)-Alkoxy; ($C_1$-$C_4$)-Alkoxy-($C_1$-$C_4$)-Alkoxy; ($C_3$-$C_8$)-Alkenyloxy; ($C_3$-$C_8$)-Alkinyloxy; ($C_1$-$C_8$)-Alkylthio; ($C_3$-$C_8$)-Alkenylthio; ($C_3$-$C_8$)-Alkinylthio; Amino; mono($C_1$-$C_4$)-Alkylamino; di-($C_1$-$C_4$)-Alkylamino; Phenylamino, gegebenenfalls bis zu dreifach substituiert durch Halogen, ($C_1$-$C_4$)-Alkyl, ($C_1$-$C_4$)-Alkoxy oder ($C_1$-$C_4$)-Halogenalkyl oder einfach substituiert durch Cyano oder Nitro;

bedeutet.

In der obigen Definition umfassen die angegebenen generischen Begriffe, sowie die durch Kombination einzelner Unterbegriffe erhältlichen Substituenten, beispielsweise die folgenden spezifischen Einzelsubstituenten, wobei diese Aufzählung keine Einschränkung der Erfindung darstellt:

Alkyl:

Methyl, Ethyl, n-Propyl, i-Propyl, n-Butyl, i-Butyl, s-Butyl, t-Butyl, n-Pentyl und sec-Pentyl, vorzugsweise Methyl, Ethyl und Isopropyl.

Halogen:

Fluor, Chlor, Brom und Jod, vorzugsweise Fluor, Chlor und Brom.

Alkoxy:
Methoxy, Ethoxy, Propyloxy, i-Propyloxy, n-Butyloxy, i-Butyloxy, s-Butyloxy und t-Butyloxy, vorzugsweise Methoxy oder Ethoxy.

Halogenalkyl:
Fluormethyl, Difluormethyl, Trifluormethyl, Chlormethyl, Dichlormethyl, Trichlormethyl, 2,2,2-Trifluorethyl, 2-Fluorethyl, 2-Chlorethyl und 2,2,2-Trichlorethyl, vorzugsweise Chlormethyl, 2-Chlorethyl und Trifluormethyl.

Halogenalkoxy:
Fluormethoxy, Difluormethoxy, Trifluormethoxy, 2,2,2-Trifluorethoxy, 1,1,2,2-Tetrafluorethoxy, 2-Fluorethoxy, 2-Chlorethoxy und 2,2,2-Trichlorethoxy, vorzugsweise Difluormethoxy, 2-Chlorethoxy und Trifluormethoxy.

Alkoxycarbonyl:
Methoxycarbonyl, Ethoxycarbonyl, 4-Propyloxycarbonyl, i-Propyloxycarbonyl und n-Butyloxycarbonyl, vorzugsweise Methoxycarbonyl und Ethoxycarbonyl.

Alkoxyalkyl:
Methoxymethyl, Ethoxymethyl, Propyloxymethyl, 2-Methoxyethyl, 1-Methoxyethyl, Ethoxyethyl, Propyloxyethyl, Methoxypropyl, Aethoxypropyl oder Propyloxypropyl.

Alkylthio:
Methylthio, Ethylthio, Propylthio, Isopropylthio, (n)-Butylthio, (i)-Butylthio, (s)-Butylthio oder (t)-Butylthio.

Alkylamino:
Methylamino, Dimethylamino, Ethylamino oder Diethylamino.

Cyanoalkyl:
Cyanomethyl, Cyanoethyl oder Cyanopropyl.

Cycloalkyl:
Cyclopropyl, Cyclobutyl, Cyclopentyl, Cyclohexyl oder Cycloheptyl. Bevorzugt sind Cyclopropyl, Cyclopentyl oder Cyclohexyl.

Halogencycloalkyl:
2,2-Dichlorcyclopropyl oder Pentachlorcyclohexyl.

Alkenyl:
sind insbesondere die über ein gesättigtes Kohlenstoffatom gebundenen Allyl-, 2-Butenyl-, 3-Butenyl- oder Methallylreste.

Alkinyl:
sind insbesondere die über ein gesättigtes Kohlenstoffatom gebundenen Propargyl-, 2-Butinyl- oder 3-Butinylreste.

Cycloalkoxycarbonyl:
Cyclopentyloxycarbonyl oder Cyclohexyloxycarbonyl.

Phenyl, auch als Teil eines grösseren Substituenten wie Phenoxy, Phenylthio, Phenoxycarbonyl, Phenylaminocarbonyl, Benzyl oder Benzoyl, kann im allgemeinen unsubstituiert oder durch weitere Substituenten substituiert vorliegen. Die Substituenten können dann in ortho-, meta- oder para-Stellung stehen. Bevorzugte Substituentenstellungen sind die ortho- und para-Position zur Ringverknüpfungsstelle. Bevorzugte Substituenten sind Halogenatome.

In den Fällen, in denen $R^3$ für Wasserstoff und $R^4$ für einen gegebenenfalls substituierten Phenylrest steht, sind neben dem unsubstituierten Phenylrest im allgemeinen Phenylreste bevorzugt, welche die Lipophilie des Gesamtmoleküls begünstigen, wie etwa die isomeren Chlorphenyl-, Fluorphenyl-, Methylphenyl-, Dimethylphenyl-, Trimethylphenyl-, Methoxyphenyl- oder Dimethoxyphenylreste.

Wenn die Substituenten $R^3$ und $R^4$ gemeinsam für eine $-(CH_2)_n$-Brücke stehen, bilden sie zusammen mit dem Kohlenstoffatom, an welches sie gebunden sind, einen carbocyclischen 3 bis 7-Ring. Bevorzugt sind 3, 5 und 6-Ringe. Dieser Carbocyclus kann seinerseits wieder durch bis zu drei $(C_1$-$C_4)$-Alkylgruppen substituiert sein.

In den weiteren Substituenten, welche aus mehreren Grundelementen zusammengesetzt sind, haben die Teilelemente die oben an Beispielen erläuterten Bedeutungen. Diese Aufzählungen bedeuten auch in diesen Fällen keine Einschränkdung der Erfindung: sie haben illustrierenden Charakter.

Bei bestimmter Substitution können die Verbindungen der Formel I ein oder mehrere Asymmetriezentren aufweisen. Die Erfindung umfasst sowohl das Racemat, wie auch die optisch reinen Isomeren und die mit einer oder mehreren optisch aktiven Formel angereicherten Gemische.

Die Isomeren können im allgemeinen nach bekannten Verfahren, wie fraktionierte Kristallisation oder Auftrennung mittels besonderer Chromatographieverfahren hergestellt werden. Darüber hinaus kann in vielen Fällen das gewünschte enantiomere Produkt durch die Verwendung chiraler Edukte hergestellt werden.

Bevorzugt sind Verbindungen der Formel I,
worin
$R^1$ Wasserstoff; $(C_1$-$C_4)$-Alkyl;
$R^2$ Wasserstoff; $(C_1$-$C_4)$-Alkyl; oder
$R^1$ und $R^2$ gemeinsam eine gegebenenfalls einfach durch $(C_1$-$C_4)$-Alkyl substituierte $-(CH_2)_4$-Brücke;
$R^3$ und $R^4$ unabhängig voneinander Wasserstoff; oder $(C_1$-$C_4)$-Alkyl; oder
einer der Reste $R^3$ oder $R^4$ Wasserstoff und der andere ein gegebenenfalls bis zu dreifach durch Fluor, Chlor, Methyl oder Methoxy substituierter Phenylrest; oder
$R^3$ und $R^4$ zusammen eine $-(CH_2)_n$-Brücke, mit $n = 2$, 4 oder 5, die gewünschtenfalls bis zu dreifach durch $(C_1$-$C_4)$-Alkyl substituiert sein kann;
$R^5$ Wasserstoff; oder Fluor;
$R^6$ Fluor; Chlor; oder Brom;
$R^7$ Wasserstoff; $(C_1$-$C_5)$-Alkyl; $(C_3$-$C_5)$-Alkenyl; $(C_3$-$C_5)$-Alkinyl; $(C_5$-$C_6)$-Cycloalkyl, $(C_1$-$C_4)$-Alkyl, einfach substituiert durch: $(C_1$-$C_4)$-Alkoxy, $(C_1$-$C_4)$-Alkylthio, mono-$(C_1$-$C_4)$-Alkylamino, di- $(C_1$-$C_4)$-Alkylamino, Cyano, Cyclohexyl, Phenyl, oder CO-$R^8$; $(C_1$-$C_4)$-Alkyl oder $(C_3$-$C_5)$-Alkenyl, welche ein oder mehrfach durch Halogen substituiert sind; oder CO-$R^8$.
$R^8$ $(C_1$-$C_4)$-Alkyl; Phenyl; $(C_1$-$C_4)$-Alkylthio; $(C_1$-$C_4)$-Alkoxy; Amino; oder di-$(C_1$-$C_4)$-Alkylamino;
bedeutet.

Insbesondere betrifft die Erfindung Verbindungen der Formel I, worin
$R^1$ Wasserstoff; $(C_1$-$C_4)$-Alkyl;
$R^2$ Wasserstoff; $(C_1$-$C_4)$-Alkyl; oder
$R^1$ und $R^2$ gemeinsam eine gegebenenfalls einfach durch Methyl substituierte $-(CH_2)_4$-Brücke;
$R^3$ und $R^4$ unabhängig voneinander Wasserstoff; oder $(C_1$-$C_4)$-Alkyl; oder
einer der Reste $R^3$ oder $R^4$ Wasserstoff und der andere ein unsubstituierter oder einfach durch Fluor oder Chlor, oder bis zu dreifach durch Methyl oder Methoxy substituierter Phenylrest; oder
$R^3$ und $R^4$ zusammen eine $-(CH_2)_n$-Brücke, mit $n = 2$, 4 oder 5,
$R^5$ Wasserstoff; oder Fluor;
$R^6$ Fluor; Chlor; oder Chlor;
$R^7$ Wasserstoff; $(C_1$-$C_5)$-Alkyl; Propargyl; Allyl; $(C_1$-$C_3)$-Alkyl, einfach substituiert durch Chlor, Cyano, Hydroxy, Methoxy, Cyclohexyl, $(C_1$-$C_3)$-Alkylthio, Dimethylamino, Phenyl oder CO-$R^8$; Chlorallyl; oder CO-$R^8$;
$R^8$ $(C_1$-$C_4)$-Alkoxy; Methyl; Phenyl; Methylthio; Amino; oder Dimethylamino;
bedeutet.

Im Hinblick auf ihre herbizide Wirkung sind nachstehend genannte Gruppen hervorzuheben:

A: Verbindungen der Formel I, worin $R^1$ und $R^2$ unabhängig voneinander $(C_1$-$C_4)$-Alkyl bedeuten.

B: Verbindungen der Formel I, worin $R^1$ und $R^2$ gemeinsam eine gegebenenfalls einfach durch Methyl substituierte $-(CH_2)_4$-Brücke bedeutet.

C: Verbindungen der Formel I, worin $R^3$ und $R^4$ Wasserstoff bedeutet.

D: Verbindungen der Formel I, worin $R^3$ Wasserstoff und $R^4$ $(C_1$-$C_4)$-Alkyl bedeutet.

E: Verbindungen der Formel I, worin $R^4$ Wasserstoff und $R^3$ $(C_1$-$C_4)$-Alkyl bedeutet.

F: Verbindungen der Formel I, worin $R^3$ und $R^4$ zusammen eine $-(CH_2)_n$-Brücke, mit $n = 2, 3, 4, 5$ oder 6, die gewünschtenfalls bis zu dreifach durch $(C_1$-$C_4)$-Alkyl substituiert sein kann, bedeutet.

G: Verbindungen der Formel I, worin $R^3$ und $R^4$ zusammen eine $-(CH_2)_n$-Brücke, mit $n = 2$, 4 oder 5, bedeutet.

H: Verbindungen der Formel I, worin $R^1$ und $R^2$ jeweils die gleiche Bedeutung haben.

I: Verbindungen der Formel I, worin $R^3$ und $R^4$ unabhängig voneinander $(C_1$-$C_4)$-Alkyl bedeuten.

J: Verbindungen der Formel I, worin $R^5$ Fluor und $R^6$ Chlor bedeutet.

K: Verbindungen der Formel I, worin $R^7$ einen über ein sekundäres Kohlenstoffatom gebundenen $(C_1$-$C_5)$-Alkylrest bedeutet.

L: Verbindungen der Formel I, worin $R^7$ Isopropyl bedeutet.

M: Verbindungen der Formel I, worin $R^7$ einen gegebenenfalls durch Chlor substituierten $(C_3\text{-}C_5)$-Alkenylrest bedeutet.

N: Verbindungen der Formel I, worin $R^7$ Propargyl bedeutet und

O: Verbindungen der Formel I, worin $R^7$ ein durch $COR^8$ substituierten $(C_1\text{-}C_4)$-Alkylrest bedeutet.

Namentlich zu nennen sind:

2-(4-Chlor-2-fluor-5-isopropyloxyphenyl)-3-methylen-4,5,6,7-tetrahydroisoindol-(2H)1-on,
2-(4-Chlor-2-fluor-5-propargyloxyphenyl)-3-methylen-4,5,6,7-tetrahydroisoindol-(2H)1-on,
2-[4-Chlor-2-fluor-5-(2-methylpropyloxy)phenyl]-3-methylen-4,5,6,7-tetrahydroisoindol-(2H)1-on,
2-[4-Chlor-2-fluor-5-(2-chlorprop-2-enyloxy)-phenyl]-4,5,6,7-tetrahydroisoindol-(2H)1-on,
2-[4-Chlor-2-fluor-5-(1-methoxycarbonylethoxy)phenyl]-4,5,6,7-tetrahydroisoindol-(2H)1-on,
2-[4-Chlor-2-fluor-5-(3-chlorprop-2-enyloxy)-phenyl]-4,5,6,7-tetrahydroisoindol-(2H)1-on,
2-[4-Chlor-2-fluor-5-(prop-2-inyloxy)-phenyl]-4,5,6,7-tetrahydroisoindol-(2H)1-on,
1-(4-Chlor-2-fluor-5-isopropyloxyphenyl)-3,4-dimethyl-5-methylenpyrrol-(1H)2-on,
2-(4-Chlor-2-fluor-5-isopropyloxyphenyl)-3-(1,1-dimethylmethylen)-4,5,6,7-tetrahydroisoindol-(2H)1-on,
2-(4-Chlor-2-fluor-5-isopropyloxyphenyl)-3-ethyliden-4,5,6,7-tetrahydroisoindol-(2H)1-on,
5-Benzyliden-1-(4-chlor-2-fluor-5-isopropyloxy-phenyl)-3,4-dimethylpyrrol-(1H)2-on oder
5-(o-Chlorbenzyliden)-1-(4-chlor-2-fluor-5-propargyloxy-phenyl)-3,5-dimethyl-pyrrol-(1H)2-on.

Die Verbindungen der Formel I können hergestellt werden

a) durch die Alkylierung oder Acylierung eines Phenols der Formel Ia,

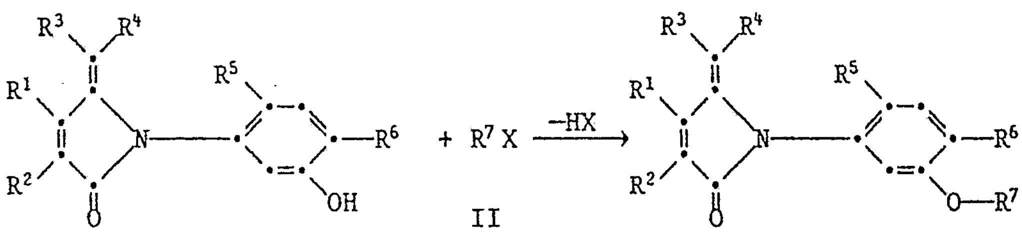


II

Ia ($R^7$ = H) I

worin die Reste $R^1$ bis $R^6$ wie zuvor definiert sind, mit einer Verbindung der Formel II, worin $R^7$ wie zuvor definiert ist, jedoch nicht für Wasserstoff und X für eine unter der Reaktionsbedingungen abspaltbare Gruppe steht, unter Basenzusatz umsetzt, wobei X vorzugsweise Halogen, wie Chlor, Brom oder Jod oder einen gewünschtenfalls im Phenylkern alkylierten oder halogenierten Phenylsulfonylrest, ein Radikal der Formel $R^7OSO_2\text{-}O$ oder wenn $R^7$ für

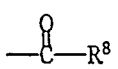 steht auch eine Gruppe 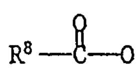

bedeutet oder

b) durch Umsetzung eines 5-Chlor-2,5-dihydrofuran-2-ons der Formel VIII, worin die Reste $R^1$ bis $R^4$ wie zuvor definiert sind mit einem Anilin der Formel VII, worin die Reste $R^5$ bis $R^7$ wie zuvor definiert sind

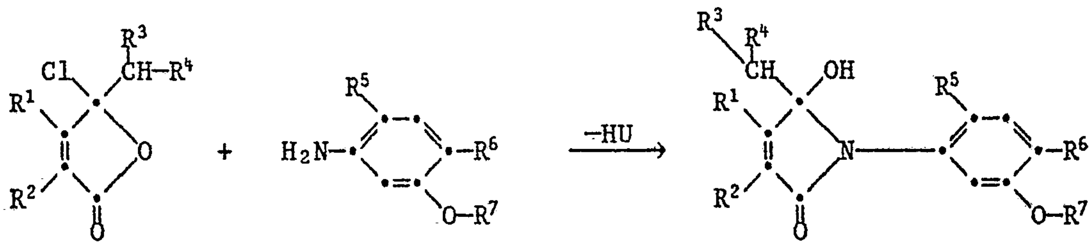


VIII VII V

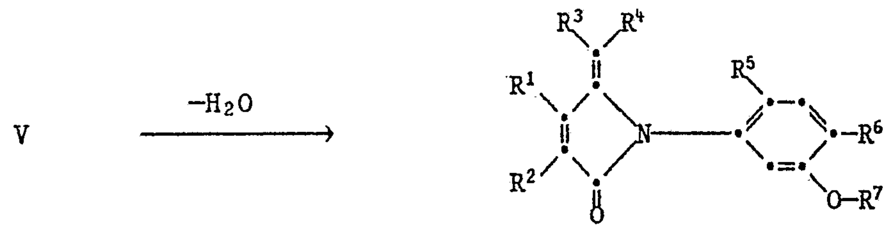


zu einem Primäraddukt der Formel V und anschliessender Wasserabspaltung zu I.

Nach einem weiteren generell anwendbaren Verfahren herstellbar sind die Verbindungen der Formel Ia

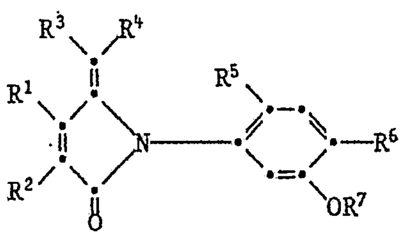

(Ia),

worin

$R^1$ Wasserstoff; $(C_1-C_8)$-Alkyl;

$R^2$ Wasserstoff; $(C_1-C_8)$-Alkyl; oder

$R^1$ und $R^2$ gemeinsam eine gegebenenfalls bis zu zweifach durch $(C_1-C_4)$-Alkyl substituierte $-(CH_2)_4$-Brücke;

$R^3$ und $R^4$ unabhängig voneinander Wasserstoff; oder $(C_1-C_4)$-Alkyl; oder

einer der Reste $R^3$ oder $R^4$ Wasserstoff und der andere ein gegebenenfalls bis zu dreifach gleich oder verschieden durch Halogen, $(C_1-C_4)$-Alkyl oder $(C_1-C_4)$-Alkoxy substituierter Phenylrest; oder

$R^3$ und $R^4$ zusammen eine $-(CH_2)_n$-Brücke, mit $n = 2, 3, 4, 5$ oder 6, die gewünschtenfalls bis zu dreifach durch $(C_1-C_4)$-Alkyl substituiert sein kann;

$R^5$ Wasserstoff; oder Fluor;

$R^6$ Halogen;

$R^7$ Wasserstoff; $(C_1-C_8)$-Alkyl; $(C_3-C_8)$-Alkenyl; $(C_3-C_8)$-Alkinyl; $(C_3-C_7)$-Cycloalkyl; $(C_1-C_4)$-Alkyl, einfach substituiert durch: $(C_1-C_4)$-Alkoxy, $(C_1-C_4)$-Alkylthio, mono-$(C_1-C_4)$-Alkylamino, di-$(C_1-C_4)$-Alkylamino, $(C_3-C_7)$-Cycloalkyl, Phenyl, Halogenphenyl, $(C_1-C_4)$-Alkylphenyl, $(C_1-C_4)$-Alkoxyphenyl, $(C_1-C_4)$-Halogenalkylphenyl, $(C_1-C_4)$-Halogenalkoxyphenyl, Nitrophenyl, Hydroxy, 1,3-Dioxolan-2-yl oder 1,3-Dioxan-2-yl; $(C_1-C_8)$-Alkyl, $(C_3-C_8)$-Alkenyl oder $(C_3-C_7)$-Cycloalkyl, welche ein oder mehrfach durch Halogen substituiert sind;

bedeutet,

c) durch die Umsetzung einer Verbindung der Formel III

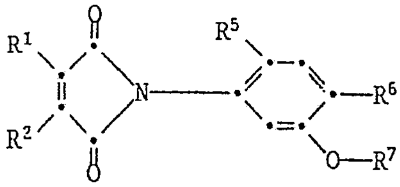

(III),

worin $R^1$, $R^2$, $R^5$, $R^6$ und $R^7$ wie zuvor definiert sind, mit einem Grignard-Reagenz der Formel IV

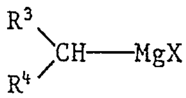

(IV),

worin $R^3$ und $R^4$ unabhängig voneinander Wasserstoff; oder ($C_1$-$C_4$)-Alkyl; $R^3$ Wasserstoff und $R^4$ einen gegebenenfalls bis zu dreifach gleich oder verschieden durch Halogen, ($C_1$-$C_4$)-Alkyl oder ($C_1$-$C_4$)-Alkoxy substituierten Phenylrest; oder $R^3$ und $R^4$ zusammen eine $-(CH_2)_n$-Brücke, mit $n = 2, 3, 4, 5$ oder 6, die gewünschtenfalls bis zu dreifach durch ($C_1$-$C_4$)-Alkyl substituiert sein kann; bedeutet, zu einer Verbindung der Formel V

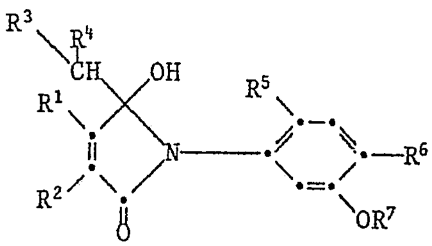

(V),

worin die Reste $R^1$ bis $R^7$ wie zuvor definiert sind, und anschliessender Wasserabspaltung zu Ia.

Die Verfahrensvariante b) kann analog zu literaturbekannten Verfahren (R. Scheffold und P. Dabs, Helv.Chim.Acta. 50 (1967) 798 sowie die dort angegebene Literatur) durchgeführt werden.

Bei der Grignard Reaktion c) kann das Grignard-Reagenz der Formel IV mit beiden Ketogruppen des Imids III reagieren, was im Falle einer unsymmetrischen Substitution von III ($R^1 \neq R^2$) zu zwei verschiedenen Produkten führt.

Im allgemeinen wird das Grignard-Reagenz von der sterisch am wenigsten gehinderten Seite aus angreifen (kinetisch kontrollierte Reaktion). Aus dem, bei unsymmetrischer Substitution, entstehenden Gemisch der Primärprodukte kann durch Trennverfahren (Extraktion, Chromatographie, Kristallisation etc.) das gewünschte Produkt in angereicherter oder reiner Form erhalten werden. Diese Trennverfahren sind sowohl auf der Stufe des Primäraddukts V, wie auch auf der Stufe der exo-Methylenverbindung Ia durchführbar.

In den Fällen, in denen in Formel I oder Ia die Reste $R^3$ und $R^4$ unterschiedliche Bedeutung haben, entsteht bei der Wasserabspaltung aus V ebenfalls ein Gemisch isomerer Verbindungen gemäss nachstehender Gleichung:

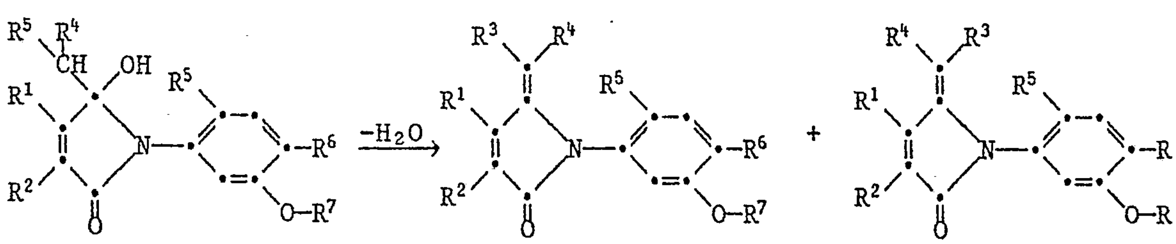


V        I oder Ia        I' oder Ia'

Das Gemisch I/I' oder Ia/Ia' kann mittels allgemein üblicher Trennverfahren (siehe oben) aufgearbeitet und

in die reinen oder angereicherten Komponenten I und I′ oder Ia und Ia′ aufgetrennt werden.

Obwohl die als Reaktionen a) und b) skizzierten Synthesen der Verbindungen der Formel I Verfahren beschreiben, mit welchen prinzipiell sämtliche von Formel I umfasste Verbindungen herstellbar sind, kann es aus ökonomischen oder verfahrenstechnischen Gründen sinnvoll sein, bestimmte Verbindungen der Formel I in andere, ebenfalls von Formel I umfasste Derivate, zu überführen. Beispiel für derartige Umwandlungen sind, neben der Reaktion a) selbst, zum Beispiel Verfahren, bei denen $R^7$ für ein Halogenalkyl bzw. Halogenalkoxyradikal steht. Diese können etwa durch Reaktion mit Alkoholen oder Aminen in Alkoxyalkyl-, Alkoxyalkoxy-, Aminoalkyl- oder Aminoalkoxyreste überführt werden. Derartige Derivatisierungsreaktionen sind dem Fachmann geläufig.

Mit Vorteil führt man die obigen Umsetzungen in einem reaktionsinerten Lösungsmittel aus. Dabei kommen als inerte Lösungsmittel, insbesondere für Reaktion a) und b), Kohlenwasserstoffe, wie Benzol, Toluol oder Xylol; Ether, wie Diethylether, Methylisopropylether, Glyme, Diglyme; cyclische Ether, wie Tetrahydrofuran und Dioxan; Ketone, wie Aceton, Methylethylketon; Amide, wie Dimethylformamid, N-Methylpyrrolidon; Sulfoxide, wie Dimethylsulfoxid; oder chlorierte Kohlenwasserstoffe, wie Dichlormethan, Trichlormethan, Tetrachlormethan oder Tetrachlorethan in Betracht.

Die Reaktionstemperatur kann in weiten Grenzen variiert werden. Geeignete Reaktionstemperaturen Liegen beispielsweise zwischen -20°C und der Rückflusstemperatur des Reaktionsgemisches. Vorzugsweise wird die Umsetzung bei einer Temperatur zwischen 0°C und 100°C durchgeführt.

Im Fall der Reaktion a) ist es vorteilhaft unter Basenzusatz zu arbeiten. Geeignete Basen sind u.a. Natrium-, Kalium- und Calciumhydroxid, Alkali-und Erdalkalicarbonate, Amine, wie etwa Triethylamin oder Heterocyclen, wie Pyridin, DABCO sowie Alkalihydride.

Die Reaktion a) können auch vorteilhaft unter Phasentransfer-Bedingungen in Zweiphasensystemen durchgeführt werden. Derartige Reaktionen sind dem Fachmann geläufig (z.B. beschrieben in Dehmlow und Dehmlow, Phase Transfer Catalysis, Verlag Chemie, Weinheim 1983; W.E. Keller, Phase Transfer Reactions Vol. 1 und Vol. 2, G. Thieme Verlag, Stuttgart 1986, 1987).

Die in Verfahrensvariante c) beschriebene organomagnesium Addition ist als Grignard-Reaktion bekannt (US-P-3 992 189 und Krauch Kunz; Reaktionen der Organischen Chemie, A. Hüthig Verlag, Heidelberg; 5.Aufl. 1976, S. 572-4; sowie die dort genannten Referenzen).

Die für Grignard-Reaktionen an sich vorteilhaften Reaktionsbedingungen (Verfahrensschritte, Lösungsmittel, Temperaturen etc.) sind dem Fachmann geläufig und brauchen hier nicht weiter erläutert zu werden.

Die Verbindungen der Formel I umfassen auch (für $R^7$) Substituenten, wie Cyano oder $-COR^8$, welche ihrerseits mit der Grignard-Verbindung IV reagieren. Daher ist Verfahren c) nur in dem auf die Formel Ia beschränkten Umfang zur Herstellung der erfindungsgemässen Endprodukte I geeignet.

Besonders hervorzuheben sind hier die Phenole der Formel Ia (mit $R^7$ = H), welche neben ihrer herbiziden Wirkung auch wertvolle Zwischenprodukte zur Herstellung der Endprodukte gemäss Verfahren a) sind.

Direktes Verfahrensprodukt der Grignard-Reaktion c) sind die Alkohole IV, welche durch Wasserabspaltung erst die erfindungsgemässen exo-Methylenverbindungen der Formel I liefern. Die Wasserabspaltung kann nach an sich bekannten Verfahren durch Protonensäuren, wie p-Toluolsulfonsäure, Halogenwasserstoffsäure, Schwefelsäure etc. bewirkt werden. Als weitere vorteilhafte Massnahme hat sich die Einwirkung von Kaliumhydrogensulfat bei erhöhter Temperatur erwiesen.

Die Alkohole der Formel V sind wertvolle Zwischenprodukte zur Herstellung der erfindungsgemässen exo-Methylenverbindungen Ia.

Die Verbindungen der Formel III sind bereits bekannt oder können analog zu bekannten Verfahren hergestellt werden durch

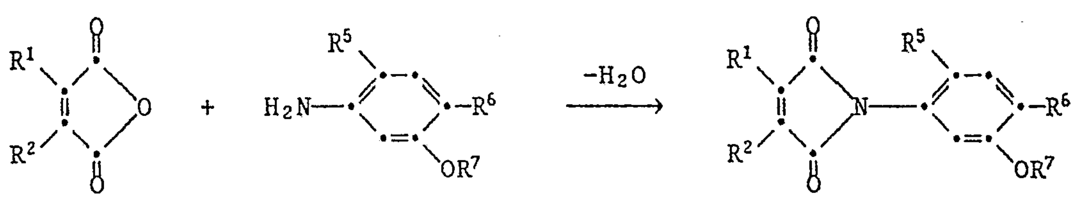


VI VII III

Umsetzung der Furandione VI mit den Anilinen VII, wobei die Reste $R^1$ bis $R^7$ wie unter Formel Ia definiert sind.

Einige Verbindungen der Formel VIII sind bekannt, wie etwa die Verbindungen VIII, in denen $R^3$ und $R^4$ Wasserstoff und die Reste $R^1$ und $R^2$ Wasserstoff oder Methyl bedeuten. Die Verbindungen der Formel VIII können gemäss nachstehendem Reaktionsschema analog zur vorgenannten Literaturstelle Scheffold und Dabs hergestellt werden.

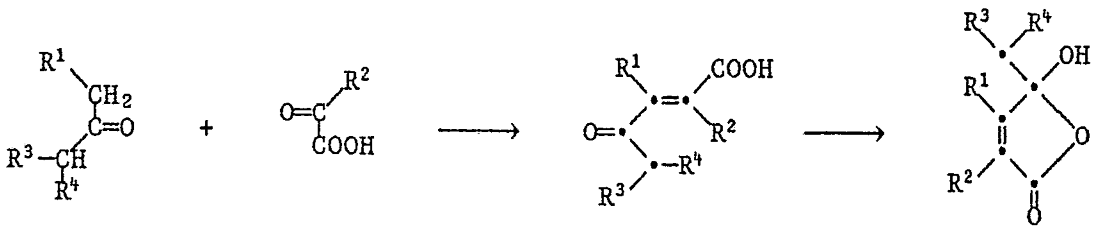

IX X XI XII

XII + $SOCl_2$

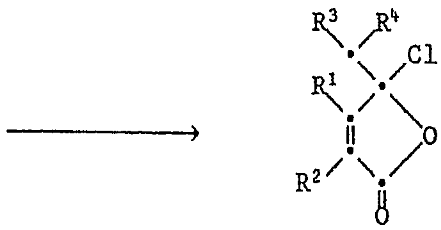

VIII

Die Verbindungen der Formel VIII, in denen $R^3$ und $R^4$ Wasserstoff und die Reste $R^1$ und $R^2$ Wasserstoff, Methyl oder $-(CH_2)_4-$ bedeuten, sind ebenfalls literaturbekannt.

Die neuen Verbindungen der Formel XII und VIII sind als wertvolle Zwischenprodukte ebenfalls Gegenstand vorliegender Erfindung.

Die Verbindungen der Formel I sind hochaktive Pflanzenwirkstoffe, welche sich bei geeigneten Aufwandmengen hervorragend als Selektivherbizide zur Unkrautbekämpfung in Nutzpflanzenkulturen eignen. Das heisst, bei diesen Aufwandmengen zeichnen sich die Wirkstoffe der Formel I durch gute selektiv-hebizide Eigenschaft gegen Unkräuter aus. Kulturpflanzen wie Roggen, Gerste, Hafer, Weizen, Mais, Hirse, Reis, Baumwolle und Soja bleiben bei niedrigen Aufwandmengen praktisch ungeschädigt. Bei gesteigerten Aufwandmengen werden die Kulturpflanzen nur geringfügig in ihrem Wachstum beeinflusst. Werden sehr hohe Aufwandmengen appliziert, entfalten die Substanzen der Formel I totalherbizide Eigenschaften.

Die erfindungsgemässen Wirkstoffe greifen in den Metabolismus der Pflanzen ein und können deshalb auch als Wachstumsregulatoren eingesetzt werden.

Für die Wirkungsweise von Pflanzenwachstumsregulatoren gilt nach der bisherigen Erfahrung, dass ein Wirkstoff auch mehrere verschiedenartige Wirkungen auf Pflanzen ausüben kann. Die Wirkungen der Stoffe hängen im wesentlichen ab von dem Zeitpunkt der Anwendung bezogen auf das Entwicklungsstadium der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in bestimmter gewünschter Weise beeinflussen.

Unter dem Einfluss von Wachstumsregulatoren kann der Blattbestand der Pflanzen so gesteuert werden, dass ein Entblättern der Pflanzen zu einem gewünschten Zeitpunkt erreicht wird. Eine derartige Entlaubung spielt bei der mechanischen Beerntung der Bauwolle eine grosse Rolle ist aber auch in anderen Kulturen wie z.B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Ebenso lässt sich mit Wachstumsregulatoren der Fruchtfall steuern. Einerseits kann ein vorzeitiger Fruchtfall verhindert werden. Andererseits kann aber auch der Fruchtfall oder sogar das Abfallen der Blüten bis zu einem gewünschten Masse gefördert werden ("Ausdünnung"), um die Alternanz zu brechen. Unter Alternanz versteht man die Eigenart einiger Obstarten, endogen bedingt von Jahr zu Jahr sehr unterschiedliche Erträge zu bringen. Schliesslich ist es möglich, mit Wachstumsregulatoren zum Zeitpunkt der Ernte die zum Ablösen der Früchte erforderlichen Kräfte zu reduzierten, um eine mechanische Beerntung zu ermöglichen oder eine manuelle Beerntung zu erleichtern.

Die selektiv-herbizide Wirkung der erfindungsgemässen Verbindungen wird sowohl bei der preemergenten als auch der postemergenten Anwendung festgestellt. Diese Wirkstoffe können daher im Vorauflaufverfahren und im Nachauflaufverfahren zur selektiven Unkrautbekämpfung gleichermassen mit gutem Erfolg verwendet werden.

Die Erfindung betrifft auch herbizide und wuchsregulatorische Mittel, welche einen neuen Wirkstoff der Formel I enthalten, sowie Verfahren zur pre- und postemergenten Unkrautbekämpfung.

In vorteilhafter Weise können die erfindungsgemässen Wirkstoffe oder Mittel auch auf das Vermehrungsgut der Kulturpflanze aufgebracht werden. Besonders zu erwähnen ist hier die Samenbeizung. Vermehrungsgut sind Samen, Stecklinge oder sonstige Teile der Pflanze, aus denen die Kulturpflanze gezogen werden kann. Das mit einer wirksamen Menge einer Verbindung der Formel I behandelte Vermehrungsgut ist ebenfalls Gegenstand der Erfindung.

Die Verbindungen der Formel I werden in unveränderter Form oder vorzugsweise als Mittel zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder -ethylether Ketone wie Cyclohexa non, stark polare Lösungsmittel wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation-und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$-$C_{22}$), wie z.B. die Na-oder K-Salze der Oel- oder Stearinsäure, oder von natürlichen Fettsäuregemischen, die z.B. aus Kokosnuss- oder Talgöl gewonnen werden können, genannt. Ferner sind auch die Fettsäuremethyl-taurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches.

Hierher gehören auch die Salze der Schwefelsäureester und Sulfonsäuren von Fettalkohol-Ethylenoxid-Addukten. Die sulfonierten Benzimidazolderivate enthalten vorzugsweise 2-Sulfonsäuregruppen und einen Fettsäurerest mit 8 bis 22 C-Atomen. Alkylarylsulfonate sind z.B. die Na-, Ca- oder Triethanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Ethylenoxid-Adduktes oder Phospholipide in Frage.

Als nicht ionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 10 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Ethylenglykolethergruppen und 10 bis 100 Propylenglykolethergruppen enthaltenden Polyethylenoxidaddukte an Polypropylenglykol, Ethylendiaminopolypropylenglykol und Alkylpolypropylenglykol mit 1 bis 10 Kohlenstoffatomen in der Alkylkette, die genannten Verbindungen enthalten üblicherweise pro Propylenglykol-Einheit 1 bis 5 Ethylenglykoleinheiten.

Als Beispiel nichtionischer Tenside seien Nonylphenolpolyethoxyethanole, Ricinusölpolyglykolether, Polypropylen-Polyethylenoxidaddukte, Tributylphenoxypolyethoxyethanol, Polyethylenglykol und Octylphenoxypolyethoxyethanol erwähnt.

Ferner kommen auch Fettsäureester von Polyoxyethylensorbitan wie das Polyoxyethylensorbitan-trioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor, z.B. das Stearyltrimethylammoniumchlorid oder das Benzyldi(2-chlorethyl)-ethylammoniumbromid.

Die in der Formulierungstechnik gebräuchlichen Tenside sind u.a. in folgenden Publikationen beschrieben: "1986 International Mc Cutcheon's Emulsifiers & Detergents" Glen Rock, N.J., USA, 1986; H. Stache, "Tensid-Taschenbuch", 2. Auflage., C. Hanser Verlag, München, Wien, 1981;
M. and J. Ash. "Encyclopedia of Surfactants", Vol. I-III, Chemical Publishing Co., New York, 1980-1981.

Die Wirkstoffzubereitungen enthalten in der Regel 0,1 bis 95 %, insbesondere 0,1 bis 80 % Wirkstoff der Formel I, 1 bis 99,9 % eines oder mehrerer fester oder flüssiger Zusatzstoffe und 0 bis 25 % eines Tensides.

Insbesondere setzen sich bevorzugte Formulierungen folgendermassen zusammen: (% = Gewichtsprozent).

Emulgierbare Konzentrate:
Aktiver Wirkstoff: 1 bis 20 %, bevorzugt 5 bis 10 %
oberflächenaktive Mittel: 5 bis 30 %, vorzugsweise 10 bis 20 %
flüssige Trägermittel: 50 bis 94 %, vorzugsweise 70 bis 85 %

Stäube:
Aktiver Wirkstoff: 0,1 bis 10 %, vorzugsweise 0,1 bis 1 %
festes Trägermittel: 99,9 bis 90 %, vorzugsweise 99,9 bis 99 %

Suspensions-Konzentrate:
Aktiver Wirkstoff: 5 bis 75 %, vorzugsweise 10 bis 50 %
Wasser: 94 bis 25 %, vorzugsweise 88 bis 30 %
oberflächenaktives Mittel: 1 bis 40 %, vorzugsweise 2 bis 30 %

Benetzbares Pulver:
Aktiver Wirkstoff: 0,5 bis 90 %, vorzugsweise 1 bis 80 %
oberflächenaktives Mittel: 0,5 bis 20 %, vorzugsweise 1 bis 15 %
festes Trägermittel: 5 bis 95 %, vorzugsweise 15 bis 90 %

Granulate:
Aktiver Wirkstoff: 0,5 bis 30 %, vorzugsweise 3 bis 15 %
festes Trägermittel: 99,5 bis 70 %, vorzugsweise 97 bis 85 %

Während als Handelsware konzentrierte Mittel bevorzugt werden, verwendet der Endverbraucher in der Regel verdünnte Mittel, Die Anwendungsformen können bis hinab zu 0,001 % an Wirkstoff verdünnt werden. Die Aufwandmengen betragen in der Regel 0,001 bis 4 kg AS/ha, vorzugsweise 0,005 bis 1 kg AS/ha.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Die nachfolgenden Beispiel erläutern die Erfindung.

## H. Herstellungsbeispiele

### H 1.1. 2-(4-Chlor-2-fluor-5-isopropyloxyphenyl)-3-hydroxy-3-methyl-1,2,4,5,6,7-hexahydroisoindol-(1H)1-on

Zu 4,0 g Magnesiumspäne in 100 ml wasserfreiem Diethylether werden 80 g Methyljodid so zugetropft, dass der Ether siedet. Nachdem das gesamte Magnesium in Lösung gegangen ist, werden 34,0 g 2-(4-Chlor-2-fluor-5-isopropyloxyphenyl)-4,5,6,7-tetrahydro isoindol-(2H)1,3-dion, gelöst in Diethylether, zugetropft. Es wird dann eine Stunde zum Sieden erhitzt. Nach dem Abkühlen wird das Reaktionsgemisch auf Eis/Wasser gegossen und mit so viel 2N-Salzsäure versetzt, bis sich der zunächst gebildete Niederschlag löst. Danach wird dreimal mit Essigester extrahiert, die Extrakte getrocknet, eingeengt und aus Acetonitril umkristallisiert.

Man isoliert 21 g der Titelverbindung der Formel

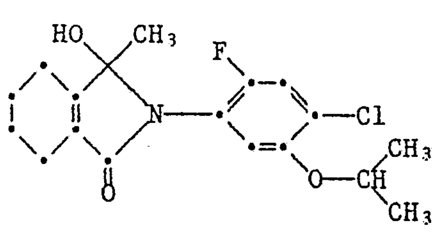

als Kristalle vom Fp. 167°C.

H 1.2. 2-(4-Chlor-2-fluor-5-hydroxyphenyl)-3-hydroxy-3-methyl-1,2,4,5,6,7-hexahydroisoindol-(1H)1-on

Analog zu Herstellungsbeispiel H 1.1. erhält man aus Methylmagnesiumiodid und 2-(4-Chlor-2-fluor-5-hydroxyphenyl)-4,5,6,7-tetrahydroisoindol-2H(1,3)-dion die Titelverbindung der Formel

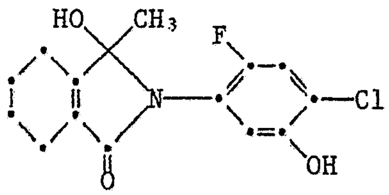

als Kristalle vom Fp. 219-220°C.

H 2.1. 2-(4-Chlor-2-fluor-5-isopropyloxyphenyl)-3-methylen-4,5,6,7-tetrahydroisoindol-(2H)1-on

Eine Mischung aus 6 g 2-[N-(4-Chlor-2-fluor-5-isopropyloxyphenyl)]-2,3,4,5,6,7-hexahydro-3-hydroxy-3-methyl-isoindol-(1H)1-on und 0,2 g Kaliumhydrogensulfat werden unter gutem Rühren für 30 Minuten bei einer Badtemperatur von 140°C erhitzt. Nach dem Abkühlen wird in Methylenchlorid aufgenommen, von anorganischen Teilen abfiltriert und eingedampft. Der Rückstand wird zweimal aus Methanol umkristallisiert.

Man isoliert 2,1 g der Titelverbindung der Formel

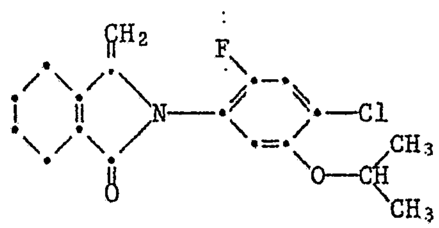

als Kristalle vom Fp. 102-103°C (Verb.No. 1.4).

H 2.2. 2-(4-Chlor-2-fluor-5-hydroxyphenyl)-3-methylen-4,5,6,7-tetrahydroisoindol-(2H)1-on

105 g 2-(4-Chlor-2-fluor-5-hydroxyphenyl)-3-hydroxy-3-methylen-2,3,4,5,6,7-hexahydroisoindol-(1H)1-on werden zusammen mit 10 g p-Toluolsulfonsäure und 30 g eines Molekularsiebes (3 Å) in 1000 ml Dichlormethan 12 Stunden auf 50°C erwärmt. Nach dem Abkühlen wird abfiltriert und das Methylenchlorid im Vakuum abdestilliert. Der Rückstand wird aus Methanol umkristallisiert.

Man isolierrt 45,1 g der Titelverbindung der Formel

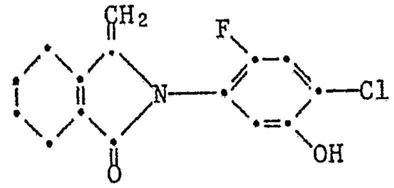

als Kristalle vom Fp. 195-198°C (Verb.No. 1.49).

H 2.3. 2-(4-Chlor-2-fluor-5-propargyloxyphenyl)-3-methylen-4,5,6,7-tetrahydroisoindol-(2H)1-on

5,8 g 2-(4-Chlor-2-fluor-5-hydroxyphenyl)-3-methylen-4,5,6,7-tetrahydroisoindol-(2H)1-on, 5,0 g Kaliumcarbonat und 2,4 g Propargylbromid werden in 100 ml Butan-2-on für 12 Stunden auf 80°C erwärmt. Nach dem Abfiltrieren vom Ungelöstem wird das Filtrat im Vakuum eingeengt.

Man isoliert 5,4 g der Titelverbindung der Formel

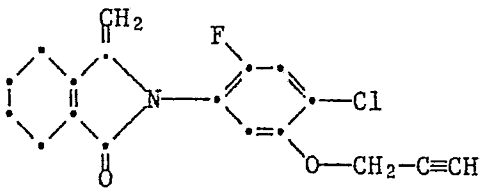

als Kristalle vom Fp. 110-111°C (Verb.No. 1.15).

Analog zu den vorstehenden Herstellungsverfahren können die Verbindungen der Tabellen 1 bis 11 hergestellt werden.

Tabelle 1

Verbindungen der Formel

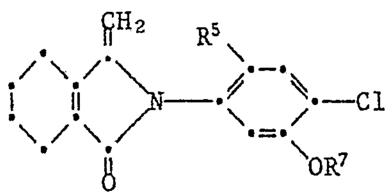

| Nr. | $R^5$ | $R^7$ | Phys. Daten |
|---|---|---|---|
| 1.1 | F | $CH_3$ | |
| 1.2 | F | $C_2H_5$ | |
| 1.3 | F | $C_3H_7$ | |
| 1.4 | F | $C_3H_7$(i) | Fp.:102-103°C |
| 1.5 | F | $C_4H_9$ | |
| 1.6 | F | $C_4H_9$(s) | |
| 1.7 | F | $C_4H_9$(i) | Fp.:120-124°C |
| 1.8 | F | $C_4H_9$(t) | |
| 1.9 | F | $-C_5H_{11}$ | |
| 1.10 | F | $-C_5H_{11}$(s) | |
| 1.11 | F | $-CH_2-CH_2-OCH_3$ | |
| 1.12 | F | $-CH_2-CH=CH_2$ | |
| 1.13 | F | $-CH_2-CCl=CH_2$ | Fp.:144-147°C |
| 1.14 | F | $-CH_2-CH=CHCl$ | Fp.: 90- 95°C |
| 1.15 | F | $-CH_2-C\equiv CH$ | Fp.:110-111°C |
| 1.16 | F | $-CH_2-C_6H_{11}$-(cycl.) | |

Tabelle 1 (Fortsetzung)

| Nr. | $R^5$ | $R^7$ | Phys. Daten |
|---|---|---|---|
| 1.17 | F | $-CH_2-$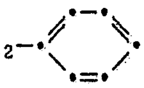 | |
| 1.18 | F | $-CH_2-COOCH_3$ | |
| 1.19 | F | $-CH(CH_3)COOCH_3$ | Fp.:130-134°C |
| 1.20 | F | $-CH(CH_3)COOC_3H_7(i)$ | |
| 1.21 | F | $-CH(CH_3)COOC_4H_9(n)$ | |
| 1.22 | F | $-CH(CH_3)COSCH_3$ | |
| 1.23 | F | $-CH(CH_3)CONH_2$ | |
| 1.24 | F | 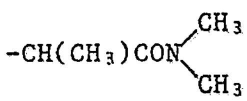 | |
| 1.25 | F | $-COCH_3$ | |
| 1.26 | F | $-COC_6H_5$ | |
| 1.27 | F | $-CH(CH_3)-CH_2-S-CH_3$ | |
| 1.28 | F | 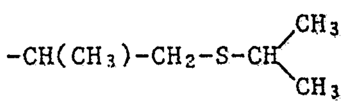 | |
| 1.29 | F | $-CH_2-CH_2-OH$ | |
| 1.30 | F | $-CH_2-CH_2-Cl$ | |
| 1.31 | F | $-CH_2-CN$ | |
| 1.32 | F | 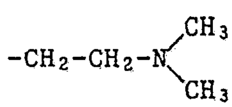 | |
| 1.33 | F | 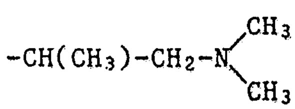 | |
| 1.34 | H | $-CH_3$ | |
| 1.35 | H | $-C_2H_5$ | |
| 1.36 | H | $-C_3H_7$ | |
| 1.37 | H | $-C_3H_7(i)$ | |
| 1.38 | H | $-C_4H_9(i)$ | |
| 1.39 | H | $-C_4H_9(s)$ | |
| 1.40 | H | $-CH_2-CH=CH_2$ | |

Tabelle 1 (Fortsetzung)

| Nr. | $R^5$ | $R^7$ | Phys. Daten |
|---|---|---|---|
| 1.41 | H | $-CH_2-CCl=CH_2$ | |
| 1.42 | H | $-CH_2-CH=CHCl$ | |
| 1.43 | H | $-CH_2-C\equiv CH$ | |
| 1.44 | H | $-CH_2-COOCH_3$ | |
| 1.45 | H | $-CH(CH_3)COOCH_3$ | |
| 1.46 | H | $-CH(CH_3)-CH_2-S-CH_3$ | |
| 1.47 | H | $-CH(CH_3)-CH_2-S-C_2H_5$ | |
| 1.48 | H | $-CH(CH_3)-CH_2-S-C_3H_7$(i) | |
| 1.49 | F | -H | Fp.:195-198°C |
| 1.50 | H | -H | |

Tabelle 2

Verbindungen der Formel

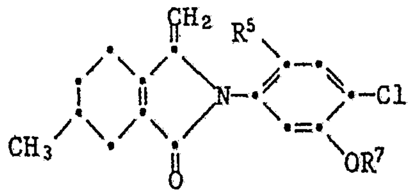

| Nr. | $R^5$ | $R^7$ | Phys. Daten |
|---|---|---|---|
| 2.1 | F | $CH_3$ | |
| 2.2 | F | $C_3H_7i$ | |
| 2.3 | F | $CH_2-CH=CH_2$ | |
| 2.4 | F | $CH_2-CCl=CH_2$ | |
| 2.5 | F | $CH_2-CH=CHCl$ | |
| 2.6 | F | $CH_2-C\equiv CH$ | |
| 2.7 | F | $-CH_2-COOCH_3$ | |
| 2.8 | F | $-CH(CH_3)-COOCH_3$ | |
| 2.9 | F | -H | |

Tabelle 3

Verbindungen der Formel

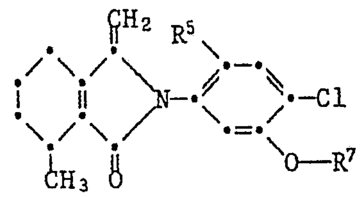

| Nr. | $R^5$ | $R^7$ | Phys. Daten |
|---|---|---|---|
| 3.1 | F | $CH_3$ | |
| 3.2 | F | $C_3H_7i$ | |
| 3.3 | F | $CH_2-CH=CH_2$ | |
| 3.4 | F | $CH_2-CCl=CH_2$ | |
| 3.5 | F | $CH_2-CH=CHCl$ | |
| 3.6 | F | $CH_2-C\equiv CH$ | |
| 3.7 | F | $-CH_2-COOCH_3$ | |
| 3.8 | F | $-CH(CH_3)-COOCH_3$ | |
| 3.9 | F | -H | |

Tabelle 4

Verbindungen der Formel

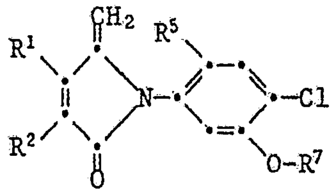

| Nr. | $R^1$ | $R^2$ | $R^5$ | $R^7$ | Phys. Daten |
|---|---|---|---|---|---|
| 4.001 | $CH_3$ | $CH_3$ | F | $CH_3$ | |
| 4.002 | $CH_3$ | $CH_3$ | F | $C_2H_5$ | |
| 4.003 | $CH_3$ | $CH_3$ | F | $C_3H_7$ | |
| 4.004 | $CH_3$ | $CH_3$ | F | $C_3H_7$(i) | Fp. 105-106°C |
| 4.005 | $CH_3$ | $CH_3$ | F | $C_4H_9$ | |
| 4.006 | $CH_3$ | $CH_3$ | F | $C_4H_9$(s) | |
| 4.007 | $CH_3$ | $CH_3$ | F | $C_4H_9$(i) | |
| 4.008 | $CH_3$ | $CH_3$ | F | $C_4H_9$(t) | |
| 4.009 | $CH_3$ | $CH_3$ | F | $-C_5H_{11}$ | |
| 4.010 | $CH_3$ | $CH_3$ | F | $-C_5H_{11}$(s) | |
| 4.011 | $CH_3$ | $CH_3$ | F | $-CH_2-CH_2-OCH_3$ | |
| 4.012 | $CH_3$ | $CH_3$ | F | $-CH_2-CH=CH_2$ | |
| 4.013 | $CH_3$ | $CH_3$ | F | $-CH_2-CCl=CH_2$ | |
| 4.014 | $CH_3$ | $CH_3$ | F | $-CH_2-CH=CHCl$ | |
| 4.015 | $CH_3$ | $CH_3$ | F | $-CH_2-C\equiv CH$ | Fp. 142-143°C |
| 4.016 | $CH_3$ | $CH_3$ | F | $-CH_2-C_6H_{11}$-(cycl.) | |

Tabelle 4 (Fortsetzung)

| Nr. | $R^1$ | $R^2$ | $R^5$ | $R^7$ | Phys. Daten |
|---|---|---|---|---|---|
| 4.017 | $CH_3$ | $CH_3$ | F | $-CH_2-$ 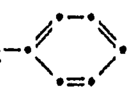 | |
| 4.018 | $CH_3$ | $CH_3$ | F | $-CH_2-COOCH_3$ | |
| 4.019 | $CH_3$ | $CH_3$ | F | $-CH(CH_3)COOCH_3$ | |
| 4.020 | $CH_3$ | $CH_3$ | F | $-CH(CH_3)COOC_3H_7(i)$ | |
| 4.021 | $CH_3$ | $CH_3$ | F | $-CH(CH_3)COOC_4H_9(n)$ | |
| 4.022 | $CH_3$ | $CH_3$ | F | $-CH(CH_3)COSCH_3$ | |
| 4.023 | $CH_3$ | $CH_3$ | F | $-CH(CH_3)CONH_2$ | |
| 4.024 | $CH_3$ | $CH_3$ | F | $CH_3$<br>$-CH(CH_3)CON$<br>$CH_3$ | |
| 4.025 | $CH_3$ | $CH_3$ | F | $-COCH_3$ | |
| 4.026 | $CH_3$ | $CH_3$ | F | $-COC_6H_5$ | |
| 4.027 | $CH_3$ | $CH_3$ | F | $-CH(CH_3)-CH_2-S-CH_3$ | |
| 4.028 | $CH_3$ | $CH_3$ | F | $CH_3$<br>$-CH(CH_3)-CH_2-S-CH$<br>$CH_3$ | |
| 4.029 | $CH_3$ | $CH_3$ | F | $-CH_2-CH_2-OH$ | |
| 4.030 | $CH_3$ | $CH_3$ | F | $-CH_2-CH_2-Cl$ | |
| 4.031 | $CH_3$ | $CH_3$ | F | $-CH_2-CN$ | |
| 4.032 | $CH_3$ | $CH_3$ | F | $CH_3$<br>$-CH_2-CH_2-N$<br>$CH_3$ | |
| 4.033 | $CH_3$ | $CH_3$ | F | $CH_3$<br>$-CH(CH_3)-CH_2-N$<br>$CH_3$ | |
| 4.034 | $CH_3$ | $CH_3$ | H | $-CH_3$ | |
| 4.035 | $CH_3$ | $CH_3$ | H | $-C_2H_5$ | |
| 4.036 | $CH_3$ | $CH_3$ | H | $-C_3H_7$ | |
| 4.037 | $CH_3$ | $CH_3$ | H | $-C_3H_7(i)$ | |
| 4.038 | $CH_3$ | $CH_3$ | H | $-C_4H_9(i)$ | |
| 4.039 | $CH_3$ | $CH_3$ | H | $-C_4H_9(s)$ | |
| 4.040 | $CH_3$ | $CH_3$ | H | $-CH_2-CH=CH_2$ | |

Tabelle 4 (Fortsetzung)

| Nr. | $R^1$ | $R^2$ | $R^5$ | $R^7$ | Phys. Daten |
|---|---|---|---|---|---|
| 4.041 | $CH_3$ | $CH_3$ | H | $-CH_2-CCl=CH_2$ | |
| 4.042 | $CH_3$ | $CH_3$ | H | $-CH_2-CH=CHCl$ | |
| 4.043 | $CH_3$ | $CH_3$ | H | $-CH_2-C\equiv CH$ | |
| 4.044 | $CH_3$ | $CH_3$ | H | $-CH_2-COOCH_3$ | |
| 4.045 | $CH_3$ | $CH_3$ | H | $-CH(CH_3)COOCH_3$ | |
| 4.046 | $CH_3$ | $CH_3$ | H | $-CH(CH_3)-CH_2-S-CH_3$ | |
| 4.047 | $CH_3$ | $CH_3$ | H | $-CH(CH_3)-CH_2-S-C_2H_5$ | |
| 4.048 | $CH_3$ | $CH_3$ | H | $-CH(CH_3)-CH_2-S-C_3H_7(i)$ | |
| 4.049 | $C_2H_5$ | $CH_3$ | F | $CH_3$ | |
| 4.050 | $C_2H_5$ | $CH_3$ | F | $C_2H_5$ | |
| 4.051 | $C_2H_5$ | $CH_3$ | F | $C_3H_7$ | |
| 4.052 | $C_2H_5$ | $CH_3$ | F | $C_3H_7(i)$ | |
| 4.053 | $C_2H_5$ | $CH_3$ | F | $C_4H_9$ | |
| 4.054 | $C_2H_5$ | $CH_3$ | F | $C_4H_9(s)$ | |
| 4.055 | $C_2H_5$ | $CH_3$ | F | $C_4H_9(i)$ | |
| 4.056 | $C_2H_5$ | $CH_3$ | F | $C_4H_9(t)$ | |
| 4.057 | $C_2H_5$ | $CH_3$ | F | $-C_5H_{11}$ | |
| 4.058 | $C_2H_5$ | $CH_3$ | F | $-C_5H_{11}(s)$ | |
| 4.059 | $C_2H_5$ | $CH_3$ | F | $-CH_2-CH_2-OCH_3$ | |
| 4.060 | $C_2H_5$ | $CH_3$ | F | $-CH_2-CH=CH_2$ | |
| 4.061 | $C_2H_5$ | $CH_3$ | F | $-CH_2-CCl=CH_2$ | |
| 4.062 | $C_2H_5$ | $CH_3$ | F | $-CH_2-CH=CHCl$ | |
| 4.063 | $C_2H_5$ | $CH_3$ | F | $-CH_2-C\equiv CH$ | |
| 4.064 | $C_2H_5$ | $CH_3$ | F | $-CH_2-C_6H_{11}-(cycl.)$ | |

Tabelle 4 (Fortsetzung)

| Nr. | $R^1$ | $R^2$ | $R^5$ | $R^7$ | Phys. Daten |
|---|---|---|---|---|---|
| 4.065 | $C_2H_5$ | $CH_3$ | F | $-CH_2-$ 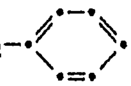 | |
| 4.066 | $C_2H_5$ | $CH_3$ | F | $-CH_2-COOCH_3$ | |
| 4.067 | $C_2H_5$ | $CH_3$ | F | $-CH(CH_3)COOCH_3$ | |
| 4.068 | $C_2H_5$ | $CH_3$ | F | $-CH(CH_3)COOC_3H_7(i)$ | |
| 4.069 | $C_2H_5$ | $CH_3$ | F | $-CH(CH_3)COOC_4H_9(n)$ | |
| 4.070 | $C_2H_5$ | $CH_3$ | F | $-CH(CH_3)COSCH_3$ | |
| 4.071 | $C_2H_5$ | $CH_3$ | F | $-CH(CH_3)CONH_2$ | |
| 4.072 | $C_2H_5$ | $CH_3$ | F | $-CH(CH_3)CON\begin{matrix}CH_3\\CH_3\end{matrix}$ | |
| 4.073 | $C_2H_5$ | $CH_3$ | F | $-COCH_3$ | |
| 4.074 | $C_2H_5$ | $CH_3$ | F | $-COC_6H_5$ | |
| 4.075 | $C_2H_5$ | $CH_3$ | F | $-CH(CH_3)-CH_2-S-CH_3$ | |
| 4.076 | $C_2H_5$ | $CH_3$ | F | $-CH(CH_3)-CH_2-S-CH\begin{matrix}CH_3\\CH_3\end{matrix}$ | |
| 4.077 | $C_2H_5$ | $CH_3$ | F | $-CH_2-CH_2-OH$ | |
| 4.078 | $C_2H_5$ | $CH_3$ | F | $-CH_2-CH_2-Cl$ | |
| 4.079 | $C_2H_5$ | $CH_3$ | F | $-CH_2-CN$ | |
| 4.080 | $C_2H_5$ | $CH_3$ | F | $-CH_2-CH_2-N\begin{matrix}CH_3\\CH_3\end{matrix}$ | |
| 4.081 | $C_2H_5$ | $CH_3$ | F | $-CH(CH_3)-CH_2-N\begin{matrix}CH_3\\CH_3\end{matrix}$ | |
| 4.082 | $C_2H_5$ | $CH_3$ | H | $-CH_3$ | |
| 4.083 | $C_2H_5$ | $CH_3$ | H | $-C_2H_5$ | |
| 4.084 | $C_2H_5$ | $CH_3$ | H | $-C_3H_7$ | |
| 4.085 | $C_2H_5$ | $CH_3$ | H | $-C_3H_7(i)$ | |
| 4.086 | $C_2H_5$ | $CH_3$ | H | $-C_4H_9(i)$ | |
| 4.087 | $C_2H_5$ | $CH_3$ | H | $-C_4H_9(s)$ | |
| 4.088 | $C_2H_5$ | $CH_3$ | H | $-CH_2-CH=CH_2$ | |

Tabelle 4 (Fortsetzung)

| Nr. | $R^1$ | $R^2$ | $R^5$ | $R^7$ | Phys. Daten |
|---|---|---|---|---|---|
| 4.089 | $C_2H_5$ | $CH_3$ | H | $-CH_2-CCl=CH_2$ | |
| 4.090 | $C_2H_5$ | $CH_3$ | H | $-CH_2-CH=CHCl$ | |
| 4.091 | $C_2H_5$ | $CH_3$ | H | $-CH_2-C\equiv CH$ | |
| 4.092 | $C_2H_5$ | $CH_3$ | H | $-CH_2-COOCH_3$ | |
| 4.093 | $C_2H_5$ | $CH_3$ | H | $-CH(CH_3)COOCH_3$ | |
| 4.094 | $C_2H_5$ | $CH_3$ | H | $-CH(CH_3)-CH_2-S-CH_3$ | |
| 4.095 | $C_2H_5$ | $CH_3$ | H | $-CH(CH_3)-CH_2-S-C_2H_5$ | |
| 4.096 | $C_2H_5$ | $CH_3$ | H | $-CH(CH_3)-CH_2-S-C_3H_7(i)$ | |
| 4.097 | $C_2H_5$ | $C_2H_5$ | F | $CH_3$ | |
| 4.098 | $C_2H_5$ | $C_2H_5$ | F | $C_2H_5$ | |
| 4.099 | $C_2H_5$ | $C_2H_5$ | F | $C_3H_7$ | |
| 4.100 | $C_2H_5$ | $C_2H_5$ | F | $C_3H_7(i)$ | |
| 4.101 | $C_2H_5$ | $C_2H_5$ | F | $C_4H_9$ | |
| 4.102 | $C_2H_5$ | $C_2H_5$ | F | $C_4H_9(s)$ | |
| 4.103 | $C_2H_5$ | $C_2H_5$ | F | $C_4H_9(i)$ | |
| 4.104 | $C_2H_5$ | $C_2H_5$ | F | $C_4H_9(t)$ | |
| 4.105 | $C_2H_5$ | $C_2H_5$ | F | $-C_5H_{11}$ | |
| 4.106 | $C_2H_5$ | $C_2H_5$ | F | $-C_5H_{11}(s)$ | |
| 4.107 | $C_2H_5$ | $C_2H_5$ | F | $-CH_2-CH_2-OCH_3$ | |
| 4.108 | $C_2H_5$ | $C_2H_5$ | F | $-CH_2-CH=CH_2$ | |
| 4.109 | $C_2H_5$ | $C_2H_5$ | F | $-CH_2-CCl=CH_2$ | |
| 4.110 | $C_2H_5$ | $C_2H_5$ | F | $-CH_2-CH=CHCl$ | |
| 4.111 | $C_2H_5$ | $C_2H_5$ | F | $-CH_2-C\equiv CH$ | |
| 4.112 | $C_2H_5$ | $C_2H_5$ | F | $-CH_2-C_6H_{11}-(cycl.)$ | |

Tabelle 4 (Fortsetzung)

| Nr. | $R^1$ | $R^2$ | $R^5$ | $R^7$ | Phys. Daten |
|---|---|---|---|---|---|
| 4.113 | $C_2H_5$ | $C_2H_5$ | F | $-CH_2-$ 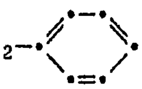 | |
| 4.114 | $C_2H_5$ | $C_2H_5$ | F | $-CH_2-COOCH_3$ | |
| 4.115 | $C_2H_5$ | $C_2H_5$ | F | $-CH(CH_3)COOCH_3$ | |
| 4.116 | $C_2H_5$ | $C_2H_5$ | F | $-CH(CH_3)COOC_3H_7(i)$ | |
| 4.117 | $C_2H_5$ | $C_2H_5$ | F | $-CH(CH_3)COOC_4H_9(n)$ | |
| 4.118 | $C_2H_5$ | $C_2H_5$ | F | $-CH(CH_3)COSCH_3$ | |
| 4.119 | $C_2H_5$ | $C_2H_5$ | F | $-CH(CH_3)CONH_2$ | |
| 4.120 | $C_2H_5$ | $C_2H_5$ | F | $-CH(CH_3)CON\begin{smallmatrix}CH_3\\CH_3\end{smallmatrix}$ | |
| 4.121 | $C_2H_5$ | $C_2H_5$ | F | $-COCH_3$ | |
| 4.122 | $C_2H_5$ | $C_2H_5$ | F | $-COC_6H_5$ | |
| 4.123 | $C_2H_5$ | $C_2H_5$ | F | $-CH(CH_3)-CH_2-S-CH_3$ | |
| 4.124 | $C_2H_5$ | $C_2H_5$ | F | $-CH(CH_3)-CH_2-S-CH\begin{smallmatrix}CH_3\\CH_3\end{smallmatrix}$ | |
| 4.125 | $C_2H_5$ | $C_2H_5$ | F | $-CH_2-CH_2-OH$ | |
| 4.126 | $C_2H_5$ | $C_2H_5$ | F | $-CH_2-CH_2-Cl$ | |
| 4.127 | $C_2H_5$ | $C_2H_5$ | F | $-CH_2-CN$ | |
| 4.128 | $C_2H_5$ | $C_2H_5$ | F | $-CH_2-CH_2-N\begin{smallmatrix}CH_3\\CH_3\end{smallmatrix}$ | |
| 4.129 | $C_2H_5$ | $C_2H_5$ | F | $-CH(CH_3)-CH_2-N\begin{smallmatrix}CH_3\\CH_3\end{smallmatrix}$ | |
| 4.130 | $C_2H_5$ | $C_2H_5$ | H | $-CH_3$ | |
| 4.131 | $C_2H_5$ | $C_2H_5$ | H | $-C_2H_5$ | |
| 4.132 | $C_2H_5$ | $C_2H_5$ | H | $-C_3H_7$ | |

Tabelle 4 (Fortsetzung)

| Nr. | $R^1$ | $R^2$ | $R^5$ | $R^7$ | Phys. Daten |
|---|---|---|---|---|---|
| 4.133 | $C_2H_5$ | $C_2H_5$ | H | $-C_3H_7(i)$ | |
| 4.134 | $C_2H_5$ | $C_2H_5$ | H | $-C_4H_9(i)$ | |
| 4.135 | $C_2H_5$ | $C_2H_5$ | H | $-C_4H_9(s)$ | |
| 4.136 | $C_2H_5$ | $C_2H_5$ | H | $-CH_2-CH=CH_2$ | |
| 4.137 | $C_2H_5$ | $C_2H_5$ | H | $-CH_2-CCl=CH_2$ | |
| 4.138 | $C_2H_5$ | $C_2H_5$ | H | $-CH_2-CH=CHCl$ | |
| 4.139 | $C_2H_5$ | $C_2H_5$ | H | $-CH_2-C\equiv CH$ | |
| 4.140 | $C_2H_5$ | $C_2H_5$ | H | $-CH_2-COOCH_3$ | |
| 4.141 | $C_2H_5$ | $C_2H_5$ | H | $-CH(CH_3)COOCH_3$ | |
| 4.142 | $C_2H_5$ | $C_2H_5$ | H | $-CH(CH_3)-CH_2-S-CH_3$ | |
| 4.143 | $C_2H_5$ | $C_2H_5$ | H | $-CH(CH_3)-CH_2-S-C_2H_5$ | |
| 4.144 | $C_2H_5$ | $C_2H_5$ | H | $-CH(CH_3)-CH_2-S-C_3H_7(i)$ | |
| 4.145 | $CH_3$ | $CH_3$ | F | H | Fp. 203-204°C |
| 4.146 | $CH_3$ | $CH_3$ | H | H | |
| 4.147 | $C_2H_5$ | $CH_3$ | F | H | |
| 4.148 | $C_2H_5$ | $CH_3$ | H | H | |
| 4.149 | $C_2H_5$ | $C_2H_5$ | F | H | |
| 4.150 | $C_2H_5$ | $C_2H_5$ | H | H | |
| 4.151 | $CH_3$ | $C_2H_5$ | F | H | |
| 4.152 | $CH_3$ | $C_2H_5$ | H | H | |
| 4.153 | $CH_3$ | $C_2H_5$ | F | $CH_3$ | |
| 4.154 | $CH_3$ | $C_2H_5$ | F | $C_2H_5$ | |
| 4.155 | $CH_3$ | $C_2H_5$ | F | $C_3H_7$ | |
| 4.156 | $CH_3$ | $C_2H_5$ | F | $C_3H_7(i)$ | |
| 4.157 | $CH_3$ | $C_2H_5$ | F | $C_4H_9$ | |
| 4.158 | $CH_3$ | $C_2H_5$ | F | $C_4H_9(s)$ | |
| 4.159 | $CH_3$ | $C_2H_5$ | F | $C_4H_9(i)$ | |
| 4.160 | $CH_3$ | $C_2H_5$ | F | $C_4H_9(t)$ | |
| 4.161 | $CH_3$ | $C_2H_5$ | F | $-C_5H_{11}$ | |
| 4.162 | $CH_3$ | $C_2H_5$ | F | $-C_5H_{11}(s)$ | |
| 4.163 | $CH_3$ | $C_2H_5$ | F | $-CH_2-CH_2-OCH_3$ | |
| 4.164 | $CH_3$ | $C_2H_5$ | F | $-CH_2-CH=CH_2$ | |
| 4.165 | $CH_3$ | $C_2H_5$ | F | $-CH_2-CCl=CH_2$ | |
| 4.166 | $CH_3$ | $C_2H_5$ | F | $-CH_2-CH=CHCl$ | |
| 4.167 | $CH_3$ | $C_2H_5$ | F | $-CH_2-C\equiv CH$ | |
| 4.168 | $CH_3$ | $C_2H_5$ | F | $-CH_2-C_6H_{11}-(cycl.)$ | |

Tabelle 4 (Fortsetzung)

| Nr. | $R^1$ | $R^2$ | $R^5$ | $R^7$ | Phys. Daten |
|---|---|---|---|---|---|
| 4.169 | $CH_3$ | $C_2H_5$ | F | $-CH_2-$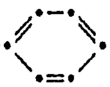 | |
| 4.170 | $CH_3$ | $C_2H_5$ | F | $-CH_2-COOCH_3$ | |
| 4.171 | $CH_3$ | $C_2H_5$ | F | $-CH(CH_3)COOCH_3$ | |
| 4.172 | $CH_3$ | $C_2H_5$ | F | $-CH(CH_3)COOC_3H_7(i)$ | |
| 4.173 | $CH_3$ | $C_2H_5$ | F | $-CH(CH_3)COOC_4H_9(n)$ | |
| 4.174 | $CH_3$ | $C_2H_5$ | F | $-CH(CH_3)COSCH_3$ | |
| 4.175 | $CH_3$ | $C_2H_5$ | F | $-CH(CH_3)CONH_2$ | |
| 4.176 | $CH_3$ | $C_2H_5$ | F | $-CH(CH_3)CON{<}^{CH_3}_{CH_3}$ | |
| 4.177 | $CH_3$ | $C_2H_5$ | F | $-COCH_3$ | |
| 4.178 | $CH_3$ | $C_2H_5$ | F | $-COC_6H_5$ | |
| 4.179 | $CH_3$ | $C_2H_5$ | F | $-CH(CH_3)-CH_2-S-CH_3$ | |
| 4.180 | $CH_3$ | $C_2H_5$ | F | $-CH(CH_3)-CH_2-S-CH{<}^{CH_3}_{CH_3}$ | |
| 4.181 | $CH_3$ | $C_2H_5$ | F | $-CH_2-CH_2-OH$ | |
| 4.182 | $CH_3$ | $C_2H_5$ | F | $-CH_2-CH_2-Cl$ | |
| 4.183 | $CH_3$ | $C_2H_5$ | F | $-CH_2-CN$ | |
| 4.184 | $CH_3$ | $C_2H_5$ | F | $-CH_2-CH_2-N{<}^{CH_3}_{CH_3}$ | |
| 4.185 | $CH_3$ | $C_2H_5$ | F | $-CH(CH_3)-CH_2-N{<}^{CH_3}_{CH_3}$ | |
| 4.186 | $CH_3$ | $C_2H_5$ | H | $-CH_3$ | |
| 4.187 | $CH_3$ | $C_2H_5$ | H | $-C_2H_5$ | |
| 4.188 | $CH_3$ | $C_2H_5$ | H | $-C_3H_7$ | |
| 4.189 | $CH_3$ | $C_2H_5$ | H | $-C_3H_7(i)$ | |
| 4.190 | $CH_3$ | $C_2H_5$ | H | $-C_4H_9(i)$ | |
| 4.191 | $CH_3$ | $C_2H_5$ | H | $-C_4H_9(s)$ | |
| 4.192 | $CH_3$ | $C_2H_5$ | H | $-CH_2-CH=CH_2$ | |

Tabelle 4 (Fortsetzung)

| Nr. | $R^1$ | $R^2$ | $R^5$ | $R^7$ | Phys. Daten |
|---|---|---|---|---|---|
| 4.193 | $CH_3$ | $C_2H_5$ | H | $-CH_2-CCl=CH_2$ | |
| 4.194 | $CH_3$ | $C_2H_5$ | H | $-CH_2-CH=CHCl$ | |
| 4.195 | $CH_3$ | $C_2H_5$ | H | $-CH_2-C\equiv CH$ | |
| 4.196 | $CH_3$ | $C_2H_5$ | H | $-CH_2-COOCH_3$ | |
| 4.197 | $CH_3$ | $C_2H_5$ | H | $-CH(CH_3)COOCH_3$ | |
| 4.198 | $CH_3$ | $C_2H_5$ | H | $-CH(CH_3)-CH_2-S-CH_3$ | |
| 4.199 | $CH_3$ | $C_2H_5$ | H | $-CH(CH_3)-CH_2-S-C_2H_5$ | |
| 4.200 | $CH_3$ | $C_2H_5$ | H | $-CH(CH_3)-CH_2-S-C_3H_7(i)$ | |

Tabelle 5:

Verbindungen der Formel

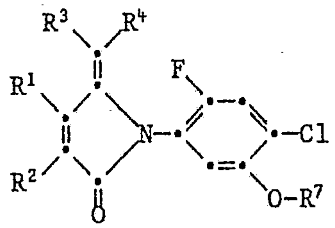

| Nr. | $R^1$ | $R^2$ | $R^3$ | $R^4$ | $R^7$ | Phys. Daten |
|---|---|---|---|---|---|---|
| 5.001 | $-(CH_2)_4-$ | | $CH_3$ | $CH_3$ | $CH_3$ | |
| 5.002 | $-(CH_2)_4-$ | | $CH_3$ | $CH_3$ | $C_3H_7(i)$ | Fp. 101-103°C |
| 5.003 | $-(CH_2)_4-$ | | $CH_3$ | $CH_3$ | $CH_2-CH=CH_2$ | |
| 5.004 | $-(CH_2)_4-$ | | $CH_3$ | $CH_3$ | $CH_2-CCl=CH_2$ | |
| 5.005 | $-(CH_2)_4-$ | | $CH_3$ | $CH_3$ | $CH_2-CH=CHCl$ | |
| 5.006 | $-(CH_2)_4-$ | | $CH_3$ | $CH_3$ | $CH_2-C\equiv CH$ | |
| 5.007 | $-(CH_2)_4-$ | | $CH_3$ | $CH_3$ | $CH_2-COOCH_3$ | |
| 5.008 | $-(CH_2)_4-$ | | $CH_3$ | $CH_3$ | $CH(CH_3)-COOCH_3$ | |
| 5.009 | $-(CH_2)_4-$ | | $CH_3$ | $CH_3$ | H | |
| 5.010 | $-(CH_2)_4-$ | | H | $CH_3$ | $CH_3$ | |
| 5.011 | $-(CH_2)_4-$ | | H | $CH_3$ | $C_3H_7(i)$ | $n_D^{21}$: 1,5472 |
| 5.012 | $-(CH_2)_4-$ | | H | $CH_3$ | $CH_2-CH=CH_2$ | |
| 5.013 | $-(CH_2)_4-$ | | H | $CH_3$ | $CH_2-CCl=CH_2$ | |
| 5.014 | $-(CH_2)_4-$ | | H | $CH_3$ | $CH_2-CH=CHCl$ | |
| 5.015 | $-(CH_2)_4-$ | | H | $CH_3$ | $CH_2-C\equiv CH$ | |
| 5.016 | $-(CH_2)_4-$ | | H | $CH_3$ | $CH_2-COOCH_3$ | |
| 5.017 | $-(CH_2)_4-$ | | H | $CH_3$ | $CH(CH_3)-COOCH_3$ | |
| 5.018 | $-(CH_2)_4-$ | | H | $CH_3$ | H | |
| 5.019 | $-(CH_2)_4-$ | | $CH_3$ | H | $CH_3$ | |
| 5.020 | $-(CH_2)_4-$ | | $CH_3$ | H | $C_3H_7(i)$ | |
| 5.021 | $-(CH_2)_4-$ | | $CH_3$ | H | $CH_2-CH=CH_2$ | |
| 5.022 | $-(CH_2)_4-$ | | $CH_3$ | H | $CH_2-CCl=CH_2$ | |
| 5.023 | $-(CH_2)_4-$ | | $CH_3$ | H | $CH_2-CH=CHCl$ | |
| 5.024 | $-(CH_2)_4-$ | | $CH_3$ | H | $CH_2-C\equiv CH$ | |
| 5.025 | $-(CH_2)_4-$ | | $CH_3$ | H | $CH_2-COOCH_3$ | |
| 5.026 | $-(CH_2)_4-$ | | $CH_3$ | H | $CH(CH_3)-COOCH_3$ | |
| 5.027 | $-(CH_2)_4-$ | | $CH_3$ | H | H | |
| 5.028 | $CH_3$ | $CH_3$ | $CH_3$ | $CH_3$ | $CH_3$ | |
| 5.029 | $CH_3$ | $CH_3$ | $CH_3$ | $CH_3$ | $C_3H_7(i)$ | |
| 5.030 | $CH_3$ | $CH_3$ | $CH_3$ | $CH_3$ | $CH_2-CH=CH_2$ | |
| 5.031 | $CH_3$ | $CH_3$ | $CH_3$ | $CH_3$ | $CH_2-CCl=CH_2$ | |
| 5.032 | $CH_3$ | $CH_3$ | $CH_3$ | $CH_3$ | $CH_2-CH=CHCl$ | |
| 5.033 | $CH_3$ | $CH_3$ | $CH_3$ | $CH_3$ | $CH_2-C\equiv CH$ | |
| 5.034 | $CH_3$ | $CH_3$ | $CH_3$ | $CH_3$ | $CH_2-COOCH_3$ | |
| 5.035 | $CH_3$ | $CH_3$ | $CH_3$ | $CH_3$ | $CH(CH_3)-COOCH_3$ | |

| Nr. | $R^1$ | $R^2$ | $R^3$ | $R^4$ | $R^7$ | Phys. Daten |
|---|---|---|---|---|---|---|
| 5.036 | $CH_3$ | $CH_3$ | $CH_3$ | $CH_3$ | H | |
| 5.037 | $CH_3$ | $CH_3$ | H | $CH_3$ | $CH_3$ | |
| 5.038 | $CH_3$ | $CH_3$ | H | $CH_3$ | $C_3H_7(i)$ | |
| 5.039 | $CH_3$ | $CH_3$ | H | $CH_3$ | $CH_2-CH=CH_2$ | |
| 5.040 | $CH_3$ | $CH_3$ | H | $CH_3$ | $CH_2-CCl=CH_2$ | |
| 5.041 | $CH_3$ | $CH_3$ | H | $CH_3$ | $CH_2-CH=CHCl$ | |
| 5.042 | $CH_3$ | $CH_3$ | H | $CH_3$ | $CH_2-C{\equiv}CH$ | |
| 5.043 | $CH_3$ | $CH_3$ | H | $CH_3$ | $CH_2-COOCH_3$ | |
| 5.044 | $CH_3$ | $CH_3$ | H | $CH_3$ | $CH(CH_3)-COOCH_3$ | |
| 5.045 | $CH_3$ | $CH_3$ | H | $CH_3$ | H | |
| 5.046 | $CH_3$ | $CH_3$ | $CH_3$ | H | $CH_3$ | |
| 5.047 | $CH_3$ | $CH_3$ | $CH_3$ | H | $C_3H_7(i)$ | |
| 5.048 | $CH_3$ | $CH_3$ | $CH_3$ | H | $CH_2-CH=CH_2$ | |
| 5.049 | $CH_3$ | $CH_3$ | $CH_3$ | H | $CH_2-CCl=CH_2$ | |
| 5.050 | $CH_3$ | $CH_3$ | $CH_3$ | H | $CH_2-CH=CHCl$ | |
| 5.051 | $CH_3$ | $CH_3$ | $CH_3$ | H | $CH_2-C{\equiv}CH$ | |
| 5.052 | $CH_3$ | $CH_3$ | $CH_3$ | H | $CH_2-COOCH_3$ | |
| 5.053 | $CH_3$ | $CH_3$ | $CH_3$ | H | $CH(CH_3)-COOCH_3$ | |
| 5.054 | $CH_3$ | $CH_3$ | $CH_3$ | H | H | |
| 5.055 | $C_2H_5$ | $C_2H_5$ | $CH_3$ | $CH_3$ | $CH_3$ | |
| 5.056 | $C_2H_5$ | $C_2H_5$ | $CH_3$ | $CH_3$ | $C_3H_7(i)$ | |
| 5.057 | $C_2H_5$ | $C_2H_5$ | $CH_3$ | $CH_3$ | $CH_2-CH=CH_2$ | |
| 5.058 | $C_2H_5$ | $C_2H_5$ | $CH_3$ | $CH_3$ | $CH_2-CCl=CH_2$ | |
| 5.059 | $C_2H_5$ | $C_2H_5$ | $CH_3$ | $CH_3$ | $CH_2-CH=CHCl$ | |
| 5.060 | $C_2H_5$ | $C_2H_5$ | $CH_3$ | $CH_3$ | $CH_2-C{\equiv}CH$ | |
| 5.061 | $C_2H_5$ | $C_2H_5$ | $CH_3$ | $CH_3$ | $CH_2-COOCH_3$ | |
| 5.062 | $C_2H_5$ | $C_2H_5$ | $CH_3$ | $CH_3$ | $CH(CH_3)-COOCH_3$ | |
| 5.063 | $C_2H_5$ | $C_2H_5$ | $CH_3$ | $CH_3$ | H | |
| 5.064 | $C_2H_5$ | $C_2H_5$ | H | $CH_3$ | $CH_3$ | |
| 5.065 | $C_2H_5$ | $C_2H_5$ | H | $CH_3$ | $C_3H_7(i)$ | |
| 5.066 | $C_2H_5$ | $C_2H_5$ | H | $CH_3$ | $CH_2-CH=CH_2$ | |
| 5.067 | $C_2H_5$ | $C_2H_5$ | H | $CH_3$ | $CH_2-CCl=CH_2$ | |
| 5.068 | $C_2H_5$ | $C_2H_5$ | H | $CH_3$ | $CH_2-CH=CHCl$ | |
| 5.069 | $C_2H_5$ | $C_2H_5$ | H | $CH_3$ | $CH_2-C{\equiv}CH$ | |
| 5.070 | $C_2H_5$ | $C_2H_5$ | H | $CH_3$ | $CH_2-COOCH_3$ | |
| 5.071 | $C_2H_5$ | $C_2H_5$ | H | $CH_3$ | $CH(CH_3)-COOCH_3$ | |
| 5.072 | $C_2H_5$ | $C_2H_5$ | H | $CH_3$ | H | |
| 5.073 | $C_2H_5$ | $C_2H_5$ | $CH_3$ | H | $CH_3$ | |
| 5.074 | $C_2H_5$ | $C_2H_5$ | $CH_3$ | H | $C_3H_7(i)$ | |
| 5.075 | $C_2H_5$ | $C_2H_5$ | $CH_3$ | H | $CH_2-CH=CH_2$ | |
| 5.076 | $C_2H_5$ | $C_2H_5$ | $CH_3$ | H | $CH_2-CCl=CH_2$ | |
| 5.077 | $C_2H_5$ | $C_2H_5$ | $CH_3$ | H | $CH_2-CH=CHCl$ | |
| 5.078 | $C_2H_5$ | $C_2H_5$ | $CH_3$ | H | $CH_2-C{\equiv}CH$ | |
| 5.079 | $C_2H_5$ | $C_2H_5$ | $CH_3$ | H | $CH_2-COOCH_3$ | |
| 5.080 | $C_2H_5$ | $C_2H_5$ | $CH_3$ | H | $CH(CH_3)-COOCH_3$ | |
| 5.081 | $C_2H_5$ | $C_2H_5$ | $CH_3$ | H | H | |

| Nr. | $R^1$ | $R^2$ | $R^3$ | $R^4$ | $R^7$ | Phys. Daten |
|---|---|---|---|---|---|---|
| 5.082 | $-(CH_2)_4-$ | | $-(CH_2)_4-$ | | $CH_3$ | |
| 5.083 | $-(CH_2)_4-$ | | $-(CH_2)_4-$ | | $C_3H_7(i)$ | |
| 5.084 | $-(CH_2)_4-$ | | $-(CH_2)_4-$ | | $CH_2-CH=CH_2$ | |
| 5.085 | $-(CH_2)_4-$ | | $-(CH_2)_4-$ | | $CH_2-CCl=CH_2$ | |
| 5.086 | $-(CH_2)_4-$ | | $-(CH_2)_4-$ | | $CH_2-CH=CHCl$ | |
| 5.087 | $-(CH_2)_4-$ | | $-(CH_2)_4-$ | | $CH_2-C\equiv CH$ | |
| 5.088 | $-(CH_2)_4-$ | | $-(CH_2)_4-$ | | $CH_2-COOCH_3$ | |
| 5.089 | $-(CH_2)_4-$ | | $-(CH_2)_4-$ | | $CH(CH_3)-COOCH_3$ | |
| 5.090 | $-(CH_2)_4-$ | | $-(CH_2)_4-$ | | H | |
| 5.091 | $-(CH_2)_4-$ | | $-(CH_2)_5-$ | | $CH_3$ | |
| 5.092 | $-(CH_2)_4-$ | | $-(CH_2)_5-$ | | $C_3H_7(i)$ | |
| 5.093 | $-(CH_2)_4-$ | | $-(CH_2)_5-$ | | $CH_2-CH=CH_2$ | |
| 5.094 | $-(CH_2)_4-$ | | $-(CH_2)_5-$ | | $CH_2-CCl=CH_2$ | |
| 5.095 | $-(CH_2)_4-$ | | $-(CH_2)_5-$ | | $CH_2-CH=CHCl$ | |
| 5.096 | $-(CH_2)_4-$ | | $-(CH_2)_5-$ | | $CH_2-C\equiv CH$ | |
| 5.097 | $-(CH_2)_4-$ | | $-(CH_2)_5-$ | | $CH_2-COOCH_3$ | |
| 5.098 | $-(CH_2)_4-$ | | $-(CH_2)_5-$ | | $CH(CH_3)-COOCH_3$ | |
| 5.099 | $-(CH_2)_4-$ | | $-(CH_2)_5-$ | | H | |
| 5.100 | $CH_3$ | $CH_3$ | $-(CH_2)_4-$ | | $CH_3$ | |
| 5.101 | $CH_3$ | $CH_3$ | $-(CH_2)_4-$ | | $C_3H_7(i)$ | |
| 5.102 | $CH_3$ | $CH_3$ | $-(CH_2)_4-$ | | $CH_2-CH=CH_2$ | |
| 5.103 | $CH_3$ | $CH_3$ | $-(CH_2)_4-$ | | $CH_2-CCl=CH_2$ | |
| 5.104 | $CH_3$ | $CH_3$ | $-(CH_2)_4-$ | | $CH_2-CH=CHCl$ | |
| 5.105 | $CH_3$ | $CH_3$ | $-(CH_2)_4-$ | | $CH_2-C\equiv CH$ | |
| 5.106 | $CH_3$ | $CH_3$ | $-(CH_2)_4-$ | | $CH_2-COOCH_3$ | |
| 5.107 | $CH_3$ | $CH_3$ | $-(CH_2)_4-$ | | $CH(CH_3)-COOCH_3$ | |
| 5.108 | $CH_3$ | $CH_3$ | $-(CH_2)_4-$ | | H | |
| 5.109 | $CH_3$ | $CH_3$ | $-(CH_2)_5-$ | | $CH_3$ | |
| 5.110 | $CH_3$ | $CH_3$ | $-(CH_2)_5-$ | | $C_3H_7(i)$ | |
| 5.111 | $CH_3$ | $CH_3$ | $-(CH_2)_5-$ | | $CH_2-CH=CH_2$ | |
| 5.112 | $CH_3$ | $CH_3$ | $-(CH_2)_5-$ | | $CH_2-CCl=CH_2$ | |
| 5.113 | $CH_3$ | $CH_3$ | $-(CH_2)_5-$ | | $CH_2-CH=CHCl$ | |
| 5.114 | $CH_3$ | $CH_3$ | $-(CH_2)_5-$ | | $CH_2-C\equiv CH$ | |

| Nr. | $R^1$ | $R^2$ | $R^3$ | $R^4$ | $R^7$ | Phys. Daten |
|---|---|---|---|---|---|---|
| 5.115 | $CH_3$ | $CH_3$ | $-(CH_2)_5-$ | | $CH_2-COOCH_3$ | |
| 5.116 | $CH_3$ | $CH_3$ | $-(CH_2)_5-$ | | $CH(CH_3)-COOCH_3$ | |
| 5.117 | $CH_3$ | $CH_3$ | $-(CH_2)_5-$ | | H | |
| 5.118 | $C_2H_5$ | $C_2H_5$ | $-(CH_2)_4-$ | | $CH_3$ | |
| 5.119 | $C_2H_5$ | $C_2H_5$ | $-(CH_2)_4-$ | | $C_3H_7(i)$ | |
| 5.120 | $C_2H_5$ | $C_2H_5$ | $-(CH_2)_4-$ | | $CH_2-CH=CH_2$ | |
| 5.121 | $C_2H_5$ | $C_2H_5$ | $-(CH_2)_4-$ | | $CH_2-CCl=CH_2$ | |
| 5.122 | $C_2H_5$ | $C_2H_5$ | $-(CH_2)_4-$ | | $CH_2-CH=CHCl$ | |
| 5.123 | $C_2H_5$ | $C_2H_5$ | $-(CH_2)_4-$ | | $CH_2-C\equiv CH$ | |
| 5.124 | $C_2H_5$ | $C_2H_5$ | $-(CH_2)_4-$ | | $CH_2-COOCH_3$ | |
| 5.125 | $C_2H_5$ | $C_2H_5$ | $-(CH_2)_4-$ | | $CH(CH_3)-COOCH_3$ | |
| 5.126 | $C_2H_5$ | $C_2H_5$ | $-(CH_2)_4-$ | | H | |
| 5.127 | $C_2H_5$ | $C_2H_5$ | $-(CH_2)_5-$ | | $CH_3$ | |
| 5.128 | $C_2H_5$ | $C_2H_5$ | $-(CH_2)_5-$ | | $C_3H_7(i)$ | |
| 5.129 | $C_2H_5$ | $C_2H_5$ | $-(CH_2)_5-$ | | $CH_2-CH=CH_2$ | |
| 5.130 | $C_2H_5$ | $C_2H_5$ | $-(CH_2)_5-$ | | $CH_2-CCl=CH_2$ | |
| 5.131 | $C_2H_5$ | $C_2H_5$ | $-(CH_2)_5-$ | | $CH_2-CH=CHCl$ | |
| 5.132 | $C_2H_5$ | $C_2H_5$ | $-(CH_2)_5-$ | | $CH_2-C\equiv CH$ | |
| 5.133 | $C_2H_5$ | $C_2H_5$ | $-(CH_2)_5-$ | | $CH_2-COOCH_3$ | |
| 5.134 | $C_2H_5$ | $C_2H_5$ | $-(CH_2)_5-$ | | $CH(CH_3)-COOCH_3$ | |
| 5.135 | $C_2H_5$ | $C_2H_5$ | $-(CH_2)_5-$ | | H | |
| 5.136 | $CH_3$ | $CH_3$ | H | $C_6H_5$ | $CH_3$ | |
| 5.137 | $CH_3$ | $CH_3$ | H | $C_6H_5$ | $C_3H_7(i)$ | |
| 5.138 | $CH_3$ | $CH_3$ | H | $C_6H_5$ | $CH_2-CH=CH_2$ | |
| 5.139 | $CH_3$ | $CH_3$ | H | $C_6H_5$ | $CH_2-CCl=CH_2$ | |
| 5.140 | $CH_3$ | $CH_3$ | H | $C_6H_5$ | $CH_2-CH=CHCl$ | |
| 5.141 | $CH_3$ | $CH_3$ | H | $C_6H_5$ | $CH_2-C\equiv CH$ | Fp.103-106°C |
| 5.142 | $CH_3$ | $CH_3$ | H | $C_6H_5$ | $CH_2-COOCH_3$ | |
| 5.143 | $CH_3$ | $CH_3$ | H | $C_6H_5$ | $CH(CH_3)-COOCH_3$ | |
| 5.144 | $CH_3$ | $CH_3$ | H | $C_6H_5$ | H | |

| Nr. | $R^1$ | $R^2$ | $R^3$ | $R^4$ | $R^7$ | Phys. Daten |
|---|---|---|---|---|---|---|
| 5.145 | $CH_3$ | $CH_3$ | H | o-Cl-$C_6H_4$ | $CH_3$ | |
| 5.146 | $CH_3$ | $CH_3$ | H | o-Cl-$C_6H_4$ | $C_3H_7$(i) | |
| 5.147 | $CH_3$ | $CH_3$ | H | o-Cl-$C_6H_4$ | $CH_2$-CH=$CH_2$ | |
| 5.148 | $CH_3$ | $CH_3$ | H | o-Cl-$C_6H_4$ | $CH_2$-CCl=$CH_2$ | |
| 5.149 | $CH_3$ | $CH_3$ | H | o-Cl-$C_6H_4$ | $CH_2$-CH=CHCl | |
| 5.150 | $CH_3$ | $CH_3$ | H | o-Cl-$C_6H_4$ | $CH_2$-C≡CH | |
| 5.151 | $CH_3$ | $CH_3$ | H | o-Cl-$C_6H_4$ | $CH_2$-$COOCH_3$ | |
| 5.152 | $CH_3$ | $CH_3$ | H | o-Cl-$C_6H_4$ | CH($CH_3$)-$COOCH_3$ | |
| 5.153 | $CH_3$ | $CH_3$ | H | o-Cl-$C_6H_4$ | H | |
| 5.154 | $CH_3$ | $CH_3$ | H | m-Cl-$C_6H_4$ | $CH_3$ | |
| 5.155 | $CH_3$ | $CH_3$ | H | m-Cl-$C_6H_4$ | $C_3H_7$(i) | $n_D^{23}$:1,5779 |
| 5.156 | $CH_3$ | $CH_3$ | H | m-Cl-$C_6H_4$ | $CH_2$-CH=$CH_2$ | |
| 5.157 | $CH_3$ | $CH_3$ | H | m-Cl-$C_6H_4$ | $CH_2$-CCl=$CH_2$ | |
| 5.158 | $CH_3$ | $CH_3$ | H | m-Cl-$C_6H_4$ | $CH_2$-CH=CHCl | |
| 5.159 | $CH_3$ | $CH_3$ | H | m-Cl-$C_6H_4$ | $CH_2$-C≡CH | |
| 5.160 | $CH_3$ | $CH_3$ | H | m-Cl-$C_6H_4$ | $CH_2$-$COOCH_3$ | |
| 5.161 | $CH_3$ | $CH_3$ | H | m-Cl-$C_6H_4$ | CH($CH_3$)-$COOCH_3$ | |
| 5.162 | $CH_3$ | $CH_3$ | H | m-Cl-$C_6H_4$ | H | |
| 5.163 | $CH_3$ | $CH_3$ | H | p-Cl-$C_6H_4$ | $CH_3$ | |
| 5.164 | $CH_3$ | $CH_3$ | H | p-Cl-$C_6H_4$ | $C_3H_7$(i) | |
| 5.165 | $CH_3$ | $CH_3$ | H | p-Cl-$C_6H_4$ | $CH_2$-CH=$CH_2$ | |
| 5.166 | $CH_3$ | $CH_3$ | H | p-Cl-$C_6H_4$ | $CH_2$-CCl=$CH_2$ | |
| 5.167 | $CH_3$ | $CH_3$ | H | p-Cl-$C_6H_4$ | $CH_2$-CH=CHCl | |
| 5.168 | $CH_3$ | $CH_3$ | H | p-Cl-$C_6H_4$ | $CH_2$-C≡CH | |
| 5.169 | $CH_3$ | $CH_3$ | H | p-Cl-$C_6H_4$ | $CH_2$-$COOCH_3$ | |
| 5.170 | $CH_3$ | $CH_3$ | H | p-Cl-$C_6H_4$ | CH($CH_3$)-$COOCH_3$ | |
| 5.171 | $CH_3$ | $CH_3$ | H | p-Cl-$C_6H_4$ | H | |
| 5.172 | $CH_3$ | $CH_3$ | H | o-$CH_3$-$C_6H_4$ | $CH_3$ | |
| 5.173 | $CH_3$ | $CH_3$ | H | o-$CH_3$-$C_6H_4$ | $C_3H_7$(i) | |
| 5.174 | $CH_3$ | $CH_3$ | H | o-$CH_3$-$C_6H_4$ | $CH_2$-CH=$CH_2$ | |
| 5.175 | $CH_3$ | $CH_3$ | H | o-$CH_3$-$C_6H_4$ | $CH_2$-CCl=$CH_2$ | |

| Nr. | $R^1$ | $R^2$ | $R^3$ | $R^4$ | $R^7$ | Phys. Daten |
|---|---|---|---|---|---|---|
| 5.176 | $CH_3$ | $CH_3$ | H | o-$CH_3$-$C_6H_4$ | $CH_2$-CH=CHCl | |
| 5.177 | $CH_3$ | $CH_3$ | H | o-$CH_3$-$C_6H_4$ | $CH_2$-C≡CH | |
| 5.178 | $CH_3$ | $CH_3$ | H | o-$CH_3$-$C_6H_4$ | $CH_2$-$COOCH_3$ | |
| 5.179 | $CH_3$ | $CH_3$ | H | o-$CH_3$-$C_6H_4$ | CH($CH_3$)-$COOCH_3$ | |
| 5.180 | $CH_3$ | $CH_3$ | H | o-$CH_3$-$C_6H_4$ | H | |
| 5.181 | $CH_3$ | $CH_3$ | H | m-$CH_3$-$C_6H_4$ | $CH_3$ | |
| 5.182 | $CH_3$ | $CH_3$ | H | m-$CH_3$-$C_6H_4$ | $C_3H_7$(i) | |
| 5.183 | $CH_3$ | $CH_3$ | H | m-$CH_3$-$C_6H_4$ | $CH_2$-CH=$CH_2$ | |
| 5.184 | $CH_3$ | $CH_3$ | H | m-$CH_3$-$C_6H_4$ | $CH_2$-CCl=$CH_2$ | |
| 5.185 | $CH_3$ | $CH_3$ | H | m-$CH_3$-$C_6H_4$ | $CH_2$-CH=CHCl | |
| 5.186 | $CH_3$ | $CH_3$ | H | m-$CH_3$-$C_6H_4$ | $CH_2$-C≡CH | |
| 5.187 | $CH_3$ | $CH_3$ | H | m-$CH_3$-$C_6H_4$ | $CH_2$-$COOCH_3$ | |
| 5.188 | $CH_3$ | $CH_3$ | H | m-$CH_3$-$C_6H_4$ | CH($CH_3$)-$COOCH_3$ | |
| 5.189 | $CH_3$ | $CH_3$ | H | m-$CH_3$-$C_6H_4$ | H | |
| 5.190 | $CH_3$ | $CH_3$ | H | p-$CH_3$-$C_6H_4$ | $CH_3$ | |
| 5.191 | $CH_3$ | $CH_3$ | H | p-$CH_3$-$C_6H_4$ | $C_3H_7$(i) | |
| 5.192 | $CH_3$ | $CH_3$ | H | p-$CH_3$-$C_6H_4$ | $CH_2$-CH=$CH_2$ | |
| 5.193 | $CH_3$ | $CH_3$ | H | p-$CH_3$-$C_6H_4$ | $CH_2$-CCl=$CH_2$ | |
| 5.194 | $CH_3$ | $CH_3$ | H | p-$CH_3$-$C_6H_4$ | $CH_2$-CH=CHCl | |
| 5.195 | $CH_3$ | $CH_3$ | H | p-$CH_3$-$C_6H_4$ | $CH_2$-C≡CH | |
| 5.196 | $CH_3$ | $CH_3$ | H | p-$CH_3$-$C_6H_4$ | $CH_2$-$COOCH_3$ | |
| 5.197 | $CH_3$ | $CH_3$ | H | p-$CH_3$-$C_6H_4$ | CH($CH_3$)-$COOCH_3$ | |
| 5.198 | $CH_3$ | $CH_3$ | H | p-$CH_3$-$C_6H_4$ | H | |
| 5.199 | $CH_3$ | $CH_3$ | H | o-$CH_3O$-$C_6H_4$ | $CH_3$ | |
| 5.200 | $CH_3$ | $CH_3$ | H | o-$CH_3O$-$C_6H_4$ | $C_3H_7$(i) | |
| 5.201 | $CH_3$ | $CH_3$ | H | o-$CH_3O$-$C_6H_4$ | $CH_2$-CH=$CH_2$ | |
| 5.202 | $CH_3$ | $CH_3$ | H | o-$CH_3O$-$C_6H_4$ | $CH_2$-CCl=$CH_2$ | |
| 5.203 | $CH_3$ | $CH_3$ | H | o-$CH_3O$-$C_6H_4$ | $CH_2$-CH=CHCl | |
| 5.204 | $CH_3$ | $CH_3$ | H | o-$CH_3O$-$C_6H_4$ | $CH_2$-C≡CH | |
| 5.205 | $CH_3$ | $CH_3$ | H | o-$CH_3O$-$C_6H_4$ | $CH_2$-$COOCH_3$ | |
| 5.206 | $CH_3$ | $CH_3$ | H | o-$CH_3O$-$C_6H_4$ | CH($CH_3$)-$COOCH_3$ | |

| Nr. | $R^1$ | $R^2$ | $R^3$ | $R^4$ | $R^7$ | Phys. Daten |
|---|---|---|---|---|---|---|
| 5.207 | $CH_3$ | $CH_3$ | H | o-$CH_3O$-$C_6H_4$ | H | |
| 5.208 | $CH_3$ | $CH_3$ | H | m-$CH_3O$-$C_6H_4$ | $CH_3$ | |
| 5.209 | $CH_3$ | $CH_3$ | H | m-$CH_3O$-$C_6H_4$ | $C_3H_7$(i) | |
| 5.210 | $CH_3$ | $CH_3$ | H | m-$CH_3O$-$C_6H_4$ | $CH_2$-CH=$CH_2$ | |
| 5.211 | $CH_3$ | $CH_3$ | H | m-$CH_3O$-$C_6H_4$ | $CH_2$-CCl=$CH_2$ | |
| 5.212 | $CH_3$ | $CH_3$ | H | m-$CH_3O$-$C_6H_4$ | $CH_2$-CH=CHCl | |
| 5.213 | $CH_3$ | $CH_3$ | H | m-$CH_3O$-$C_6H_4$ | $CH_2$-C≡CH | |
| 5.214 | $CH_3$ | $CH_3$ | H | m-$CH_3O$-$C_6H_4$ | $CH_2$-$COOCH_3$ | |
| 5.215 | $CH_3$ | $CH_3$ | H | m-$CH_3O$-$C_6H_4$ | CH($CH_3$)-$COOCH_3$ | |
| 5.216 | $CH_3$ | $CH_3$ | H | m-$CH_3O$-$C_6H_4$ | H | |
| 5.217 | $CH_3$ | $CH_3$ | H | p-$CH_3O$-$C_6H_4$ | $CH_3$ | |
| 5.218 | $CH_3$ | $CH_3$ | H | p-$CH_3O$-$C_6H_4$ | $C_3H_7$(i) | |
| 5.219 | $CH_3$ | $CH_3$ | H | p-$CH_3O$-$C_6H_4$ | $CH_2$-CH=$CH_2$ | |
| 5.220 | $CH_3$ | $CH_3$ | H | p-$CH_3O$-$C_6H_4$ | $CH_2$-CCl=$CH_2$ | |
| 5.221 | $CH_3$ | $CH_3$ | H | p-$CH_3O$-$C_6H_4$ | $CH_2$-CH=CHCl | |
| 5.222 | $CH_3$ | $CH_3$ | H | p-$CH_3O$-$C_6H_4$ | $CH_2$-C≡CH | |
| 5.223 | $CH_3$ | $CH_3$ | H | p-$CH_3O$-$C_6H_4$ | $CH_2$-$COOCH_3$ | |
| 5.224 | $CH_3$ | $CH_3$ | H | p-$CH_3O$-$C_6H_4$ | CH($CH_3$)-$COOCH_3$ | |
| 5.225 | $CH_3$ | $CH_3$ | H | p-$CH_3O$-$C_6H_4$ | H | |
| 5.226 | $CH_3$ | $CH_3$ | H | o-F-$C_6H_4$ | $CH_3$ | |
| 5.227 | $CH_3$ | $CH_3$ | H | o-F-$C_6H_4$ | $C_3H_7$(i) | |
| 5.228 | $CH_3$ | $CH_3$ | H | o-F-$C_6H_4$ | $CH_2$-CH=$CH_2$ | |
| 5.229 | $CH_3$ | $CH_3$ | H | o-F-$C_6H_4$ | $CH_2$-CCl=$CH_2$ | |
| 5.230 | $CH_3$ | $CH_3$ | H | o-F-$C_6H_4$ | $CH_2$-CH=CHCl | |
| 5.231 | $CH_3$ | $CH_3$ | H | o-F-$C_6H_4$ | $CH_2$-C≡CH | |
| 5.232 | $CH_3$ | $CH_3$ | H | o-F-$C_6H_4$ | $CH_2$-$COOCH_3$ | |
| 5.233 | $CH_3$ | $CH_3$ | H | o-F-$C_6H_4$ | CH($CH_3$)-$COOCH_3$ | |
| 5.234 | $CH_3$ | $CH_3$ | H | o-F-$C_6H_4$ | H | |
| 5.235 | $CH_3$ | $CH_3$ | H | m-F-$C_6H_4$ | $CH_3$ | |
| 5.236 | $CH_3$ | $CH_3$ | H | m-F-$C_6H_4$ | $C_3H_7$(i) | |
| 5.237 | $CH_3$ | $CH_3$ | H | m-F-$C_6H_4$ | $CH_2$-CH=$CH_2$ | |

| Nr. | $R^1$ | $R^2$ | $R^3$ | $R^4$ | $R^7$ | Phys. Daten |
|---|---|---|---|---|---|---|
| 5.238 | $CH_3$ | $CH_3$ | H | m-F-$C_6H_4$ | $CH_2$-CCl=$CH_2$ | |
| 5.239 | $CH_3$ | $CH_3$ | H | m-F-$C_6H_4$ | $CH_2$-CH=CHCl | |
| 5.240 | $CH_3$ | $CH_3$ | H | m-F-$C_6H_4$ | $CH_2$-C≡CH | |
| 5.241 | $CH_3$ | $CH_3$ | H | m-F-$C_6H_4$ | $CH_2$-$COOCH_3$ | |
| 5.242 | $CH_3$ | $CH_3$ | H | m-F-$C_6H_4$ | CH($CH_3$)-$COOCH_3$ | |
| 5.243 | $CH_3$ | $CH_3$ | H | m-F-$C_6H_4$ | H | |
| 5.244 | $CH_3$ | $CH_3$ | H | p-F-$C_6H_4$ | $CH_3$ | |
| 5.245 | $CH_3$ | $CH_3$ | H | p-F-$C_6H_4$ | $C_3H_7$(i) | |
| 5.246 | $CH_3$ | $CH_3$ | H | p-F-$C_6H_4$ | $CH_2$-CH=$CH_2$ | |
| 5.247 | $CH_3$ | $CH_3$ | H | p-F-$C_6H_4$ | $CH_2$-CCl=$CH_2$ | |
| 5.248 | $CH_3$ | $CH_3$ | H | p-F-$C_6H_4$ | $CH_2$-CH=CHCl | |
| 5.249 | $CH_3$ | $CH_3$ | H | p-F-$C_6H_4$ | $CH_2$-C≡CH | |
| 5.250 | $CH_3$ | $CH_3$ | H | p-F-$C_6H_4$ | $CH_2$-$COOCH_3$ | |
| 5.251 | $CH_3$ | $CH_3$ | H | p-F-$C_6H_4$ | CH($CH_3$)-$COOCH_3$ | |
| 5.252 | $CH_3$ | $CH_3$ | H | p-F-$C_6H_4$ | H | |
| 5.253 | $CH_3$ | $CH_3$ | H | 2,4-($CH_3$)$_2$-$C_6H_3$ | $CH_3$ | |
| 5.254 | $CH_3$ | $CH_3$ | H | 2,4-($CH_3$)$_2$-$C_6H_3$ | $C_3H_7$(i) | |
| 5.255 | $CH_3$ | $CH_3$ | H | 2,4-($CH_3$)$_2$-$C_6H_3$ | $CH_2$-CH=$CH_2$ | |
| 5.256 | $CH_3$ | $CH_3$ | H | 2,4-($CH_3$)$_2$-$C_6H_3$ | $CH_2$-CCl=$CH_2$ | |
| 5.257 | $CH_3$ | $CH_3$ | H | 2,4-($CH_3$)$_2$-$C_6H_3$ | $CH_2$-CH=CHCl | |
| 5.258 | $CH_3$ | $CH_3$ | H | 2,4-($CH_3$)$_2$-$C_6H_3$ | $CH_2$-C≡CH | |
| 5.259 | $CH_3$ | $CH_3$ | H | 2,4-($CH_3$)$_2$-$C_6H_3$ | $CH_2$-$COOCH_3$ | |
| 5.260 | $CH_3$ | $CH_3$ | H | 2,4-($CH_3$)$_2$-$C_6H_3$ | CH($CH_3$)-$COOCH_3$ | |
| 5.261 | $CH_3$ | $CH_3$ | H | 2,4-($CH_3$)$_2$-$C_6H_3$ | H | |
| 5.262 | $CH_3$ | $CH_3$ | H | 2,4-($CH_3O$)$_2$-$C_6H_3$ | $CH_3$ | |
| 5.263 | $CH_3$ | $CH_3$ | H | 2,4-($CH_3O$)$_2$-$C_6H_3$ | $C_3H_7$(i) | |
| 5.264 | $CH_3$ | $CH_3$ | H | 2,4-($CH_3O$)$_2$-$C_6H_3$ | $CH_2$-CH=$CH_2$ | |
| 5.265 | $CH_3$ | $CH_3$ | H | 2,4-($CH_3O$)$_2$-$C_6H_3$ | $CH_2$-CCl=$CH_2$ | |
| 5.266 | $CH_3$ | $CH_3$ | H | 2,4-($CH_3O$)$_2$-$C_6H_3$ | $CH_2$-CH=CHCl | |
| 5.267 | $CH_3$ | $CH_3$ | H | 2,4-($CH_3O$)$_2$-$C_6H_3$ | $CH_2$-C≡CH | |
| 5.268 | $CH_3$ | $CH_3$ | H | 2,4-($CH_3O$)$_2$-$C_6H_3$ | $CH_2$-$COOCH_3$ | |

| Nr. | $R^1$ | $R^2$ | $R^3$ | $R^4$ | $R^7$ | Phys. Daten |
|---|---|---|---|---|---|---|
| 5.269 | $CH_3$ | $CH_3$ | H | $2,4-(CH_3O)_2-C_6H_3$ | $CH(CH_3)-COOCH_3$ | |
| 5.270 | $CH_3$ | $CH_3$ | H | $2,4-(CH_3O)_2-C_6H_3$ | H | |
| 5.271 | $CH_3$ | $CH_3$ | H | $3,4-(CH_3)_2-C_6H_3$ | $CH_3$ | |
| 5.272 | $CH_3$ | $CH_3$ | H | $3,4-(CH_3)_2-C_6H_3$ | $C_3H_7(i)$ | |
| 5.273 | $CH_3$ | $CH_3$ | H | $3,4-(CH_3)_2-C_6H_3$ | $CH_2-CH=CH_2$ | |
| 5.274 | $CH_3$ | $CH_3$ | H | $3,4-(CH_3)_2-C_6H_3$ | $CH_2-CCl=CH_2$ | |
| 5.275 | $CH_3$ | $CH_3$ | H | $3,4-(CH_3)_2-C_6H_3$ | $CH_2-CH=CHCl$ | |
| 5.276 | $CH_3$ | $CH_3$ | H | $3,4-(CH_3)_2-C_6H_3$ | $CH_2-C{\equiv}CH$ | |
| 5.277 | $CH_3$ | $CH_3$ | H | $3,4-(CH_3)_2-C_6H_3$ | $CH_2-COOCH_3$ | |
| 5.278 | $CH_3$ | $CH_3$ | H | $3,4-(CH_3)_2-C_6H_3$ | $CH(CH_3)-COOCH_3$ | |
| 5.279 | $CH_3$ | $CH_3$ | H | $3,4-(CH_3)_2-C_6H_3$ | H | |
| 5.280 | $CH_3$ | $CH_3$ | H | $3,4-(CH_3O)_2-C_6H_3$ | $CH_3$ | |
| 5.281 | $CH_3$ | $CH_3$ | H | $3,4-(CH_3O)_2-C_6H_3$ | $C_3H_7(i)$ | |
| 5.282 | $CH_3$ | $CH_3$ | H | $3,4-(CH_3O)_2-C_6H_3$ | $CH_2-CH=CH_2$ | |
| 5.283 | $CH_3$ | $CH_3$ | H | $3,4-(CH_3O)_2-C_6H_3$ | $CH_2-CCl=CH_2$ | |
| 5.284 | $CH_3$ | $CH_3$ | H | $3,4-(CH_3O)_2-C_6H_3$ | $CH_2-CH=CHCl$ | |
| 5.285 | $CH_3$ | $CH_3$ | H | $3,4-(CH_3O)_2-C_6H_3$ | $CH_2-C{\equiv}CH$ | |
| 5.286 | $CH_3$ | $CH_3$ | H | $3,4-(CH_3O)_2-C_6H_3$ | $CH_2-COOCH_3$ | |
| 5.287 | $CH_3$ | $CH_3$ | H | $3,4-(CH_3O)_2-C_6H_3$ | $CH(CH_3)-COOCH_3$ | |
| 5.288 | $CH_3$ | $CH_3$ | H | $3,4-(CH_3O)_2-C_6H_3$ | H | |
| 5.289 | $CH_3$ | $CH_3$ | H | $2,3,5-(CH_3)_3-C_6H_2$ | $CH_3$ | |
| 5.290 | $CH_3$ | $CH_3$ | H | $2,3,5-(CH_3)_3-C_6H_2$ | $C_3H_7(i)$ | |
| 5.291 | $CH_3$ | $CH_3$ | H | $2,3,5-(CH_3)_3-C_6H_2$ | $CH_2-CH=CH_2$ | |
| 5.292 | $CH_3$ | $CH_3$ | H | $2,3,5-(CH_3)_3-C_6H_2$ | $CH_2-CCl=CH_2$ | |
| 5.293 | $CH_3$ | $CH_3$ | H | $2,3,5-(CH_3)_3-C_6H_2$ | $CH_2-CH=CHCl$ | |
| 5.294 | $CH_3$ | $CH_3$ | H | $2,3,5-(CH_3)_3-C_6H_2$ | $CH_2-C{\equiv}CH$ | |
| 5.295 | $CH_3$ | $CH_3$ | H | $2,3,5-(CH_3)_3-C_6H_2$ | $CH_2-COOCH_3$ | |
| 5.296 | $CH_3$ | $CH_3$ | H | $2,3,5-(CH_3)_3-C_6H_2$ | $CH(CH_3)-COOCH_3$ | |
| 5.297 | $CH_3$ | $CH_3$ | H | $2,3,5-(CH_3)_3-C_6H_2$ | H | |
| 5.298 | $CH_3$ | $CH_3$ | H | $3,5-(CH_3O)_2-C_6H_3$ | $CH_3$ | |
| 5.299 | $CH_3$ | $CH_3$ | H | $3,5-(CH_3O)_2-C_6H_3$ | $C_3H_7(i)$ | |

| Nr. | $R^1$ | $R^2$ | $R^3$ | $R^4$ | $R^7$ | Phys. Daten |
|---|---|---|---|---|---|---|
| 5.300 | $CH_3$ | $CH_3$ | H | $3,5-(CH_3O)_2-C_6H_3$ | $CH_2-CH=CH_2$ | |
| 5.301 | $CH_3$ | $CH_3$ | H | $3,5-(CH_3O)_2-C_6H_3$ | $CH_2-CCl=CH_2$ | |
| 5.302 | $CH_3$ | $CH_3$ | H | $3,5-(CH_3O)_2-C_6H_3$ | $CH_2-CH=CHCl$ | |
| 5.303 | $CH_3$ | $CH_3$ | H | $3,5-(CH_3O)_2-C_6H_3$ | $CH_2-C\equiv CH$ | |
| 5.304 | $CH_3$ | $CH_3$ | H | $3,5-(CH_3O)_2-C_6H_3$ | $CH_2-COOCH_3$ | |
| 5.305 | $CH_3$ | $CH_3$ | H | $3,5-(CH_3O)_2-C_6H_3$ | $CH(CH_3)-COOCH_3$ | |
| 5.306 | $CH_3$ | $CH_3$ | H | $3,5-(CH_3O)_2-C_6H_3$ | H | |
| 5.307 | $C_2H_5$ | $C_2H_5$ | H | $C_6H_5$ | $CH_3$ | |
| 5.308 | $C_2H_5$ | $C_2H_5$ | H | $C_6H_5$ | $C_3H_7(i)$ | |
| 5.309 | $C_2H_5$ | $C_2H_5$ | H | $C_6H_5$ | $CH_2-CH=CH_2$ | |
| 5.310 | $C_2H_5$ | $C_2H_5$ | H | $C_6H_5$ | $CH_2-CCl=CH_2$ | |
| 5.311 | $C_2H_5$ | $C_2H_5$ | H | $C_6H_5$ | $CH_2-CH=CHCl$ | |
| 5.312 | $C_2H_5$ | $C_2H_5$ | H | $C_6H_5$ | $CH_2-C\equiv CH$ | |
| 5.313 | $C_2H_5$ | $C_2H_5$ | H | $C_6H_5$ | $CH_2-COOCH_3$ | |
| 5.314 | $C_2H_5$ | $C_2H_5$ | H | $C_6H_5$ | $CH(CH_3)-COOCH_3$ | |
| 5.315 | $C_2H_5$ | $C_2H_5$ | H | $C_6H_5$ | H | |
| 5.316 | $C_2H_5$ | $C_2H_5$ | H | $o-Cl-C_6H_4$ | $CH_3$ | |
| 5.317 | $C_2H_5$ | $C_2H_5$ | H | $o-Cl-C_6H_4$ | $C_3H_7(i)$ | |
| 5.318 | $C_2H_5$ | $C_2H_5$ | H | $o-Cl-C_6H_4$ | $CH_2-CH=CH_2$ | |
| 5.319 | $C_2H_5$ | $C_2H_5$ | H | $o-Cl-C_6H_4$ | $CH_2-CCl=CH_2$ | |
| 5.320 | $C_2H_5$ | $C_2H_5$ | H | $o-Cl-C_6H_4$ | $CH_2-CH=CHCl$ | |
| 5.321 | $C_2H_5$ | $C_2H_5$ | H | $o-Cl-C_6H_4$ | $CH_2-C\equiv CH$ | |
| 5.322 | $C_2H_5$ | $C_2H_5$ | H | $o-Cl-C_6H_4$ | $CH_2-COOCH_3$ | |
| 5.323 | $C_2H_5$ | $C_2H_5$ | H | $o-Cl-C_6H_4$ | $CH(CH_3)-COOCH_3$ | |
| 5.324 | $C_2H_5$ | $C_2H_5$ | H | $o-Cl-C_6H_4$ | H | |
| 5.325 | $C_2H_5$ | $C_2H_5$ | H | $m-Cl-C_6H_4$ | $CH_3$ | |
| 5.326 | $C_2H_5$ | $C_2H_5$ | H | $m-Cl-C_6H_4$ | $C_3H_7(i)$ | |
| 5.327 | $C_2H_5$ | $C_2H_5$ | H | $m-Cl-C_6H_4$ | $CH_2-CH=CH_2$ | |
| 5.328 | $C_2H_5$ | $C_2H_5$ | H | $m-Cl-C_6H_4$ | $CH_2-CCl=CH_2$ | |
| 5.329 | $C_2H_5$ | $C_2H_5$ | H | $m-Cl-C_6H_4$ | $CH_2-CH=CHCl$ | |
| 5.330 | $C_2H_5$ | $C_2H_5$ | H | $m-Cl-C_6H_4$ | $CH_2-C\equiv CH$ | |

| Nr. | $R^1$ | $R^2$ | $R^3$ | $R^4$ | $R^7$ | Phys. Daten |
|---|---|---|---|---|---|---|
| 5.331 | $C_2H_5$ | $C_2H_5$ | H | m-Cl-$C_6H_4$ | $CH_2$-$COOCH_3$ | |
| 5.332 | $C_2H_5$ | $C_2H_5$ | H | m-Cl-$C_6H_4$ | $CH(CH_3)$-$COOCH_3$ | |
| 5.333 | $C_2H_5$ | $C_2H_5$ | H | m-Cl-$C_6H_4$ | H | |
| 5.334 | $C_2H_5$ | $C_2H_5$ | H | p-Cl-$C_6H_4$ | $CH_3$ | |
| 5.335 | $C_2H_5$ | $C_2H_5$ | H | p-Cl-$C_6H_4$ | $C_3H_7$(i) | |
| 5.336 | $C_2H_5$ | $C_2H_5$ | H | p-Cl-$C_6H_4$ | $CH_2$-CH=$CH_2$ | |
| 5.337 | $C_2H_5$ | $C_2H_5$ | H | p-Cl-$C_6H_4$ | $CH_2$-CCl=$CH_2$ | |
| 5.338 | $C_2H_5$ | $C_2H_5$ | H | p-Cl-$C_6H_4$ | $CH_2$-CH=CHCl | |
| 5.339 | $C_2H_5$ | $C_2H_5$ | H | p-Cl-$C_6H_4$ | $CH_2$-C≡CH | |
| 5.340 | $C_2H_5$ | $C_2H_5$ | H | p-Cl-$C_6H_4$ | $CH_2$-$COOCH_3$ | |
| 5.341 | $C_2H_5$ | $C_2H_5$ | H | p-Cl-$C_6H_4$ | $CH(CH_3)$-$COOCH_3$ | |
| 5.342 | $C_2H_5$ | $C_2H_5$ | H | p-Cl-$C_6H_4$ | H | |
| 5.343 | $C_2H_5$ | $C_2H_5$ | H | o-$CH_3$-$C_6H_4$ | $CH_3$ | |
| 5.344 | $C_2H_5$ | $C_2H_5$ | H | o-$CH_3$-$C_6H_4$ | $C_3H_7$(i) | |
| 5.345 | $C_2H_5$ | $C_2H_5$ | H | o-$CH_3$-$C_6H_4$ | $CH_2$-CH=$CH_2$ | |
| 5.346 | $C_2H_5$ | $C_2H_5$ | H | o-$CH_3$-$C_6H_4$ | $CH_2$-CCl=$CH_2$ | |
| 5.347 | $C_2H_5$ | $C_2H_5$ | H | o-$CH_3$-$C_6H_4$ | $CH_2$-CH=CHCl | |
| 5.348 | $C_2H_5$ | $C_2H_5$ | H | o-$CH_3$-$C_6H_4$ | $CH_2$-C≡CH | |
| 5.349 | $C_2H_5$ | $C_2H_5$ | H | o-$CH_3$-$C_6H_4$ | $CH_2$-$COOCH_3$ | |
| 5.350 | $C_2H_5$ | $C_2H_5$ | H | o-$CH_3$-$C_6H_4$ | $CH(CH_3)$-$COOCH_3$ | |
| 5.351 | $C_2H_5$ | $C_2H_5$ | H | o-$CH_3$-$C_6H_4$ | H | |
| 5.352 | $C_2H_5$ | $C_2H_5$ | H | m-$CH_3$-$C_6H_4$ | $CH_3$ | |
| 5.353 | $C_2H_5$ | $C_2H_5$ | H | m-$CH_3$-$C_6H_4$ | $C_3H_7$(i) | |
| 5.354 | $C_2H_5$ | $C_2H_5$ | H | m-$CH_3$-$C_6H_4$ | $CH_2$-CH=$CH_2$ | |
| 5.355 | $C_2H_5$ | $C_2H_5$ | H | m-$CH_3$-$C_6H_4$ | $CH_2$-CCl=$CH_2$ | |
| 5.356 | $C_2H_5$ | $C_2H_5$ | H | m-$CH_3$-$C_6H_4$ | $CH_2$-CH=CHCl | |
| 5.357 | $C_2H_5$ | $C_2H_5$ | H | m-$CH_3$-$C_6H_4$ | $CH_2$-C≡CH | |
| 5.358 | $C_2H_5$ | $C_2H_5$ | H | m-$CH_3$-$C_6H_4$ | $CH_2$-$COOCH_3$ | |
| 5.359 | $C_2H_5$ | $C_2H_5$ | H | m-$CH_3$-$C_6H_4$ | $CH(CH_3)$-$COOCH_3$ | |
| 5.360 | $C_2H_5$ | $C_2H_5$ | H | m-$CH_3$-$C_6H_4$ | H | |
| 5.361 | $C_2H_5$ | $C_2H_5$ | H | p-$CH_3$-$C_6H_4$ | $CH_3$ | |

| Nr. | $R^1$ | $R^2$ | $R^3$ | $R^4$ | $R^7$ | Phys. Daten |
|---|---|---|---|---|---|---|
| 5.362 | $C_2H_5$ | $C_2H_5$ | H | $p-CH_3-C_6H_4$ | $C_3H_7(i)$ | |
| 5.363 | $C_2H_5$ | $C_2H_5$ | H | $p-CH_3-C_6H_4$ | $CH_2-CH=CH_2$ | |
| 5.364 | $C_2H_5$ | $C_2H_5$ | H | $p-CH_3-C_6H_4$ | $CH_2-CCl=CH_2$ | |
| 5.365 | $C_2H_5$ | $C_2H_5$ | H | $p-CH_3-C_6H_4$ | $CH_2-CH=CHCl$ | |
| 5.366 | $C_2H_5$ | $C_2H_5$ | H | $p-CH_3-C_6H_4$ | $CH_2-C{\equiv}CH$ | |
| 5.367 | $C_2H_5$ | $C_2H_5$ | H | $p-CH_3-C_6H_4$ | $CH_2-COOCH_3$ | |
| 5.368 | $C_2H_5$ | $C_2H_5$ | H | $p-CH_3-C_6H_4$ | $CH(CH_3)-COOCH_3$ | |
| 5.369 | $C_2H_5$ | $C_2H_5$ | H | $p-CH_3-C_6H_4$ | H | |
| 5.370 | $C_2H_5$ | $C_2H_5$ | H | $o-CH_3O-C_6H_4$ | $CH_3$ | |
| 5.371 | $C_2H_5$ | $C_2H_5$ | H | $o-CH_3O-C_6H_4$ | $C_3H_7(i)$ | |
| 5.372 | $C_2H_5$ | $C_2H_5$ | H | $o-CH_3O-C_6H_4$ | $CH_2-CH=CH_2$ | |
| 5.373 | $C_2H_5$ | $C_2H_5$ | H | $o-CH_3O-C_6H_4$ | $CH_2-CCl=CH_2$ | |
| 5.374 | $C_2H_5$ | $C_2H_5$ | H | $o-CH_3O-C_6H_4$ | $CH_2-CH=CHCl$ | |
| 5.375 | $C_2H_5$ | $C_2H_5$ | H | $o-CH_3O-C_6H_4$ | $CH_2-C{\equiv}CH$ | |
| 5.376 | $C_2H_5$ | $C_2H_5$ | H | $o-CH_3O-C_6H_4$ | $CH_2-COOCH_3$ | |
| 5.377 | $C_2H_5$ | $C_2H_5$ | H | $o-CH_3O-C_6H_4$ | $CH(CH_3)-COOCH_3$ | |
| 5.378 | $C_2H_5$ | $C_2H_5$ | H | $o-CH_3O-C_6H_4$ | H | |
| 5.379 | $C_2H_5$ | $C_2H_5$ | H | $m-CH_3O-C_6H_4$ | $CH_3$ | |
| 5.380 | $C_2H_5$ | $C_2H_5$ | H | $m-CH_3O-C_6H_4$ | $C_3H_7(i)$ | |
| 5.381 | $C_2H_5$ | $C_2H_5$ | H | $m-CH_3O-C_6H_4$ | $CH_2-CH=CH_2$ | |
| 5.382 | $C_2H_5$ | $C_2H_5$ | H | $m-CH_3O-C_6H_4$ | $CH_2-CCl=CH_2$ | |
| 5.383 | $C_2H_5$ | $C_2H_5$ | H | $m-CH_3O-C_6H_4$ | $CH_2-CH=CHCl$ | |
| 5.384 | $C_2H_5$ | $C_2H_5$ | H | $m-CH_3O-C_6H_4$ | $CH_2-C{\equiv}CH$ | |
| 5.385 | $C_2H_5$ | $C_2H_5$ | H | $m-CH_3O-C_6H_4$ | $CH_2-COOCH_3$ | |
| 5.386 | $C_2H_5$ | $C_2H_5$ | H | $m-CH_3O-C_6H_4$ | $CH(CH_3)-COOCH_3$ | |
| 5.387 | $C_2H_5$ | $C_2H_5$ | H | $m-CH_3O-C_6H_4$ | H | |
| 5.388 | $C_2H_5$ | $C_2H_5$ | H | $p-CH_3O-C_6H_4$ | $CH_3$ | |
| 5.389 | $C_2H_5$ | $C_2H_5$ | H | $p-CH_3O-C_6H_4$ | $C_3H_7(i)$ | |
| 5.390 | $C_2H_5$ | $C_2H_5$ | H | $p-CH_3O-C_6H_4$ | $CH_2-CH=CH_2$ | |
| 5.391 | $C_2H_5$ | $C_2H_5$ | H | $p-CH_3O-C_6H_4$ | $CH_2-CCl=CH_2$ | |
| 5.392 | $C_2H_5$ | $C_2H_5$ | H | $p-CH_3O-C_6H_4$ | $CH_2-CH=CHCl$ | |

| Nr. | $R^1$ | $R^2$ | $R^3$ | $R^4$ | $R^7$ | Phys. Daten |
|---|---|---|---|---|---|---|
| 5.393 | $C_2H_5$ | $C_2H_5$ | H | p-$CH_3O$-$C_6H_4$ | $CH_2$-C≡CH | |
| 5.394 | $C_2H_5$ | $C_2H_5$ | H | p-$CH_3O$-$C_6H_4$ | $CH_2$-$COOCH_3$ | |
| 5.395 | $C_2H_5$ | $C_2H_5$ | H | p-$CH_3O$-$C_6H_4$ | $CH(CH_3)$-$COOCH_3$ | |
| 5.396 | $C_2H_5$ | $C_2H_5$ | H | p-$CH_3O$-$C_6H_4$ | H | |
| 5.397 | $C_2H_5$ | $C_2H_5$ | H | o-F-$C_6H_4$ | $CH_3$ | |
| 5.398 | $C_2H_5$ | $C_2H_5$ | H | o-F-$C_6H_4$ | $C_3H_7$(i) | |
| 5.399 | $C_2H_5$ | $C_2H_5$ | H | o-F-$C_6H_4$ | $CH_2$-CH=$CH_2$ | |
| 5.400 | $C_2H_5$ | $C_2H_5$ | H | o-F-$C_6H_4$ | $CH_2$-CCl=$CH_2$ | |
| 5.401 | $C_2H_5$ | $C_2H_5$ | H | o-F-$C_6H_4$ | $CH_2$-CH=CHCl | |
| 5.402 | $C_2H_5$ | $C_2H_5$ | H | o-F-$C_6H_4$ | $CH_2$-C≡CH | |
| 5.403 | $C_2H_5$ | $C_2H_5$ | H | o-F-$C_6H_4$ | $CH_2$-$COOCH_3$ | |
| 5.404 | $C_2H_5$ | $C_2H_5$ | H | o-F-$C_6H_4$ | $CH(CH_3)$-$COOCH_3$ | |
| 5.405 | $C_2H_5$ | $C_2H_5$ | H | o-F-$C_6H_4$ | H | |
| 5.406 | $C_2H_5$ | $C_2H_5$ | H | m-F-$C_6H_4$ | $CH_3$ | |
| 5.407 | $C_2H_5$ | $C_2H_5$ | H | m-F-$C_6H_4$ | $C_3H_7$(i) | |
| 5.408 | $C_2H_5$ | $C_2H_5$ | H | m-F-$C_6H_4$ | $CH_2$-CH=$CH_2$ | |
| 5.409 | $C_2H_5$ | $C_2H_5$ | H | m-F-$C_6H_4$ | $CH_2$-CCl=$CH_2$ | |
| 5.410 | $C_2H_5$ | $C_2H_5$ | H | m-F-$C_6H_4$ | $CH_2$-CH=CHCl | |
| 5.411 | $C_2H_5$ | $C_2H_5$ | H | m-F-$C_6H_4$ | $CH_2$-C≡CH | |
| 5.412 | $C_2H_5$ | $C_2H_5$ | H | m-F-$C_6H_4$ | $CH_2$-$COOCH_3$ | |
| 5.413 | $C_2H_5$ | $C_2H_5$ | H | m-F-$C_6H_4$ | $CH(CH_3)$-$COOCH_3$ | |
| 5.414 | $C_2H_5$ | $C_2H_5$ | H | m-F-$C_6H_4$ | H | |
| 5.415 | $C_2H_5$ | $C_2H_5$ | H | p-F-$C_6H_4$ | $CH_3$ | |
| 5.416 | $C_2H_5$ | $C_2H_5$ | H | p-F-$C_6H_4$ | $C_3H_7$(i) | |
| 5.417 | $C_2H_5$ | $C_2H_5$ | H | p-F-$C_6H_4$ | $CH_2$-CH=$CH_2$ | |
| 5.418 | $C_2H_5$ | $C_2H_5$ | H | p-F-$C_6H_4$ | $CH_2$-CCl=$CH_2$ | |
| 5.419 | $C_2H_5$ | $C_2H_5$ | H | p-F-$C_6H_4$ | $CH_2$-CH=CHCl | |
| 5.420 | $C_2H_5$ | $C_2H_5$ | H | p-F-$C_6H_4$ | $CH_2$-C≡CH | |
| 5.421 | $C_2H_5$ | $C_2H_5$ | H | p-F-$C_6H_4$ | $CH_2$-$COOCH_3$ | |
| 5.422 | $C_2H_5$ | $C_2H_5$ | H | p-F-$C_6H_4$ | $CH(CH_3)$-$COOCH_3$ | |
| 5.423 | $C_2H_5$ | $C_2H_5$ | H | p-F-$C_6H_4$ | H | |

| Nr. | $R^1$ | $R^2$ | $R^3$ | $R^4$ | $R^7$ | Phys. Daten |
|---|---|---|---|---|---|---|
| 5.424 | $C_2H_5$ | $C_2H_5$ | H | $2,4-(CH_3)_2-C_6H_3$ | $CH_3$ | |
| 5.425 | $C_2H_5$ | $C_2H_5$ | H | $2,4-(CH_3)_2-C_6H_3$ | $C_3H_7(i)$ | |
| 5.426 | $C_2H_5$ | $C_2H_5$ | H | $2,4-(CH_3)_2-C_6H_3$ | $CH_2-CH=CH_2$ | |
| 5.427 | $C_2H_5$ | $C_2H_5$ | H | $2,4-(CH_3)_2-C_6H_3$ | $CH_2-CCl=CH_2$ | |
| 5.428 | $C_2H_5$ | $C_2H_5$ | H | $2,4-(CH_3)_2-C_6H_3$ | $CH_2-CH=CHCl$ | |
| 5.429 | $C_2H_5$ | $C_2H_5$ | H | $2,4-(CH_3)_2-C_6H_3$ | $CH_2-C\equiv CH$ | |
| 5.430 | $C_2H_5$ | $C_2H_5$ | H | $2,4-(CH_3)_2-C_6H_3$ | $CH_2-COOCH_3$ | |
| 5.431 | $C_2H_5$ | $C_2H_5$ | H | $2,4-(CH_3)_2-C_6H_3$ | $CH(CH_3)-COOCH_3$ | |
| 5.432 | $C_2H_5$ | $C_2H_5$ | H | $2,4-(CH_3)_2-C_6H_3$ | H | |
| 5.433 | $C_2H_5$ | $C_2H_5$ | H | $2,4-(CH_3O)_2-C_6H_3$ | $CH_3$ | |
| 5.434 | $C_2H_5$ | $C_2H_5$ | H | $2,4-(CH_3O)_2-C_6H_3$ | $C_3H_7(i)$ | |
| 5.435 | $C_2H_5$ | $C_2H_5$ | H | $2,4-(CH_3O)_2-C_6H_3$ | $CH_2-CH=CH_2$ | |
| 5.436 | $C_2H_5$ | $C_2H_5$ | H | $2,4-(CH_3O)_2-C_6H_3$ | $CH_2-CCl=CH_2$ | |
| 5.437 | $C_2H_5$ | $C_2H_5$ | H | $2,4-(CH_3O)_2-C_6H_3$ | $CH_2-CH=CHCl$ | |
| 5.438 | $C_2H_5$ | $C_2H_5$ | H | $2,4-(CH_3O)_2-C_6H_3$ | $CH_2-C\equiv CH$ | |
| 5.439 | $C_2H_5$ | $C_2H_5$ | H | $2,4-(CH_3O)_2-C_6H_3$ | $CH_2-COOCH_3$ | |
| 5.440 | $C_2H_5$ | $C_2H_5$ | H | $2,4-(CH_3O)_2-C_6H_3$ | $CH(CH_3)-COOCH_3$ | |
| 5.441 | $C_2H_5$ | $C_2H_5$ | H | $2,4-(CH_3O)_2-C_6H_3$ | H | |
| 5.442 | $C_2H_5$ | $C_2H_5$ | H | $3,4-(CH_3)_2-C_6H_3$ | $CH_3$ | |
| 5.443 | $C_2H_5$ | $C_2H_5$ | H | $3,4-(CH_3)_2-C_6H_3$ | $C_3H_7(i)$ | |
| 5.444 | $C_2H_5$ | $C_2H_5$ | H | $3,4-(CH_3)_2-C_6H_3$ | $CH_2-CH=CH_2$ | |
| 5.445 | $C_2H_5$ | $C_2H_5$ | H | $3,4-(CH_3)_2-C_6H_3$ | $CH_2-CCl=CH_2$ | |
| 5.446 | $C_2H_5$ | $C_2H_5$ | H | $3,4-(CH_3)_2-C_6H_3$ | $CH_2-CH=CHCl$ | |
| 5.447 | $C_2H_5$ | $C_2H_5$ | H | $3,4-(CH_3)_2-C_6H_3$ | $CH_2-C\equiv CH$ | |
| 5.448 | $C_2H_5$ | $C_2H_5$ | H | $3,4-(CH_3)_2-C_6H_3$ | $CH_2-COOCH_3$ | |
| 5.449 | $C_2H_5$ | $C_2H_5$ | H | $3,4-(CH_3)_2-C_6H_3$ | $CH(CH_3)-COOCH_3$ | |
| 5.450 | $C_2H_5$ | $C_2H_5$ | H | $3,4-(CH_3)_2-C_6H_3$ | H | |
| 5.451 | $C_2H_5$ | $C_2H_5$ | H | $3,4-(CH_3O)_2-C_6H_3$ | $CH_3$ | |
| 5.452 | $C_2H_5$ | $C_2H_5$ | H | $3,4-(CH_3O)_2-C_6H_3$ | $C_3H_7(i)$ | |
| 5.453 | $C_2H_5$ | $C_2H_5$ | H | $3,4-(CH_3O)_2-C_6H_3$ | $CH_2-CH=CH_2$ | |
| 5.454 | $C_2H_5$ | $C_2H_5$ | H | $3,4-(CH_3O)_2-C_6H_3$ | $CH_2-CCl=CH_2$ | |

| Nr. | $R^1$ | $R^2$ | $R^3$ | $R^4$ | $R^7$ | Phys. Daten |
|---|---|---|---|---|---|---|
| 5.455 | $C_2H_5$ | $C_2H_5$ | H | $3,4-(CH_3O)_2-C_6H_3$ | $CH_2-CH=CHCl$ | |
| 5.456 | $C_2H_5$ | $C_2H_5$ | H | $3,4-(CH_3O)_2-C_6H_3$ | $CH_2-C\equiv CH$ | |
| 5.457 | $C_2H_5$ | $C_2H_5$ | H | $3,4-(CH_3O)_2-C_6H_3$ | $CH_2-COOCH_3$ | |
| 5.458 | $C_2H_5$ | $C_2H_5$ | H | $3,4-(CH_3O)_2-C_6H_3$ | $CH(CH_3)-COOCH_3$ | |
| 5.459 | $C_2H_5$ | $C_2H_5$ | H | $3,4-(CH_3O)_2-C_6H_3$ | H | |
| 5.460 | $C_2H_5$ | $C_2H_5$ | H | $2,3,5-(CH_3)_3-C_6H_2$ | $CH_3$ | |
| 5.461 | $C_2H_5$ | $C_2H_5$ | H | $2,3,5-(CH_3)_3-C_6H_2$ | $C_3H_7(i)$ | |
| 5.462 | $C_2H_5$ | $C_2H_5$ | H | $2,3,5-(CH_3)_3-C_6H_2$ | $CH_2-CH=CH_2$ | |
| 5.463 | $C_2H_5$ | $C_2H_5$ | H | $2,3,5-(CH_3)_3-C_6H_2$ | $CH_2-CCl=CH_2$ | |
| 5.464 | $C_2H_5$ | $C_2H_5$ | H | $2,3,5-(CH_3)_3-C_6H_2$ | $CH_2-CH=CHCl$ | |
| 5.465 | $C_2H_5$ | $C_2H_5$ | H | $2,3,5-(CH_3)_3-C_6H_2$ | $CH_2-C\equiv CH$ | |
| 5.466 | $C_2H_5$ | $C_2H_5$ | H | $2,3,5-(CH_3)_3-C_6H_2$ | $CH_2-COOCH_3$ | |
| 5.467 | $C_2H_5$ | $C_2H_5$ | H | $2,3,5-(CH_3)_3-C_6H_2$ | $CH(CH_3)-COOCH_3$ | |
| 5.468 | $C_2H_5$ | $C_2H_5$ | H | $2,3,5-(CH_3)_3-C_6H_2$ | H | |
| 5.469 | $C_2H_5$ | $C_2H_5$ | H | $3,5-(CH_3O)_2-C_6H_3$ | $CH_3$ | |
| 5.470 | $C_2H_5$ | $C_2H_5$ | H | $3,5-(CH_3O)_2-C_6H_3$ | $C_3H_7(i)$ | |
| 5.471 | $C_2H_5$ | $C_2H_5$ | H | $3,5-(CH_3O)_2-C_6H_3$ | $CH_2-CH=CH_2$ | |
| 5.472 | $C_2H_5$ | $C_2H_5$ | H | $3,5-(CH_3O)_2-C_6H_3$ | $CH_2-CCl=CH_2$ | |
| 5.473 | $C_2H_5$ | $C_2H_5$ | H | $3,5-(CH_3O)_2-C_6H_3$ | $CH_2-CH=CHCl$ | |
| 5.474 | $C_2H_5$ | $C_2H_5$ | H | $3,5-(CH_3O)_2-C_6H_3$ | $CH_2-C\equiv CH$ | |
| 5.475 | $C_2H_5$ | $C_2H_5$ | H | $3,5-(CH_3O)_2-C_6H_3$ | $CH_2-COOCH_3$ | |
| 5.476 | $C_2H_5$ | $C_2H_5$ | H | $3,5-(CH_3O)_2-C_6H_3$ | $CH(CH_3)-COOCH_3$ | |
| 5.477 | $C_2H_5$ | $C_2H_5$ | H | $3,5-(CH_3O)_2-C_6H_3$ | H | |
| 5.478 | $-(CH_2)_4-$ | | H | $C_6H_5$ | $CH_3$ | |
| 5.479 | $-(CH_2)_4-$ | | H | $C_6H_5$ | $C_3H_7(i)$ | |
| 5.480 | $-(CH_2)_4-$ | | H | $C_6H_5$ | $CH_2-CH=CH_2$ | |
| 5.481 | $-(CH_2)_4-$ | | H | $C_6H_5$ | $CH_2-CCl=CH_2$ | |
| 5.482 | $-(CH_2)_4-$ | | H | $C_6H_5$ | $CH_2-CH=CHCl$ | |
| 5.483 | $-(CH_2)_4-$ | | H | $C_6H_5$ | $CH_2-C\equiv CH$ | |
| 5.484 | $-(CH_2)_4-$ | | H | $C_6H_5$ | $CH_2-COOCH_3$ | |
| 5.485 | $-(CH_2)_4-$ | | H | $C_6H_5$ | $CH(CH_3)-COOCH_3$ | |

| Nr. | $R^1$ $R^2$ | $R^3$ | $R^4$ | $R^7$ | Phys. Daten |
|---|---|---|---|---|---|
| 5.486 | $-(CH_2)_4-$ | H | $C_6H_5$ | H | |
| 5.487 | $-(CH_2)_4-$ | H | $o-Cl-C_6H_4$ | $CH_3$ | |
| 5.488 | $-(CH_2)_4-$ | H | $o-Cl-C_6H_4$ | $C_3H_7(i)$ | |
| 5.489 | $-(CH_2)_4-$ | H | $o-Cl-C_6H_4$ | $CH_2-CH=CH_2$ | |
| 5.490 | $-(CH_2)_4-$ | H | $o-Cl-C_6H_4$ | $CH_2-CCl=CH_2$ | |
| 5.491 | $-(CH_2)_4-$ | H | $o-Cl-C_6H_4$ | $CH_2-CH=CHCl$ | |
| 5.492 | $-(CH_2)_4-$ | H | $o-Cl-C_6H_4$ | $CH_2-C\equiv CH$ | |
| 5.493 | $-(CH_2)_4-$ | H | $o-Cl-C_6H_4$ | $CH_2-COOCH_3$ | |
| 5.494 | $-(CH_2)_4-$ | H | $o-Cl-C_6H_4$ | $CH(CH_3)-COOCH_3$ | |
| 5.495 | $-(CH_2)_4-$ | H | $o-Cl-C_6H_4$ | H | |
| 5.496 | $-(CH_2)_4-$ | H | $m-Cl-C_6H_4$ | $CH_3$ | |
| 5.497 | $-(CH_2)_4-$ | H | $m-Cl-C_6H_4$ | $C_3H_7(i)$ | |
| 5.498 | $-(CH_2)_4-$ | H | $m-Cl-C_6H_4$ | $CH_2-CH=CH_2$ | |
| 5.499 | $-(CH_2)_4-$ | H | $m-Cl-C_6H_4$ | $CH_2-CCl=CH_2$ | |
| 5.500 | $-(CH_2)_4-$ | H | $m-Cl-C_6H_4$ | $CH_2-CH=CHCl$ | |
| 5.501 | $-(CH_2)_4-$ | H | $m-Cl-C_6H_4$ | $CH_2-C\equiv CH$ | |
| 5.502 | $-(CH_2)_4-$ | H | $m-Cl-C_6H_4$ | $CH_2-COOCH_3$ | |
| 5.503 | $-(CH_2)_4-$ | H | $m-Cl-C_6H_4$ | $CH(CH_3)-COOCH_3$ | |
| 5.504 | $-(CH_2)_4-$ | H | $m-Cl-C_6H_4$ | H | |
| 5.505 | $-(CH_2)_4-$ | H | $p-Cl-C_6H_4$ | $CH_3$ | |
| 5.506 | $-(CH_2)_4-$ | H | $p-Cl-C_6H_4$ | $C_3H_7(i)$ | |
| 5.507 | $-(CH_2)_4-$ | H | $p-Cl-C_6H_4$ | $CH_2-CH=CH_2$ | |
| 5.508 | $-(CH_2)_4-$ | H | $p-Cl-C_6H_4$ | $CH_2-CCl=CH_2$ | |
| 5.509 | $-(CH_2)_4-$ | H | $p-Cl-C_6H_4$ | $CH_2-CH=CHCl$ | |
| 5.510 | $-(CH_2)_4-$ | H | $p-Cl-C_6H_4$ | $CH_2-C\equiv CH$ | |
| 5.511 | $-(CH_2)_4-$ | H | $p-Cl-C_6H_4$ | $CH_2-COOCH_3$ | |
| 5.512 | $-(CH_2)_4-$ | H | $p-Cl-C_6H_4$ | $CH(CH_3)-COOCH_3$ | |
| 5.513 | $-(CH_2)_4-$ | H | $p-Cl-C_6H_4$ | H | |
| 5.514 | $-(CH_2)_4-$ | H | $o-CH_3-C_6H_4$ | $CH_3$ | |
| 5.515 | $-(CH_2)_4-$ | H | $o-CH_3-C_6H_4$ | $C_3H_7(i)$ | |
| 5.516 | $-(CH_2)_4-$ | H | $o-CH_3-C_6H_4$ | $CH_2-CH=CH_2$ | |

| Nr. | $R^1$ | $R^2$ | $R^3$ | $R^4$ | $R^7$ | Phys. Daten |
|---|---|---|---|---|---|---|
| 5.517 | $-(CH_2)_4-$ | | H | $o-CH_3-C_6H_4$ | $CH_2-CCl=CH_2$ | |
| 5.518 | $-(CH_2)_4-$ | | H | $o-CH_3-C_6H_4$ | $CH_2-CH=CHCl$ | |
| 5.519 | $-(CH_2)_4-$ | | H | $o-CH_3-C_6H_4$ | $CH_2-C{\equiv}CH$ | |
| 5.520 | $-(CH_2)_4-$ | | H | $o-CH_3-C_6H_4$ | $CH_2-COOCH_3$ | |
| 5.521 | $-(CH_2)_4-$ | | H | $o-CH_3-C_6H_4$ | $CH(CH_3)-COOCH_3$ | |
| 5.522 | $-(CH_2)_4-$ | | H | $o-CH_3-C_6H_4$ | H | |
| 5.523 | $-(CH_2)_4-$ | | H | $m-CH_3-C_6H_4$ | $CH_3$ | |
| 5.524 | $-(CH_2)_4-$ | | H | $m-CH_3-C_6H_4$ | $C_3H_7(i)$ | |
| 5.525 | $-(CH_2)_4-$ | | H | $m-CH_3-C_6H_4$ | $CH_2-CH=CH_2$ | |
| 5.526 | $-(CH_2)_4-$ | | H | $m-CH_3-C_6H_4$ | $CH_2-CCl=CH_2$ | |
| 5.527 | $-(CH_2)_4-$ | | H | $m-CH_3-C_6H_4$ | $CH_2-CH=CHCl$ | |
| 5.528 | $-(CH_2)_4-$ | | H | $m-CH_3-C_6H_4$ | $CH_2-C{\equiv}CH$ | |
| 5.529 | $-(CH_2)_4-$ | | H | $m-CH_3-C_6H_4$ | $CH_2-COOCH_3$ | |
| 5.530 | $-(CH_2)_4-$ | | H | $m-CH_3-C_6H_4$ | $CH(CH_3)-COOCH_3$ | |
| 5.531 | $-(CH_2)_4-$ | | H | $m-CH_3-C_6H_4$ | H | |
| 5.532 | $-(CH_2)_4-$ | | H | $p-CH_3-C_6H_4$ | $CH_3$ | |
| 5.533 | $-(CH_2)_4-$ | | H | $p-CH_3-C_6H_4$ | $C_3H_7(i)$ | |
| 5.534 | $-(CH_2)_4-$ | | H | $p-CH_3-C_6H_4$ | $CH_2-CH=CH_2$ | |
| 5.535 | $-(CH_2)_4-$ | | H | $p-CH_3-C_6H_4$ | $CH_2-CCl=CH_2$ | |
| 5.536 | $-(CH_2)_4-$ | | H | $p-CH_3-C_6H_4$ | $CH_2-CH=CHCl$ | |
| 5.537 | $-(CH_2)_4-$ | | H | $p-CH_3-C_6H_4$ | $CH_2-C{\equiv}CH$ | |
| 5.538 | $-(CH_2)_4-$ | | H | $p-CH_3-C_6H_4$ | $CH_2-COOCH_3$ | |
| 5.539 | $-(CH_2)_4-$ | | H | $p-CH_3-C_6H_4$ | $CH(CH_3)-COOCH_3$ | |
| 5.540 | $-(CH_2)_4-$ | | H | $p-CH_3-C_6H_4$ | H | |
| 5.541 | $-(CH_2)_4-$ | | H | $o-CH_3O-C_6H_4$ | $CH_3$ | |
| 5.542 | $-(CH_2)_4-$ | | H | $o-CH_3O-C_6H_4$ | $C_3H_7(i)$ | |
| 5.543 | $-(CH_2)_4-$ | | H | $o-CH_3O-C_6H_4$ | $CH_2-CH=CH_2$ | |
| 5.544 | $-(CH_2)_4-$ | | H | $o-CH_3O-C_6H_4$ | $CH_2-CCl=CH_2$ | |
| 5.545 | $-(CH_2)_4-$ | | H | $o-CH_3O-C_6H_4$ | $CH_2-CH=CHCl$ | |
| 5.546 | $-(CH_2)_4-$ | | H | $o-CH_3O-C_6H_4$ | $CH_2-C{\equiv}CH$ | |
| 5.547 | $-(CH_2)_4-$ | | H | $o-CH_3O-C_6H_4$ | $CH_2-COOCH_3$ | |

| Nr. | $R^1$ | $R^2$ | $R^3$ | $R^4$ | $R^7$ | Phys. Daten |
|---|---|---|---|---|---|---|
| 5.548 | $-(CH_2)_4-$ | | H | $o-CH_3O-C_6H_4$ | $CH(CH_3)-COOCH_3$ | |
| 5.549 | $-(CH_2)_4-$ | | H | $o-CH_3O-C_6H_4$ | H | |
| 5.550 | $-(CH_2)_4-$ | | H | $m-CH_3O-C_6H_4$ | $CH_3$ | |
| 5.551 | $-(CH_2)_4-$ | | H | $m-CH_3O-C_6H_4$ | $C_3H_7(i)$ | |
| 5.552 | $-(CH_2)_4-$ | | H | $m-CH_3O-C_6H_4$ | $CH_2-CH=CH_2$ | |
| 5.553 | $-(CH_2)_4-$ | | H | $m-CH_3O-C_6H_4$ | $CH_2-CCl=CH_2$ | |
| 5.554 | $-(CH_2)_4-$ | | H | $m-CH_3O-C_6H_4$ | $CH_2-CH=CHCl$ | |
| 5.555 | $-(CH_2)_4-$ | | H | $m-CH_3O-C_6H_4$ | $CH_2-C\equiv CH$ | |
| 5.556 | $-(CH_2)_4-$ | | H | $m-CH_3O-C_6H_4$ | $CH_2-COOCH_3$ | |
| 5.557 | $-(CH_2)_4-$ | | H | $m-CH_3O-C_6H_4$ | $CH(CH_3)-COOCH_3$ | |
| 5.558 | $-(CH_2)_4-$ | | H | $m-CH_3O-C_6H_4$ | H | |
| 5.559 | $-(CH_2)_4-$ | | H | $p-CH_3O-C_6H_4$ | $CH_3$ | |
| 5.560 | $-(CH_2)_4-$ | | H | $p-CH_3O-C_6H_4$ | $C_3H_7(i)$ | |
| 5.561 | $-(CH_2)_4-$ | | H | $p-CH_3O-C_6H_4$ | $CH_2-CH=CH_2$ | |
| 5.562 | $-(CH_2)_4-$ | | H | $p-CH_3O-C_6H_4$ | $CH_2-CCl=CH_2$ | |
| 5.563 | $-(CH_2)_4-$ | | H | $p-CH_3O-C_6H_4$ | $CH_2-CH=CHCl$ | |
| 5.564 | $-(CH_2)_4-$ | | H | $p-CH_3O-C_6H_4$ | $CH_2-C\equiv CH$ | |
| 5.565 | $-(CH_2)_4-$ | | H | $p-CH_3O-C_6H_4$ | $CH_2-COOCH_3$ | |
| 5.566 | $-(CH_2)_4-$ | | H | $p-CH_3O-C_6H_4$ | $CH(CH_3)-COOCH_3$ | |
| 5.567 | $-(CH_2)_4-$ | | H | $p-CH_3O-C_6H_4$ | H | |
| 5.568 | $-(CH_2)_4-$ | | H | $o-F-C_6H_4$ | $CH_3$ | |
| 5.569 | $-(CH_2)_4-$ | | H | $o-F-C_6H_4$ | $C_3H_7(i)$ | |
| 5.570 | $-(CH_2)_4-$ | | H | $o-F-C_6H_4$ | $CH_2-CH=CH_2$ | |
| 5.571 | $-(CH_2)_4-$ | | H | $o-F-C_6H_4$ | $CH_2-CCl=CH_2$ | |
| 5.572 | $-(CH_2)_4-$ | | H | $o-F-C_6H_4$ | $CH_2-CH=CHCl$ | |
| 5.573 | $-(CH_2)_4-$ | | H | $o-F-C_6H_4$ | $CH_2-C\equiv CH$ | |
| 5.574 | $-(CH_2)_4-$ | | H | $o-F-C_6H_4$ | $CH_2-COOCH_3$ | |
| 5.575 | $-(CH_2)_4-$ | | H | $o-F-C_6H_4$ | $CH(CH_3)-COOCH_3$ | |
| 5.576 | $-(CH_2)_4-$ | | H | $o-F-C_6H_4$ | H | |
| 5.577 | $-(CH_2)_4-$ | | H | $m-F-C_6H_4$ | $CH_3$ | |
| 5.578 | $-(CH_2)_4-$ | | H | $m-F-C_6H_4$ | $C_3H_7(i)$ | |

| Nr. | $R^1$ $R^2$ | $R^3$ | $R^4$ | $R^7$ | Phys. Daten |
|---|---|---|---|---|---|
| 5.579 | $-(CH_2)_4-$ | H | m-F-$C_6H_4$ | $CH_2$-CH=$CH_2$ | |
| 5.580 | $-(CH_2)_4-$ | H | m-F-$C_6H_4$ | $CH_2$-CCl=$CH_2$ | |
| 5.581 | $-(CH_2)_4-$ | H | m-F-$C_6H_4$ | $CH_2$-CH=CHCl | |
| 5.582 | $-(CH_2)_4-$ | H | m-F-$C_6H_4$ | $CH_2$-C≡CH | |
| 5.583 | $-(CH_2)_4-$ | H | m-F-$C_6H_4$ | $CH_2$-$COOCH_3$ | |
| 5.584 | $-(CH_2)_4-$ | H | m-F-$C_6H_4$ | $CH(CH_3)$-$COOCH_3$ | |
| 5.585 | $-(CH_2)_4-$ | H | m-F-$C_6H_4$ | H | |
| 5.586 | $-(CH_2)_4-$ | H | p-F-$C_6H_4$ | $CH_3$ | |
| 5.587 | $-(CH_2)_4-$ | H | p-F-$C_6H_4$ | $C_3H_7$(i) | |
| 5.588 | $-(CH_2)_4-$ | H | p-F-$C_6H_4$ | $CH_2$-CH=$CH_2$ | |
| 5.589 | $-(CH_2)_4-$ | H | p-F-$C_6H_4$ | $CH_2$-CCl=$CH_2$ | |
| 5.590 | $-(CH_2)_4-$ | H | p-F-$C_6H_4$ | $CH_2$-CH=CHCl | |
| 5.591 | $-(CH_2)_4-$ | H | p-F-$C_6H_4$ | $CH_2$-C≡CH | |
| 5.592 | $-(CH_2)_4-$ | H | p-F-$C_6H_4$ | $CH_2$-$COOCH_3$ | |
| 5.593 | $-(CH_2)_4-$ | H | p-F-$C_6H_4$ | $CH(CH_3)$-$COOCH_3$ | |
| 5.594 | $-(CH_2)_4-$ | H | p-F-$C_6H_4$ | H | |
| 5.595 | $-(CH_2)_4-$ | H | 2,4-$(CH_3)_2$-$C_6H_3$ | $CH_3$ | |
| 5.596 | $-(CH_2)_4-$ | H | 2,4-$(CH_3)_2$-$C_6H_3$ | $C_3H_7$(i) | |
| 5.597 | $-(CH_2)_4-$ | H | 2,4-$(CH_3)_2$-$C_6H_3$ | $CH_2$-CH=$CH_2$ | |
| 5.598 | $-(CH_2)_4-$ | H | 2,4-$(CH_3)_2$-$C_6H_3$ | $CH_2$-CCl=$CH_2$ | |
| 5.599 | $-(CH_2)_4-$ | H | 2,4-$(CH_3)_2$-$C_6H_3$ | $CH_2$-CH=CHCl | |
| 5.600 | $-(CH_2)_4-$ | H | 2,4-$(CH_3)_2$-$C_6H_3$ | $CH_2$-C≡CH | |
| 5.601 | $-(CH_2)_4-$ | H | 2,4-$(CH_3)_2$-$C_6H_3$ | $CH_2$-$COOCH_3$ | |
| 5.602 | $-(CH_2)_4-$ | H | 2,4-$(CH_3)_2$-$C_6H_3$ | $CH(CH_3)$-$COOCH_3$ | |
| 5.603 | $-(CH_2)_4-$ | H | 2,4-$(CH_3)_2$-$C_6H_3$ | H | |
| 5.604 | $-(CH_2)_4-$ | H | 2,4-$(CH_3O)_2$-$C_6H_3$ | $CH_3$ | |
| 5.605 | $-(CH_2)_4-$ | H | 2,4-$(CH_3O)_2$-$C_6H_3$ | $C_3H_7$(i) | |
| 5.606 | $-(CH_2)_4-$ | H | 2,4-$(CH_3O)_2$-$C_6H_3$ | $CH_2$-CH=$CH_2$ | |
| 5.607 | $-(CH_2)_4-$ | H | 2,4-$(CH_3O)_2$-$C_6H_3$ | $CH_2$-CCl=$CH_2$ | |
| 5.608 | $-(CH_2)_4-$ | H | 2,4-$(CH_3O)_2$-$C_6H_3$ | $CH_2$-CH=CHCl | |
| 5.609 | $-(CH_2)_4-$ | H | 2,4-$(CH_3O)_2$-$C_6H_3$ | $CH_2$-C≡CH | |

| Nr. | $R^1$ $R^2$ | $R^3$ | $R^4$ | $R^7$ | Phys. Daten |
|---|---|---|---|---|---|
| 5.610 | $-(CH_2)_4-$ | H | $2,4-(CH_3O)_2-C_6H_3$ | $CH_2-COOCH_3$ | |
| 5.611 | $-(CH_2)_4-$ | H | $2,4-(CH_3O)_2-C_6H_3$ | $CH(CH_3)-COOCH_3$ | |
| 5.612 | $-(CH_2)_4-$ | H | $2,4-(CH_3O)_2-C_6H_3$ | H | |
| 5.613 | $-(CH_2)_4-$ | H | $3,4-(CH_3)_2-C_6H_3$ | $CH_3$ | |
| 5.614 | $-(CH_2)_4-$ | H | $3,4-(CH_3)_2-C_6H_3$ | $C_3H_7(i)$ | |
| 5.615 | $-(CH_2)_4-$ | H | $3,4-(CH_3)_2-C_6H_3$ | $CH_2-CH=CH_2$ | |
| 5.616 | $-(CH_2)_4-$ | H | $3,4-(CH_3)_2-C_6H_3$ | $CH_2-CCl=CH_2$ | |
| 5.617 | $-(CH_2)_4-$ | H | $3,4-(CH_3)_2-C_6H_3$ | $CH_2-CH=CHCl$ | |
| 5.618 | $-(CH_2)_4-$ | H | $3,4-(CH_3)_2-C_6H_3$ | $CH_2-C\equiv CH$ | |
| 5.619 | $-(CH_2)_4-$ | H | $3,4-(CH_3)_2-C_6H_3$ | $CH_2-COOCH_3$ | |
| 5.620 | $-(CH_2)_4-$ | H | $3,4-(CH_3)_2-C_6H_3$ | $CH(CH_3)-COOCH_3$ | |
| 5.621 | $-(CH_2)_4-$ | H | $3,4-(CH_3)_2-C_6H_3$ | H | |
| 5.622 | $-(CH_2)_4-$ | H | $3,4-(CH_3O)_2-C_6H_3$ | $CH_3$ | |
| 5.623 | $-(CH_2)_4-$ | H | $3,4-(CH_3O)_2-C_6H_3$ | $C_3H_7(i)$ | |
| 5.624 | $-(CH_2)_4-$ | H | $3,4-(CH_3O)_2-C_6H_3$ | $CH_2-CH=CH_2$ | |
| 5.625 | $-(CH_2)_4-$ | H | $3,4-(CH_3O)_2-C_6H_3$ | $CH_2-CCl=CH_2$ | |
| 5.626 | $-(CH_2)_4-$ | H | $3,4-(CH_3O)_2-C_6H_3$ | $CH_2-CH=CHCl$ | |
| 5.627 | $-(CH_2)_4-$ | H | $3,4-(CH_3O)_2-C_6H_3$ | $CH_2-C\equiv CH$ | |
| 5.628 | $-(CH_2)_4-$ | H | $3,4-(CH_3O)_2-C_6H_3$ | $CH_2-COOCH_3$ | |
| 5.629 | $-(CH_2)_4-$ | H | $3,4-(CH_3O)_2-C_6H_3$ | $CH(CH_3)-COOCH_3$ | |
| 5.630 | $-(CH_2)_4-$ | H | $3,4-(CH_3O)_2-C_6H_3$ | H | |
| 5.631 | $-(CH_2)_4-$ | H | $2,3,5-(CH_3)_3-C_6H_2$ | $CH_3$ | |
| 5.632 | $-(CH_2)_4-$ | H | $2,3,5-(CH_3)_3-C_6H_2$ | $C_3H_7(i)$ | |
| 5.633 | $-(CH_2)_4-$ | H | $2,3,5-(CH_3)_3-C_6H_2$ | $CH_2-CH=CH_2$ | |
| 5.634 | $-(CH_2)_4-$ | H | $2,3,5-(CH_3)_3-C_6H_2$ | $CH_2-CCl=CH_2$ | |
| 5.635 | $-(CH_2)_4-$ | H | $2,3,5-(CH_3)_3-C_6H_2$ | $CH_2-CH=CHCl$ | |
| 5.636 | $-(CH_2)_4-$ | H | $2,3,5-(CH_3)_3-C_6H_2$ | $CH_2-C\equiv CH$ | |
| 5.637 | $-(CH_2)_4-$ | H | $2,3,5-(CH_3)_3-C_6H_2$ | $CH_2-COOCH_3$ | |
| 5.638 | $-(CH_2)_4-$ | H | $2,3,5-(CH_3)_3-C_6H_2$ | $CH(CH_3)-COOCH_3$ | |
| 5.639 | $-(CH_2)_4-$ | H | $2,3,5-(CH_3)_3-C_6H_2$ | H | |
| 5.640 | $-(CH_2)_4-$ | H | $3,5-(CH_3O)_2-C_6H_3$ | $CH_3$ | |

| Nr. | $R^1$ | $R^2$ | $R^3$ | $R^4$ | $R^7$ | Phys. Daten |
|---|---|---|---|---|---|---|
| 5.641 | $-(CH_2)_4-$ | | H | $3,5-(CH_3O)_2-C_6H_3$ | $C_3H_7(i)$ | |
| 5.642 | $-(CH_2)_4-$ | | H | $3,5-(CH_3O)_2-C_6H_3$ | $CH_2-CH=CH_2$ | |
| 5.643 | $-(CH_2)_4-$ | | H | $3,5-(CH_3O)_2-C_6H_3$ | $CH_2-CCl=CH_2$ | |
| 5.644 | $-(CH_2)_4-$ | | H | $3,5-(CH_3O)_2-C_6H_3$ | $CH_2-CH=CHCl$ | |
| 5.645 | $-(CH_2)_4-$ | | H | $3,5-(CH_3O)_2-C_6H_3$ | $CH_2-C{\equiv}CH$ | |
| 5.646 | $-(CH_2)_4-$ | | H | $3,5-(CH_3O)_2-C_6H_3$ | $CH_2-COOCH_3$ | |
| 5.647 | $-(CH_2)_4-$ | | H | $3,5-(CH_3O)_2-C_6H_3$ | $CH(CH_3)-COOCH_3$ | |
| 5.648 | $-(CH_2)_4-$ | | H | $3,5-(CH_3O)_2-C_6H_3$ | H | |

## F. Formulierungsbeispiele

### Beispiel F 1.: Formulierungsbeispiele für Wirkstoffe der Formel I

(% = Gewichtsprozent)

| a) Emulsionskonzentrate | a) | b) | c) |
|---|---|---|---|
| Wirkstoff gemäss Tabelle 1 bis 5 | 20 % | 40 % | 50 % |
| Ca-Dodecylbenzolsulfonat | 5 % | 8 % | 5,8 % |
| Ricinusöl-polyethylenglykolether (36 Mol EO) | 5 % | - | - |
| Tributylphenoyl-polyethylenglykolether (30 Mol EO) | - | 12 % | 4,2 % |
| Cyclohexanon | - | 15 % | 20 % |
| Xylolgemisch | 70 % | 25 % | 20 % |

Aus solchen Konzentraten können durch Verdünnen mit Wasser Emulsionen jeder gewünschten Konzentration hergestellt werden.

| b) Lösungen | a) | b) | c) |
|---|---|---|---|
| Wirkstoff gemäss Tabelle 1 bis 5 | 80 % | 10 % | 5 % |
| Ethylenglykol-monomethylether | 20 % | - | - |
| Polyethylenglykol MG 400 | - | 70 % | - |
| N-Methyl-2-pyrrolidon | - | 20 % | 5 % |
| Epoxidiertes Kokosnussöl | - | - | 90 % |

Die Lösungen sind zur Anwendung in Form kleinster Tropfen geeignet.

| c) Granulate | a) | b) |
|---|---|---|
| Wirkstoff gemäss Tabelle 1 bis 5 | 5 % | 10 % |
| Kaolin | 94 % | - |
| Hochdisperse Kieselsäure | 1 % | |
| Attapulgit | - | 90 % |

Der Wirkstoff wird gelöst, auf den Träger aufgesprüht und das Lösungsmittel anschliessend im Vakuum abgedampft.

| d) Stäubemittel | a) | b) | c) |
|---|---|---|---|
| Wirkstoff gemäss Tabelle 1 bis 5 | 2 % | 5 % | 8 % |
| Hochdisperse Kieselsäure | 1 % | 5 % | 5 % |
| Talkum | 97 % | - | 10 % |
| Kaolin | - | 90 % | 77 % |

Durch inniges Vermischen der Trägerstoffe mit dem Wirkstoff erhält man gebrauchsfertiges Stäubemittel.

e) Spritzpulver

| | a) | b) |
|---|---|---|
| Wirkstoff gemäss Tabelle 1 bis 5 | 20 % | 60 % |
| Na-Ligninsulfonat | 5 % | 5 % |
| Na-Laurylsulfat | 3 % | 6 % |
| Octylphenolpolyethylenglykolether (7-8 Mol EO) | - | 2 % |
| Hochdisperse Kieselsäure | 5 % | 27 % |
| Kaolin | 67 % | - |

Der Wirkstoff wird mit den Zusatzstoffen gut vermischt und in einer geeigneten Mühle gut vermahlen. Man erhält Spritzpulver, die sich mit Wasser zur Suspension jeder gewünschten Konzentration verdünnen lassen.

f) Extruder Granulat

Wirkstoff gemäss Tabelle 1 bis 5   10 %
Na-Ligninsulfonat   2 %
Carboxymethylcellulose   1 %
Kaolin   87 %

Der Wirkstoff wird mit den Zusatzstoffen vermischt, vermahlen und mit Wasser angefeuchtet. Dieses Gemisch wird extrudiert und anschliessend im Luftstrom getrocknet.

g) Umhüllungs-Granulat

Wirkstoff gemäss Tabelle 1 bis 5   3 %
Polyethylenglykol (MG 200)   3 %
Kaolin   94 %

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

h) Suspensions-Konzentrat

Wirkstoff gemäss Tabelle 1 bis 5   40 %
Ethylenglykol   10 %
Nonylphenolpolyethylenglykolether (15 Mol EO)   6 %
Na-Ligninsulfonat   10 %
Carboxymethylcellulose   1 %
37 %ige wässrige Formaldehyd-Lösung   0,2 %
Silikonöl in Form einer 75 %igen wässrigen Emulsion   0,8%
Wasser   32 %

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

B. Biologische Beispiele

Beispiel B 1: Post-emergente Herbizid-Wirkung

Eine Anzahl Unkräuter, sowohl monokotyle wir dikotyle, werden nach dem Auflaufen (im 4- bis 6-Blattstadium) mit einer wässrigen Wirkstoffdispersion in einer Dosierung von 60 g bis 1 kg Wirksubstanz pro Hektar auf die Pflanzen gespritzt und diese bei 24°-26°C und 45-60 % relativer Luftfeuchtigkeit gehalten. 15 Tage nach der Behandlung wird der Versuch ausgewertet. Auch in diesem Versuch zeigen die Verbindungen nach Tabellen 1 bis 5 starke bis sehr starke Herbizidwirkung.

Der Zustand der Pflanzen wird dabei gemäss folgender Bewertungsskala bonitiert:

1 Pflanze abgestorben oder hat nicht gekeimt

2-8 abnehmende Schadstufen
9 keine Schädigung, die Pflanze gedeiht wie unbehandelte Kontrollpflanzen.
Man findet z.B. für Verbindung No 1.7. nachstehende Testergebnisse:

| Testpflanze | Aufwandmenge [g/ha] | | |
|---|---|---|---|
| | 250 | 125 | 60 |
| Weizen | 9 | 9 | 9 |
| Chenopodium Sp. | 1 | 1 | 2 |
| Solanum nigrum | 1 | 1 | 1 |
| Viola tricolor | 1 | 1 | 1 |
| Veronica Sp. | 1 | 1 | 1 |

Beispiel B 2: Herbizidwirkung für Wasserreis (paddy)

Die Wasserunkräuter Echinochloa crus galli und Monocharia vag. werden in Plastikbechern (60 $cm^2$ Oberfläche, 500 ml Volumen) ausgesät. Nach der Saat wird bis zur Erdoberfläche mit Wasser aufgefüllt. 3 Tage nach der Saat wird der Wasserspiegel bis leicht über die Erdoberfläche erhöht (3-5 mm). Die Applikation erfolgt 3 Tage nach der Saat mit einer wässrigen Emulsion der Prüfsubstanzen durch eine Spritzung auf die Gefässe mit einer Aufwandmenge von 60 bis 250 g AS pro Hektar. Die Pflanzenbecher werden dann im Gewächshaus unter optimalen Wachstumsbedingungen für die Reisunkräuter aufgestellt, d.h. bei 25°-30°C und hoher Luftfeuchtigkeit. Die Auswertung der Versuche findet 3 Wochen nach Applikation statt. Die Verbindungen gemäss Tabelle 1 bis 5 schädigen dabei die Unkräuter, nicht aber den Reis.
Man findet z.B. für Verbindung No. 1.4. nachstehende Testergebnisse:

| Testpflanze | Aufwandmenge [g/ha] | | |
|---|---|---|---|
| | 250 | 125 | 60 |
| Rèis (verpflanzt) | 8 | 9 | 9 |
| Scirpus | 1 | 1 | 2 |
| Monochoria | 1 | 1 | 1 |

**Patentansprüche**

1. Verbindungen der Formel I

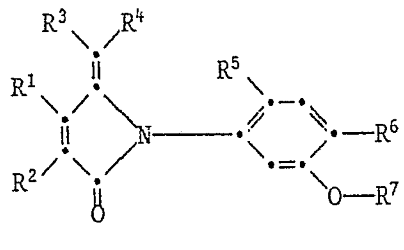

(I),

worin
$R^1$ Wasserstoff; $(C_1$-$C_8)$-Alkyl;

$R^2$ Wasserstoff; $(C_1-C_8)$-Alkyl; oder
$R^1$ und $R^2$ gemeinsam eine gegebenenfalls bis zu zweifach durch $(C_1-C_4)$-Alkyl substituierte $-(CH_2)_4$-Brücke;
$R^3$ und $R^4$ unabhängig voneinander Wasserstoff; oder $(C_1-C_4)$-Alkyl; oder
einer der Reste $R^3$ oder $R^4$ Wasserstoff und der andere ein gegebenenfalls bis zu dreifach gleich oder verschieden durch Halogen, $(C_1-C_4)$-Alky oder $(C_1-C_4)$-Alkoxy substituierter Phenylrest; oder
$R^3$ und $R^4$ zusammen eine $-(CH_2)_n$-Brücke, mit n = 2, 3, 4, 5 oder 6, die gewünschtenfalls bis zu dreifach durch $(C_1-C_4)$-Alky substituiert sein kann;
$R^5$ Wasserstoff; oder Fluor;
$R^6$ Halogen;
$R^7$ Wasserstoff; $(C_1-C_8)$-Alkyl; $(C_3-C_8)$-Alkenyl; $(C_3-C_8)$-Alkinyl; $(C_3-C_7)$-Cycloalkyl; $(C_1-C_4)$-Alkyl, einfach substituiert durch: $(C_1-C_4)$-Alkoxy, $(C_1-C_4)$-Alkylthio, mono-$(C_1-C_4)$-Alkylamino, di-$(C_1-C_4)$-Alkylamino, Cyano, $(C_3-C_7)$-Cycloalkyl, Phenyl, Halogenphenyl, $(C_1-C_4)$-Alkylphenyl, $(C_1-C_4)$-Alkoxyphenyl, $(C_1-C_4)$-Halogenalkylphenyl, $(C_1-C_4)$-Halogenalkoxyphenyl, Nitrophenyl, Cyanophenyl, Hydroxy, 1,3-Dioxolan-2-yl, 1,3-Dioxan-2-yl oder CO-$R^8$; $(C_1-C_8)$-Alkyl, $(C_3-C_8)$-Alkenyl oder $(C_3-C_7)$-Cycloalkyl, welche ein oder mehrfach durch Halogen substituiert sind; oder CO-$R^8$.
$R^8$ $(C_1-C_8)$-Alkyl; $(C_3-C_8)$-Alkenyl; Benzyl, gegebenenfalls am Phenylring einfach substituiert durch Halogen, $(C_1-C_4)$-Alkyl, $(C_1-C_4)$-Alkoxy, $(C_1-C_4)$-Halogenalkyl, Nitro oder Cyano; $(C_3-C_7)$-Cycloalkyl; Phenyl, gegebenenfalls bis zu dreifach substituiert durch Halogen, $(C_1-C_8)$-Alkyl, $(C_1-C_4)$-Alkoxy, $(C_1-C_4)$-Halogenalkyl oder einfach substituiert durch Cyano oder Nitro; Furan; Thiophen; $(C_1-C_8)$-Alkoxy; $(C_1-C_4)$-Alkoxy-$(C_1-C_4)$-alkoxy; $(C_3-C_8)$-Alkenyloxy; $(C_3-C_8)$-Alkinyloxy; $(C_1-C_8)$-Alkylthio; $(C_3-C_8)$-Alkenylthio; $(C_3-C_8)$-Alkinylthio; Amino; mono$(C_1-C_4)$-Alkylamino; di-$(C_1-C_4)$-Alkylamino; Phenylamino, gegebenenfalls bis zu dreifach substituiert durch Halogen, $(C_1-C_4)$-Alkyl, $(C_1-C_4)$-Alkoxy oder $(C_1-C_4)$-Halogenalkyl oder einfach substituiert durch Cyano oder Nitro;
bedeutet.

2. 2-(4-Chlor-2-fluor-5-isopropyloxyphenyl)-3-methylen-4,5,6,7-tetrahydroisoindol-(2H)1-on,
2-(4-Chlor-2-fluor-5-propargyloxyphenyl)-3-methylen-4,5,6,7-tetrahydroisoindol-(2H)1-on,
2-[4-Chlor-2-fluor-5-(2-methylpropyloxy)phenyl]-3-methylen-4,5,6,7-tetrahydroisoindol-(2H)1-on,
2-[4-Chlor-2-fluor-5-(2-chlorprop-2-enyloxy)-phenyl]-4,5,6,7-tetrahydroisoindol-(2H)1-on,
2-[4-Chlor-2-fluor-5-(1-methoxycarbonylethoxy)phenyl]-4,5,6,7-tetrahydroisoindol-(2H)1-on,
2-[4-Chlor-2-fluor-5-(3-chlorprop-2-enyloxy)-phenyl]-4,5,6,7-tetrahydroisoindol-(2H)1-on,
2-[4-Chlor-2-fluor-5-(prop-2-inyloxy)-phenyl]-4,5,6,7-tetrahydroisoindol-(2H)1-on,
1-(4-Chlor-2-fluor-5-isopropyloxyphenyl)-3,4-dimethyl-5-methylenpyrrol-(1H)2-on,
2-(4-Chlor-2-fluor-5-isopropyloxyphenyl)-3-(1,1-dimethylmethylen)-4,5,6,7-tetrahydroisoindol-(2H)1-on,
2-(4-Chlor-2-fluor-5-isopropyloxyphenyl)-3-ethyliden-4,5,6,7-tetrahydroisoindol-(2H)1-on,
5-Benzyliden-1-(4-chlor-2-fluor-5-isopropyloxy-phenyl)-3,4-dimethylpyrrol-(1H)2-on oder
5-(o-Chlorbenzyliden)-1-(4-chlor-2-fluor-5-propargyloxy-phenyl)-3,5-dimethyl-pyrrol-(1H)2-on als Verbindung der Formel I gemäss Anspruch 1.

3. Verfahren zur Herstellung von Verbindungen der Formel I gemäss Anspruch 1, gekennzeichnet
a) durch die Alkylierung oder Acylierung eines Phenols der Formel Ia,

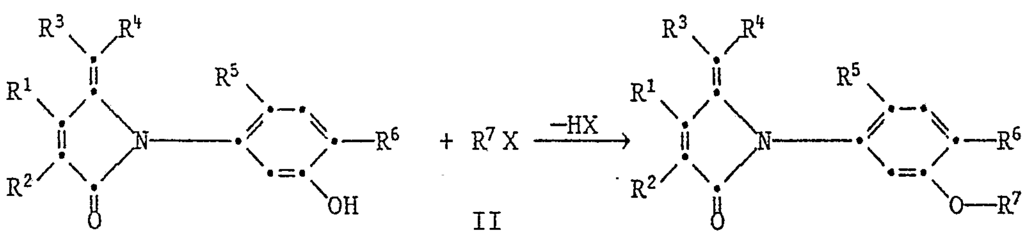


Ia ($R^7$ = H) I

worin die Reste $R^1$ bis $R^6$ wie zuvor definiert sind, mit einer Verbindung der Formel II, worin $R^7$ wie zuvor definiert ist, jedoch nicht für Wasserstoff und X für eine unter der Reaktionsbedingungen abspaltbare Gruppe steht, unter Basenzusatz umsetzt, wobei X vorzugsweise Halogen, wie Chlor, Brom oder Jod oder einen gewünschtenfalls im Phenylkern alkylierten oder halogenierten Phenylsulfonylrest, ein Radikal der Formel $R^7OSO_2$-O oder wenn $R^7$ für

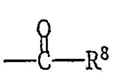 steht auch eine Gruppe 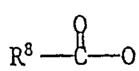

bedeutet oder
b) durch Umsetzung eines 5-Chlor-2,5-dihydrofuran-2-ons der Formel VIII, worin die Reste $R^1$ bis $R^4$ wie zuvor definiert sind mit einem Anilin der Formel VII, worin die Reste $R^5$ bis $R^7$ wie zuvor definiert sind

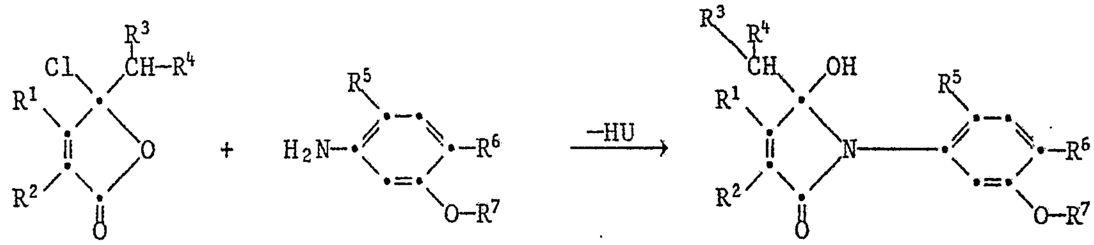


VIII VII V

V

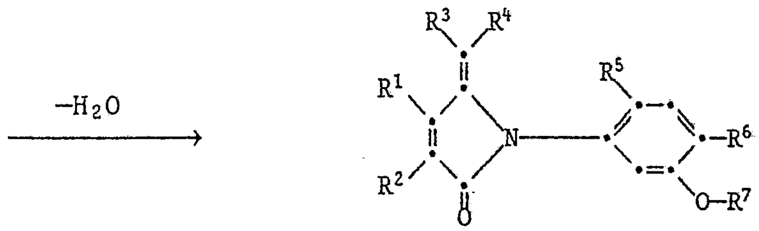


zu einem Primäraddukt der Formel V und anschliessender Wasserabspaltung zu I.

4. Verfahren zur Herstellung von Verbindungen der Formel Ia.

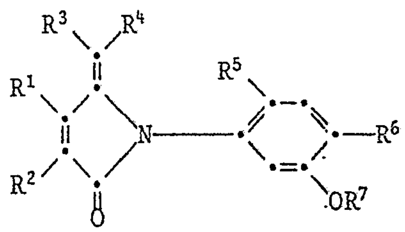

(Ia),

worin
$R^1$ Wasserstoff; ($C_1$-$C_8$)-Alkyl;
$R^2$ Wasserstoff; ($C_1$-$C_8$)-Alkyl; oder
$R^1$ und $R^2$ gemeinsam eine gegebenenfalls bis zu zweifach durch ($C_1$-$C_4$)-Alkyl substituierte -$(CH_2)_4$-Brücke;
$R^3$ und $R^4$ unabhängig voneinander Wasserstoff; oder ($C_1$-$C_4$)-Alkyl; oder
einer der Reste $R^3$ oder $R^4$ Wasserstoff und der andere ein gegebenenfalls bis zu dreifach gleich oder verschieden durch Halogen, ($C_1$-$C_4$)-Alkyl oder ($C_1$-$C_4$)-Alkoxy substituierter Phenylrest; oder
$R^3$ und $R^4$ zusammen eine -$(CH_2)_n$-Brücke, mit $n = 2, 3, 4, 5$ oder 6, die gewünschtenfalls bis zu dreifach durch ($C_1$-$C_4$)-Alkyl substituiert sein kann;
$R^5$ Wasserstoff; oder Fluor;
$R^6$ Halogen;
$R^7$ Wasserstoff; ($C_1$-$C_8$)-Alkyl; ($C_3$-$C_8$)-Alkenyl; ($C_3$-$C_8$)-Alkinyl; ($C_3$-$C_7$)-Cycloalkyl; ($C_1$-$C_4$)-Alkyl, einfach substituiert durch: ($C_1$-$C_4$)-Alkoxy, ($C_1$-$C_4$)-Alkylthio, mono-($C_1$-$C_4$)-Alkylamino, di-($C_1$-$C_4$)-Alkylamino, ($C_3$-$C_7$)-Cycloalkyl, Phenyl, Halogenphenyl, ($C_1$-$C_4$)-Alkylphenyl, ($C_1$-$C_4$)-Alkoxyphenyl, ($C_1$-$C_4$)-Halogenalkylphenyl, ($C_1$-$C_4$)-Halogenalkoxyphenyl, Nitrophenyl, Hydroxy, 1,3-Dioxolan-2-yl oder 1,3-Dioxan-2-yl; ($C_1$-$C_8$)-Alkyl, ($C_3$-$C_8$)-Alkenyl oder ($C_3$-$C_7$)-Cycloalkyl, welche ein oder mehrfach durch Halogen substituiert sind;
bedeutet, gekennzeichnet durch die Umsetzung einer Verbindung der Formel III

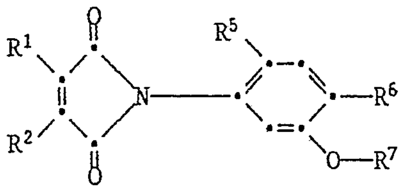 (III),

worin $R^1$, $R^2$, $R^5$, $R^6$ und $R^7$ wie zuvor definiert sind, mit einem Grignard-Reagenz der Formel IV

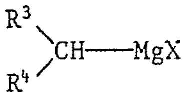 (IV),

worin $R^3$ und $R^4$ unabhängig voneinander Wasserstoff; oder $(C_1$-$C_4)$-Alkyl; $R^3$ Wasserstoff und $R^4$ einen gegebenenfalls bis zu dreifach gleich oder verschieden durch Halogen, $(C_1$-$C_4)$-Alkyl oder $(C_1$-$C_4)$-Alkoxy substituierten Phenylrest; oder $R^3$ und $R^4$ zusammen eine $-(CH_2)_n$-Brücke, mit $n = 2, 3, 4, 5$ oder 6, die gewünschtenfalls bis zu dreifach durch $(C_1$-$C_4)$-Alkyl substituiert sein kann; bedeutet, zu einer Verbindung der Formel V

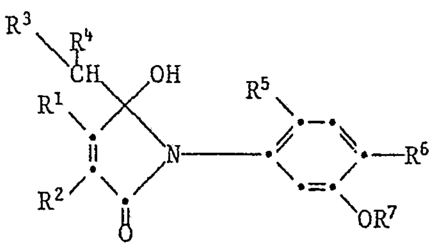 (V),

worin die Reste $R^1$ bis $R^7$ wie zuvor definiert sind, und anschliessender Wasserabspaltung zu Ia.

5. Verbindungen der Formel VIII

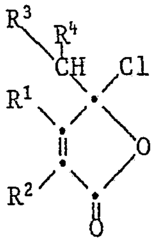 (VIII),

worin die Reste $R^1$ bis $R^4$ wie im Anspruch 1 definiert sind, mit der Massgabe, dass $R^1$ und $R^2$ nicht für Wasserstoff oder Methyl steht, wenn $R^3$ und $R^4$ Wasserstoff bedeutet.

6. Verbindungen der Formel XII

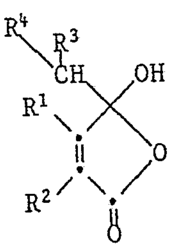 (XII),

worin die Reste $R^1$ bis $R^4$ wie in Anspruch 1 definiert sind, mit der Massgabe, dass $R^1$ und $R^2$ nicht für

Wasserstoff und Methyl oder $R^1$ und $R^2$ nicht gemeinsam für eine unsubstituierte $-(CH_2)_4$-Brücke stehen, wenn $R^3$ und $R^4$ Wasserstoff bedeuten.

7. Verbindungen der Formel V

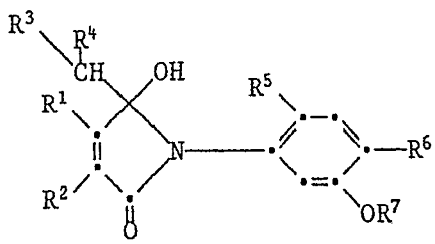

(V),

worin die Reste $R^1$, $R^2$, $R^3$, $R^4$, $R^5$, $R^6$ und $R^7$ wie in Anspruch 1 definiert sind.

8. Herbizides Mittel, enthaltend eine Verbindung der Formel I gemäss einem der Ansprüche 1 oder 2 neben weiteren Hilfs- und/oder Trägerstoffen.

9. Verwendung von Verbindungen der Formel I gemäss einem der Ansprüche 1 oder 2 zur Bekämpfung unerwünschten Pflanzenwuchses.

10. Verfahren zur Bekämpfung unerwünschten Pflanzenwuchses, dadurch gekennzeichnet, dass man eine Verbindung der Formel I gemäss Anspruch 1 oder 2 oder ein Mittel gemäss Anspruch 8 auf den Lebensraum der zu bekämpfenden Pflanze oder auf die zu bekämpfende Pflanze einwirken lässt.

11. Saatgut gekennzeichnet durch einen herbizid oder wuchsregulatorisch wirksamen Gehalt an einer Verbindung der Formel I gemäss einem der Ansprüche 1 oder 2.

12. Pflanzenwuchsregulatorisches Mittel enthaltend als Aktivsubstanz eine Verbindung der Formel I gemäss einem der Ansprüche 1 oder 2 neben weiteren Hilfs- und Trägerstoffen.

13. Verfahren zur Beeinflussung des Wachstums von Kulturpflanzen, dadurch gekennzeichnet, dass man eine wuchsregulatorisch wirksame Menge einer Verbindung der Formel I gemäss einem der Ansprüche 1 oder 2 oder eines Mittels gemäss Anspruch 12 auf die Kulturpflanze oder deren Lebensraum einwirken lässt.

**Patenansprüche für den folgenden Vertragsstaat ES:**

1. Verfahren zur Hestellung von Verbindungen der Formel I

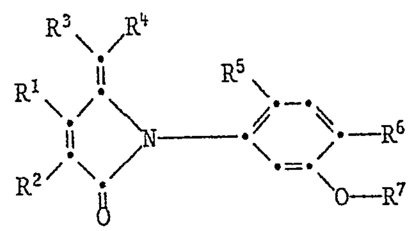

(I),

worin
$R^1$ Wasserstoff; $(C_1-C_8)$-Alkyl;
$R^2$ Wasserstoff; $(C_1-C_8)$-Alkyl; oder
$R^1$ und $R^2$ gemeinsam eine gegebenenfalls bis zu zweifach durch $(C_1-C_4)$-Alkyl substituierte $-(CH_2)_4$-Brücke;
$R^3$ und $R^4$ unabhängig voneinander Wasserstoff; oder $(C_1-C_4)$-Alkyl; oder
einer der Reste $R^3$ oder $R^4$ Wasserstoff und der andere ein gegebenenfalls bis zu dreifach gleich oder verschieden durch Halogen, $(C_1-C_4)$-Alky oder $(C_1-C_4)$-Alkoxy substituierter Phenylrest; oder
$R^3$ und $R^4$ zusammen eine $-(CH_2)_n$-Brücke, mit $n = 2, 3, 4, 5$ oder 6, die gewünschtenfalls bis zu dreifach durch $(C_1-C_4)$-Alky substituiert sein kann;
$R^5$ Wasserstoff; oder Fluor;
$R^6$ Halogen;
$R^7$ Wasserstoff; $(C_1-C_8)$-Alkyl; $(C_3-C_8)$-Alkenyl; $(C_3-C_8)$-Alkinyl; $(C_3-C_7)$-Cycloalkyl; $(C_1-C_4)$-Alkyl, einfach substituiert durch: $(C_1-C_4)$-Alkoxy, $(C_1-C_4)$-Alkylthio, mono-$(C_1-C_4)$-Alkylamino, di-$(C_1-C_4)$-Alkylamino, Cyano, $(C_3-C_7)$-Cycloalkyl, Phenyl, Halogenphenyl, $(C_1-C_4)$-Alkylphenyl, $(C_1-C_4)$-Alkoxyphenyl, $(C_1-C_4)$-Halogenalkylphenyl, $(C_1-C_4)$-Halogenalkoxyphenyl, Nitrophenyl, Cyanophenyl, Hydroxy, 1,3-Dioxolan-2-yl, 1,3-Dioxan-2-yl oder CO-$R^8$; $(C_1-C_8)$-Alkyl, $(C_3-C_8)$-Alkenyl oder $(C_3-C_7)$-Cycloalkyl, welche ein oder mehrfach durch Halogen substituiert sind; oder CO-$R^8$.
$R^8$ $(C_1-C_8)$-Alkyl; $(C_3-C_8)$-Alkenyl; Benzyl, gegebenenfalls am Phenylring einfach substituiert durch Halogen, $(C_1-C_4)$-Alkyl, $(C_1-C_4)$-Alkoxy, $(C_1-C_4)$-Halogenalkyl, Nitro oder Cyano; $(C_3-C_7)$-Cycloalkyl; Phenyl, gegebe-

nenfalls bis zu dreifach substituiert durch Halogen, $(C_1-C_8)$-Alkyl, $(C_1-C_4)$-Alkoxy, $(C_1-C_4)$-Halogenalkyl oder einfach substituiert durch Cyano oder Nitro; Furan; Thiophen; $(C_1-C_8)$-Alkoxy; $(C_1-C_4)$-Alkoxy-$(C_1-C_4)$-alkoxy; $(C_3-C_8)$-Alkenyloxy; $(C_3-C_8)$-Alkinyloxy; $(C_1-C_8)$-Alkylthio; $(C_3-C_8)$-Alkenylthio; $(C_3-C_8)$-Alkinylthio; Amino; mono$(C_1-C_4)$-Alkylamino; di-$(C_1-C_4)$-Alkylamino; Phenylamino, gegebenenfalls bis zu dreifach substituiert durch Halogen, $(C_1-C_4)$-Alkyl, $(C_1-C_4)$-Alkoxy oder $(C_1-C_4)$-Halogenalkyl oder einfach substituiert durch Cyano oder Nitro;
bedeutet, gekennzeichnet

a) durch die Alkylierung oder Acylierung eines Phenols der Formel Ia,

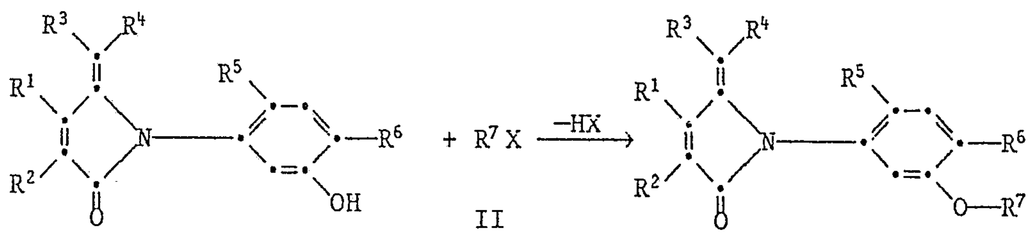


Ia ($R^7$ = H) II I

worin die Reste $R^1$ bis $R^6$ wie zuvor definiert sind, mit einer Verbindung der Formel II, worin $R^7$ wie zuvor definiert ist, jedoch nicht für Wasserstoff und X für eine unter der Reaktionsbedingungen abspaltbare Gruppe steht, unter Basenzusatz umsetzt, wobei X vorzugsweise Halogen, wie Chlor, Brom oder Jod oder einen gewünschtenfalls im Phenylkern alkylierten oder halogenierten Phenylsulfonylrest, ein Radikal der Formel $R^7OSO_2$-O oder wenn $R^7$ für

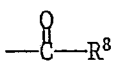 steht auch eine Gruppe 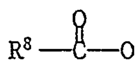

bedeutet oder

b) durch Umsetzung eines 5-Chlor-2,5-dihydrofuran-2-ons der Formel VIII, worin die Reste $R^1$ bis $R^4$ wie zuvor definiert sind mit einem Anilin der Formel VII, worin die Reste $R^5$ bis $R^7$ wie zuvor definiert sind

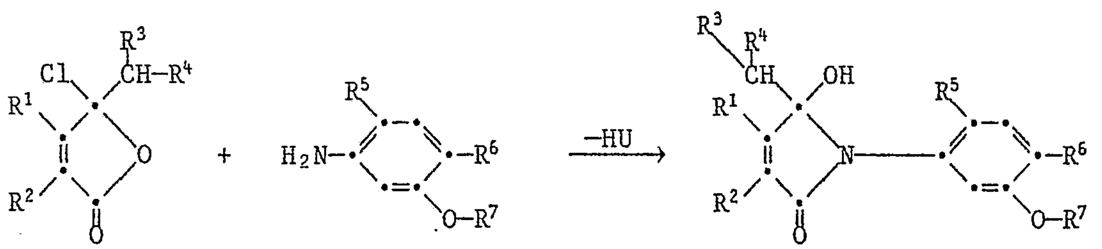


VIII VII V

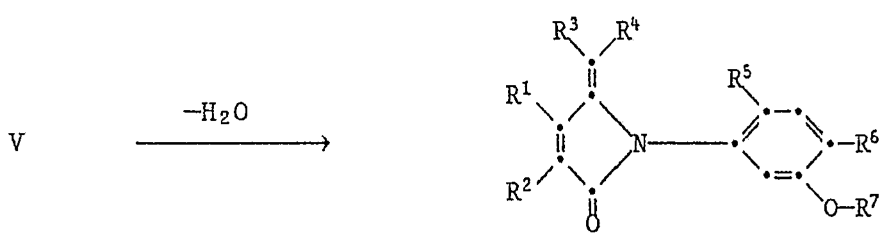


zu einem Primäraddukt der Formel V und anschliessender Wasserabspaltung zu I.

2. Verfahren zur Herstellung von Verbindungen der Formel Ia.

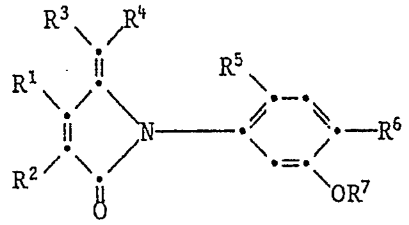 (Ia),

worin
$R^1$ Wasserstoff; ($C_1$-$C_8$)-Alkyl;
$R^2$ Wasserstoff; ($C_1$-$C_8$)-Alkyl; oder
$R^1$ und $R^2$ gemeinsam eine gegebenenfalls bis zu zweifach durch ($C_1$-$C_4$)-Alkyl substituierte -$(CH_2)_4$-Brücke;
$R^3$ und $R^4$ unabhängig voneinander Wasserstoff; oder ($C_1$-$C_4$)-Alkyl; oder
einer der Reste $R^3$ oder $R^4$ Wasserstoff und der andere ein gegebenenfalls bis zu dreifach gleich oder verschieden durch Halogen, ($C_1$-$C_4$)-Alkyl oder ($C_1$-$C_4$)-Alkoxy substituierter Phenylrest; oder
$R^3$ und $R^4$ zusammen eine -$(CH_2)_n$-Brücke, mit n = 2, 3, 4, 5 oder 6, die gewünschtenfalls bis zu dreifach durch ($C_1$-$C_4$)-Alkyl substituiert sein kann;
$R^5$ Wasserstoff; oder Fluor;
$R^6$ Halogen;
$R^7$ Wasserstoff; ($C_1$-$C_8$)-Alkyl; ($C_3$-$C_8$)-Alkenyl; ($C_3$-$C_8$)-Alkinyl; ($C_3$-$C_7$)-Cycloalkyl; ($C_1$-$C_4$)-Alkyl, einfach substituiert durch: ($C_1$-$C_4$)-Alkoxy, ($C_1$-$C_4$)-Alkylthio, mono-($C_1$-$C_4$)-Alkylamino, di-($C_1$-$C_4$)-Alkylamino, ($C_3$-$C_7$)-Cycloalkyl, Phenyl, Halogenphenyl, ($C_1$-$C_4$)-Alkylphenyl, ($C_1$-$C_4$)-Alkoxyphenyl, ($C_1$-$C_4$)-Halogenalkylphenyl, ($C_1$-$C_4$)-Halogenalkoxyphenyl, Nitrophenyl, Hydroxy, 1,3-Dioxo lan-2-yl oder 1,3-Dioxan-2-yl; ($C_1$-$C_8$)-Alkyl, ($C_3$-$C_8$)-Alkenyl oder ($C_3$-$C_7$)-Cycloalkyl, welche ein oder mehrfach durch Halogen substi tuiert sind;
bedeutet, gekennzeichnet durch die Umsetzung einer Verbindung der Formel III

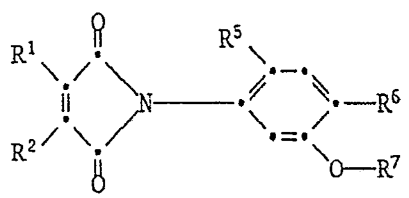 (III),

worin $R^1$, $R^2$, $R^5$, $R^6$ und $R^7$ wie zuvor definiert sind, mit einem Grignard-Reagenz der Formel IV

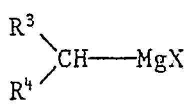 (IV),

worin $R^3$ und $R^4$ unabhängig voneinander Wasserstoff; oder ($C_1$-$C_4$)-Alkyl; $R^3$ Wasserstoff und $R^4$ einen gegebenenfalls bis zu dreifach gleich oder verschieden durch Halogen, ($C_1$-$C_4$)-Alkyl oder ($C_1$-$C_4$)-Alkoxy substituierten Phenylrest; oder $R^3$ und $R^4$ zusammen eine -$(CH_2)_n$-Brücke, mit n = 2, 3, 4, 5 oder 6, die gewünschtenfalls bis zu dreifach durch ($C_1$-$C_4$)-Alkyl substituiert sein kann; bedeutet, zu einer Verbindung der Formel V

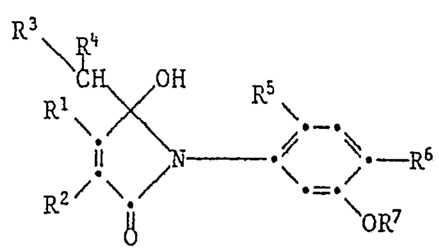 (V),

worin die Reste $R^1$ bis $R^7$ wie zuvor definiert sind, und anschliessender Wasserabspaltung zu Ia.

3. Verfahren zur Herstellung von Verbindungen der Formel VIII

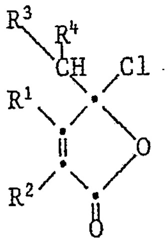

(VIII),

worin die Reste $R^1$ bis $R^4$ wie im Anspruch 1 definiert sind, mit der Massgabe, dass $R^1$ und $R^2$ nicht für Wasserstoff oder Methyl steht, wenn $R^3$ und $R^4$ Wasserstoff bedeutet, dadurch gekennzeichnet, dass man eine Verbindung der Formel XII

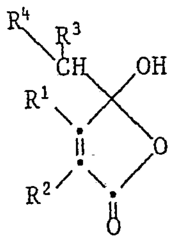

(XII),

worin die Reste $R^1$ bis $R^4$ wie in Anspruch 1 definiert sind, mit $SOCl_2$ chloriert.

4. Verfahren zur Herstellung von Verbindungen der Formel XII

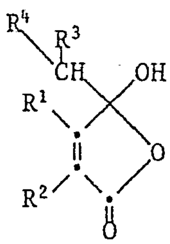

(XII),

worin die Reste $R^1$ bis $R^4$ wie im Anspruch 1 definiert sind, mit der Massgabe, dass $R^1$ und $R^2$ nicht für Wasserstoff und Methyl oder $R^1$ und $R^2$ nicht gemeinsam für eine unsubstituierte $-(CH_2)_4$-Brücke stehen, wenn $R^3$ und $R^4$ Wasserstoff bedeuten, dadurch gekennzeichnet, dass man eine Ketocarbonsäure XI

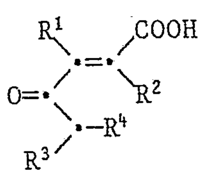

(XI),

worin $R^1$ bis $R^4$ wie zuvor definiert sind, unter Wasserabspaltung cyclisiert.

5. Verfahren zur Bekämpfung unerwünschten Pflanzenwuchses, dadurch gekennzeichnet, dass man eine Verbindung der Formel I

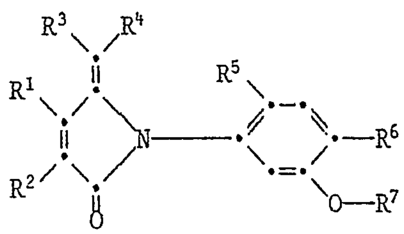

(I),

worin

$R^1$ Wasserstoff; ($C_1$-$C_8$)-Alkyl;

$R^2$ Wasserstoff; ($C_1$-$C_8$)-Alkyl; oder

$R^1$ und $R^2$ gemeinsam eine gegebenenfalls bis zu zweifach durch ($C_1$-$C_4$)-Alkyl substituierte -$(CH_2)_4$-Brücke;

$R^3$ und $R^4$ unabhängig voneinander Wasserstoff; oder ($C_1$-$C_4$)-Alkyl; oder

einer der Reste $R^3$ oder $R^4$ Wasserstoff und der andere ein gegebenenfalls bis zu dreifach gleich oder verschieden durch Halogen, ($C_1$-$C_4$)-Alky oder ($C_1$-$C_4$)-Alkoxy substituierter Phenylrest; oder

$R^3$ und $R^4$ zusammen eine -$(CH_2)_n$-Brücke, mit $n = 2, 3, 4, 5$ oder 6, die gewünschtenfalls bis zu dreifach durch ($C_1$-$C_4$)-Alky substituiert sein kann;

$R^5$ Wasserstoff; oder Fluor;

$R^6$ Halogen;

$R^7$ Wasserstoff; ($C_1$-$C_8$)-Alkyl; ($C_3$-$C_8$)-Alkenyl; ($C_1$-$C_8$)-Alkinyl; ($C_3$-$C_7$)-Cycloalkyl; ($C_1$-$C_4$)-Alkyl, einfach substituiert durch: ($C_1$-$C_4$)-Alkoxy, ($C_1$-$C_4$)-Alkylthio, mono-($C_1$-$C_4$)-Alkylamino, di-($C_1$-$C_4$)-Alkylamino, Cyano, ($C_3$-$C_7$)-Cycloalkyl, Phenyl, Halogenphenyl, ($C_1$-$C_4$)-Alkylphenyl, ($C_1$-$C_4$)-Alkoxyphenyl, ($C_1$-$C_4$)-Halogenalkylphenyl, ($C_1$-$C_4$)-Halogenalkoxyphenyl, Nitrophenyl, Cyanophenyl, Hydroxy, 1,3-Dioxolan-2-yl, 1,3-Dioxan-2-yl oder CO-$R^8$; ($C_1$-$C_8$)-Alkyl, ($C_3$-$C_8$)-Alkenyl oder ($C_3$-$C_7$)-Cycloalkyl, welche ein oder mehrfach durch Halogen substituiert sind; oder CO-$R^8$.

$R^8$ ($C_1$-$C_8$)-Alkyl; ($C_3$-$C_8$)-Alkenyl; Benzyl, gegebenenfalls am Phenylring einfach substituiert durch Halogen, ($C_1$-$C_4$)-Alkyl, ($C_1$-$C_4$)-Alkoxy, ($C_1$-$C_4$)-Halogenalkyl, Nitro oder Cyano; ($C_3$-$C_7$)-Cycloalkyl; Phenyl, gegebenenfalls bis zu dreifach substituiert durch Halogen, ($C_1$-$C_8$)-Alkyl, ($C_1$-$C_4$)-Alkoxy, ($C_1$-$C_4$)-Halogenalkyl oder einfach substituiert durch Cyano oder Nitro; Furan; Thiophen; ($C_1$-$C_8$)-Alkoxy; ($C_1$-$C_4$)-Alkoxy-($C_1$-$C_4$)-alkoxy; ($C_3$-$C_8$)-Alkenyloxy; ($C_3$-$C_8$)-Alkinyloxy; ($C_1$-$C_8$)-Alkylthio; ($C_3$-$C_8$)-Alkenylthio; ($C_3$-$C_8$)-Alkinylthio; Amino; mono($C_1$-$C_4$)-Alkylamino; di-($C_1$-$C_4$)-Alkylamino; Phenylamino, gegebenenfalls bis zu dreifach substituiert durch Halogen, ($C_1$-$C_4$)-Alkyl, ($C_1$-$C_4$)-Alkoxy oder ($C_1$-$C_4$)-Halogenalkyl oder einfach substituiert durch Cyano oder Nitro;

bedeutet auf die Pflanze oder deren Lebensraum einwirken lässt.

6. Verfahren zur Beeinflussung des Wachstums von Kulturpflanzen, dadurch gekennzeichnet, dass man eine wuchsregulatorisch wirksame Menge einer Verbindung der Formel I, gemäss Anspruch 5, auf die Kulturpflanze oder deren Lebensraum einwirken lässt.

7. Verfahren nach Anspruch 6 zur Defoliation von Baumwolle.